(19) Europäisches Patentamt
European Patent Office
Office européen des brevets

(11) EP 3 587 423 A1

(12) EUROPEAN PATENT APPLICATION

(43) Date of publication:
**01.01.2020 Bulletin 2020/01**

(51) Int Cl.:
*C07D 487/14* (2006.01) *H01L 51/50* (2006.01)

(21) Application number: **18180155.6**

(22) Date of filing: **27.06.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Idemitsu Kosan Co., Ltd.**
**Chiyoda-ku,**
**Tokyo 100-8321 (JP)**

(72) Inventors:
- **Schäfer, Thomas**
  **4410 Liestal (CH)**
- **Murer, Peter**
  **4104 Oberwil (CH)**
- **Ogiwara, Toshinari**
  **Sodegaura-shi, Chiba 299-0293 (JP)**
- **Chebotareva, Natalia**
  **68220 Hagenthal le Bas (FR)**
- **Wolleb, Heinz**
  **4232 Fehren (CH)**

(74) Representative: **Hollah, Dorothee**
**Patentanwälte**
**Isenbruck Bösl Hörschler PartG mbB**
**Eastsite One**
**Seckenheimer Landstrasse 4**
**68163 Mannheim (DE)**

# (54) ORGANIC COMPOUNDS AND AN ORGANIC ELECTROLUMINESCENCE DEVICE COMPRISING THE SAME

(57) A condensed benzimidazolo[1,2-a]benzimidazole carrying aryl- or heteroarylnitril, a process for preparing said condensed benzimidazolo[1,2-a]benzimidazole carrying aryl- or heteroarylnitril, an organic electroluminescence device comprising said condensed benzimidazolo[1,2-a]benzimidazole carrying aryl- or heteroarylnitril, an electronic device comprising said organic electroluminescence device, an emitting layer comprising at least one inventive condensed benzimidazolo[1,2-a]benzimidazole carrying aryl- or heteroarylnitril and the use of said inventive condensed benzimidazolo[1,2-a]benzimidazole carrying aryl- or heteroarylnitril in an electronic device as a delayed-fluorescent emitter and/or as a host material.

EP 3 587 423 A1

## Description

The present invention relates to a condensed benzimidazolo[1,2-a]benzimidazole carrying aryl- or heteroarylnitril, a process for preparing said condensed benzimidazolo[1,2-a]benzimidazole carrying aryl- or heteroarylnitril, an organic electroluminescence device comprising said condensed benzimidazolo[1,2-a]benzimidazole carrying aryl- or heteroarylnitril, an electronic device comprising said organic electroluminescence device, an emitting layer comprising at least one inventive condensed benzimidazolo[1,2-a]benzimidazole carrying aryl- or heteroarylnitril and the use of said inventive condensed benzimidazolo[1,2-a]benzimidazole carrying aryl- or heteroarylnitril in an electronic device as a delayed-fluorescent emitter and/or as a host material.

When a voltage is applied to an organic electroluminescence device (hereinafter, occasionally referred to as "organic EL device"), holes and electrons are injected into an emitting layer respectively from an anode and a cathode. The injected holes and electrons are recombined to generate excitons in the emitting layer. According to the electron spin statistics theory, singlet excitons are generated at a ratio of 25% and triplet excitons are generated at a ratio of 75%.

A fluorescent organic EL device, which uses emission caused by singlet excitons, is applied to a full color display of a mobile phone, a TV set and the like. It has been studied to further improve a performance of the fluorescent organic EL device. For instance, in order to further improve a luminous efficiency, an organic EL device with use of singlet excitons and triplet excitons has been studied.

An organic EL device using delayed fluorescence has been proposed and studied. For instance, a thermally activated delayed fluorescence (TADF) mechanism has been studied. The TADF mechanism uses such a phenomenon that inverse intersystem crossing from triplet excitons to singlet excitons thermally occurs when a material having a small energy gap (ΔST) between singlet energy level and triplet energy level is used. As for thermally activated delayed fluorescence, refer to, for instance, "ADACHI, Chihaya, ed. (March 22, 2012), "Yuki Hando-tai no Debaisu Bussei (Device Physics of Organic Semiconductors)", Kodansha, pp. 261-262".

For instance, the following documents also disclose organic EL devices using the TADF mechanism.

Hajime Nakanotani et al., "High-Efficiency organic light-emitting diodes with fluorescent emitters", NATURE COMMUNICATIONS, in May 30, 2014, 5, 4016 (DOI: 10.1038/ncomms5016) concerns TADF molecules as assistant dopants for obtaining highly efficient OLEDs. The following TADF molecules are mentioned:

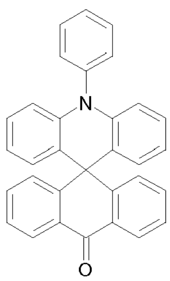 (ACRSA), 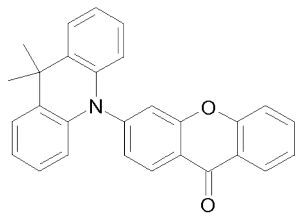 (ACRXTN), 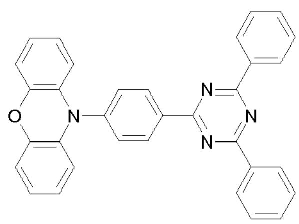 (PXZ-TRZ), 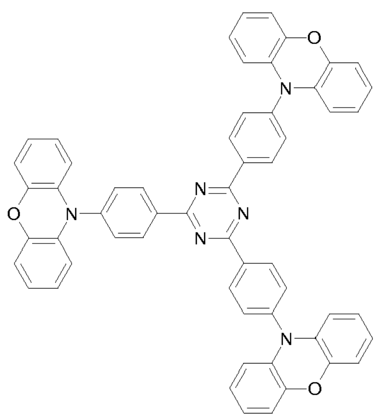 (Tri-PXZ-TRZ)

Dongdong Zhang et al., 26, 29, 2014, 5050-5055 concerns materials with small singlet-triplet splits ($\Delta E_{ST}$s) as sensitizing hosts to excite fluorescent dopants, breaking the trade-off between small $\Delta E_{ST}$ and high radiative decay rates. A highly efficient orange-fluorescent organic light-emitting diode (OLED) is prepared, showing a maximum external quantum efficiency of 12.2%. The following material is mentioned as sensitizing host:

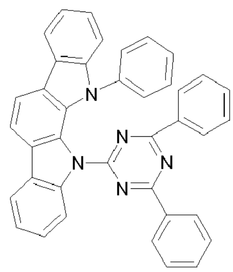

(DIC-TRZ)

WO2016/157113 A1 concerns benzimidazolo[1,2-a]benzimidazole carrying aryl- or heteroarylnitril groups for organic light emitting diodes. Said compounds of formula show low singlet triplet splitting, which makes them useful as TADF (Thermally Activated Delayed Fluorescence, Adv. Mater. 2014, 26, 7931-7958) emitter or TADF host materials in combination with fluorescent emitters.

WO 2017/115596 A1 concerns a heat-activated delayed-fluorescence organic electroluminescence element that includes a luminescent layer between a positive electrode and a negative electrode that face. The organic electroluminescence element contains, in at least one luminescent layer, a heat-activated delayed-fluorescence material or a heat-activated delayed-fluorescence material and a host material. The heat-activated delayed-fluorescence material is represented by general formula (1), wherein A is an electron-withdrawing group such as a CN group and $D^1$ and $D^2$ are electron-donating groups that have an indole-ring structure:

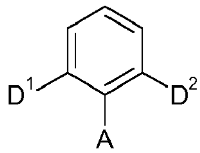

(1)

In WO 2017/093958 A1 benzimidazolo[1 ,2-a]benzimidazole derivatives of the following formula for organic light emitting diodes are described:

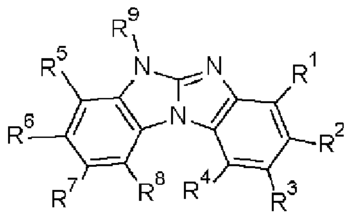

(I)

wherein at least two of the substituents $R^1$ and $R^2$, $R^2$ and $R^3$, $R^3$ and $R^4$, $R^5$ and $R^6$, $R^6$ and $R^7$, or $R^7$ and $R^8$ form together one of the following ring systems

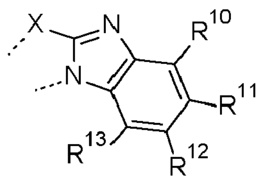

(IIa),

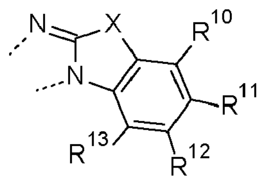

(IIb)

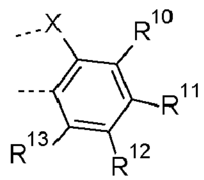

(IIc).

KR2017/0081438 discloses compounds of the following formula and organic optoelectronic devices comprising said compounds as hosts for green phosphorescent emitters.

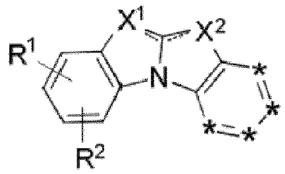 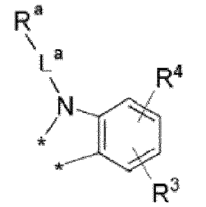 .

An object of the invention is to provide a compound useful in an organic electroluminescence device capable of prolonging the lifetime, improving the luminous efficiency, and inhibiting the roll-off when driven at a high current density, and an electronic device including the organic electroluminescence device. Especially, a compound should be provided useful in an organic electroluminescence device capable of improving luminous efficiency, especially at high current.

Said object is achieved by a compound of formula (I),

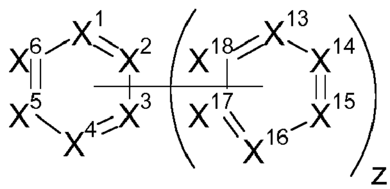
(I)

wherein

z is 0 or 1, preferably 0;
$X^1$ is $CR^{1a}$ or N;
$X^2$ is $CR^{2a}$ or N;
$X^3$ is $CR^{3a}$ or N;
$X^4$ is $CR^{4a}$ or N;
$X^5$ is $CR^{5a}$ or N;
$X^6$ is $CR^{6a}$ or N;

wherein

0, 1 or 2, preferably 0 or 1, more preferably 0 of the groups $X^1$, $X^2$, $X^3$, $X^4$, $X^5$ and $X^6$ are N;
$R^{1a}$, $R^{2a}$, $R^{3a}$, $R^{4a}$, $R^{5a}$ and $R^{6a}$ are each independently CN, $R^A$, $D^1$ or $D^2$;

wherein

1, 2, 3, 4 or 5, preferably 1, 2, 3 or 4, more preferably 1 or 2 of the residues $R^{1a}$, $R^{2a}$, $R^{3a}$, $R^{4a}$, $R^{5a}$ and $R^{6a}$ are CN;
0, 1 or 2 of the residues $R^{1a}$, $R^{2a}$, $R^{3a}$, $R^{4a}$, $R^{5a}$ and $R^{6a}$ are $R^A$;
0 or 1, 2 preferably 0 or 1 of the residues $R^{1a}$, $R^{2a}$, $R^{3a}$, $R^{4a}$, $R^{5a}$ and $R^{6a}$ are $D^2$; and
1 of the residues $R^{1a}$, $R^{2a}$, $R^{3a}$, $R^{4a}$, $R^{5a}$ and $R^{6a}$ is $D^1$;

$X^{13}$ is $CR^{13a}$ or N;
$X^{14}$ is $CR^{14a}$ or N;
$X^{15}$ is $CR^{15a}$ or N;
$X^{16}$ is $CR^{16a}$ or N;
$X^{17}$ is $CR^{17a}$ or N;
$X^{18}$ is $CR^{18a}$ or N;

wherein

0, 1 or 2, preferably 0 or 1, more preferably 0 of the groups $X^{13}$, $X^{14}$, $X^{15}$, $X^{16}$, $X^{17}$ and $X^{18}$ are N;
$R^{13a}$, $R^{14a}$, $R^{15a}$, $R^{16a}$, $R^{17a}$ and $R^{18a}$ are each independently CN, $R^A$, $D^1$ or $D^2$;

wherein

1, 2, 3 or 4, preferably 1 or 2 of the residues $R^{13a}$, $R^{14a}$, $R^{15a}$, $R^{16a}$, $R^{17a}$ and $R^{18a}$ are CN;
0, 1 or 2 of the residues $R^{13a}$, $R^{14a}$, $R^{15a}$, $R^{16a}$, $R^{17a}$ and $R^{18a}$ are $R^A$;
0 or 1 of the residues $R^{13a}$, $R^{14a}$, $R^{15a}$, $R^{16a}$, $R^{17a}$ and $R^{18a}$ are $D^1$; and
1 of the residues $R^{13a}$, $R^{14a}$, $R^{15a}$, $R^{16a}$, $R^{17a}$ and $R^{18a}$ is $D^2$;

$D_2$ is a donor group,
$D_1$ is a group of formula (III)

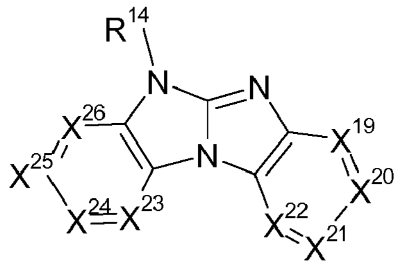
(III)

wherein

$X^{19}$ is $CR^1$ or N;
$X^{20}$ is $CR^2$ or N;
$X^{21}$ is $CR^3$ or N;
$X^{22}$ is $CR^4$ or N;
$X^{23}$ is $CR^5$ or N;
$X^{24}$ is $CR^6$ or N;
$X^{25}$ is $CR^7$ or N;
$X^{26}$ is $CR^8$ or N;

wherein

0, 1 or 2, preferably 0 or 1, more preferably 0 of the groups $X^{19}$, $X^{20}$, $X^{21}$, $X^{22}$, $X^{23}$, $X^{24}$, $X^{25}$ and $X^{26}$ are N;

wherein
at least two adjacent residues $R^1$, $R^2$, $R^3$, $R^4$, $R^5$, $R^6$, $R^7$ and $R^8$ of at least two adjacent groups $CR^1$, $CR^2$, $CR^3$, $CR^4$, $CR^5$, $CR^6$, $CR^7$ and $CR^8$ together form a ring of formula (IV)

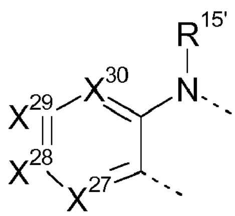
(IV)

wherein

$X^{27}$ is $CR^9$ or N;
$X^{28}$ is $CR^{10}$ or N;
$X^{29}$ is $CR^{11}$ or N;
$X^{30}$ is $CR^{12}$ or N;

wherein

0, 1 or 2, preferably 0 or 1, more preferably 0 of the groups $X^{27}$, $X^{28}$, $X^{29}$ and $X^{30}$ are N; wherein
the dotted lines are bonding sites to the group of formula (III), and
the remaining residues $R^1$ to $R^8$ and $R^9$ to $R^{12}$ are independently of each other H, -CN, a $C_1$-$C_{25}$alkyl group which is unsubstituted or substituted by at least one group E and/or interrupted by at least one group D, a $C_6$-$C_{40}$aryl group which is unsubstituted or substituted by at least one group E, a $C_1$-$C_{24}$heteroaryl group which is unsubstituted or substituted by at least one group E, a $C_7$-$C_{25}$aralkyl group which is unsubstituted or substituted by at least one group E, a $C_5$-$C_{12}$cycloalkyl group which is unsubstituted or substituted by at least one group E, $-OR^{21}$, $-SR^{21}$,$-NR^{17}R^{18}$, $-COR^{20}$, $-COOR^{19}$, $-CONR^{17}R^{18}$, $-SiR^{22}R^{23}R^{24}$, $-POR^{25}R^{27}$, or halogen, or
at least two of the remaining residues $R^1$ to $R^8$ and $R^9$ to $R^{12}$ if present at adjacent carbon atoms together form at least one $C_6$-$C_{18}$aryl or $C_1$-$C_{24}$heteroaryl ring or ring system,
$R^A$ is H, a $C_1$-$C_{25}$alkyl group which is unsubstituted or substituted by at least one group E and/or interrupted by at least one group D, a $C_6$-$C_{40}$aryl group which is unsubstituted or substituted by at least one group E, a $C_1$-$C_{24}$heteroaryl group which is unsubstituted or substituted by at least one group E, a $C_7$-$C_{25}$aralkyl group which is unsubstituted or substituted by at least one group E, a $C_5$-$C_{12}$cycloalkyl group which is unsubstituted or substituted by at least one group E, $-OR^{21}$, $-SR^{21}$, $-NR^{17}R^{18}$, $-COR^{20}$, $-COOR^{19}$, $-CONR^{17}R^{18}$, $-SiR^{22}R^{23}R^{24}$, $-POR^{25}R^{27}$, or halogen, or
at least two of the residues $R^A$ if present at adjacent carbon atoms together form at least one $C_6$-$C_{18}$aryl ring or ring system which is unsubstituted or substituted by at least one group E or $C_1$-$C_{24}$heteroaryl ring or ring system which is unsubstituted or substituted by at least one group E,
$R^{14}$ and $R^{15'}$ are each independently a $C_6$-$C_{40}$aryl group which is unsubstituted or substituted by at least one group E or a $C_1$-$C_{24}$heteroaryl group which is unsubstituted or substituted by at least one group E,
wherein one of $R^{14}$ and $R^{15'}$ is a group $-(M)_m$---,
wherein the dotted line is a bonding site to formula (I),
M is a $C_6$-$C_{40}$arylene group which is unsubstituted or substituted by at least one group E, a $C_1$-$C_{24}$heteroarylene group which is unsubstituted or substituted by at least one group E, a $C_1$-$C_{25}$alkylene group which is unsubstituted or substituted by at least one group E and/or interrupted by at least one group D, or a $C_2$-$C_{25}$alkenylene group which is unsubstituted or substituted by at least one group E and/or interrupted by at least one group D,
m is 0, 1 or 2, preferably 0 or 1, more preferably 0,
D is -CO-, -COO-, -S-, -SO-, $-SO_2-$, -O-, $-CR^{15}=CR^{16}-$, $-NR^{17}-$, $-SiR^{22}R^{23}-$, $-POR^{25}-$, $-C\equiv C-$,
E is $-OR^{21}$, $-SR^{21}$, $-NR^{17}R^{18}$, $-COR^{20}$, $-COOR^{19}$, $-CONR^{17}R^{18}$, -CN, $-SiR^{22}R^{23}R^{24}$, $-POR^{25}R^{27}$, halogen, preferably F,
a $C_6$-$C_{40}$aryl group which is unsubstituted or is substituted by at least one group selected from a $C_1$-$C_{18}$alkyl group, a $C_1$-$C_{18}$alkoxy group, a $C_1$-$C_{18}$alkyl group, which is interrupted by at least one O, a $C_6$-$C_{18}$aryl group, which is in turn unsubstituted or substituted by at least one $C_1$-$C_{18}$alkyl group or at least one $C_1$-$C_{18}$alkoxy group, a $C_5$-$C_{12}$cycloalkyl group, which is in turn unsubstituted or substituted by at least one $C_1$-$C_{18}$alkyl group or at least one $C_1$-$C_{18}$alkoxy group, a $C_1$-$C_{18}$heteroaryl group, which is in turn unsubstituted or substituted by at least one $C_1$-$C_{18}$alkyl group or at least one $C_1$-$C_{18}$alkoxy group, -F, $-CF_3$, $-CF_2CF_3$, $-CF_2CF_2CF_3$, $-CF(CF_3)_2$,$-(CF_2)_3CF_3$ and $-C(CF_3)_3$,
a $C_1$-$C_{18}$ alkyl or a $C_1$-$C_{18}$alkyl group which is interrupted by at least one O,
a $C_1$-$C_{24}$heteroaryl group which is unsubstituted or substituted by at least one group selected from a $C_1$-$C_{18}$alkyl group, a $C_1$-$C_{18}$alkoxy group, a $C_1$-$C_{18}$alkyl group, which is interrupted by at least one O, a $C_6$-$C_{18}$aryl group, which is in turn unsubstituted or substituted by at least one $C_1$-$C_{18}$alkyl group or at least one $C_1$-$C_{18}$alkoxygroup, a $C_5$-$C_{12}$cycloalkyl group, which is in turn unsubstituted or substituted by at least one $C_1$-$C_{18}$alkyl group or at least one $C_1$-$C_{18}$alkoxy group, a $C_1$-$C_{18}$heteroaryl group, which is in turn unsubstituted or substituted by at least one $C_1$-$C_{18}$alkyl group or at least one $C_1$-$C_{18}$alkoxy group, -F, $-CF_3$, $-CF_2CF_3$, $-CF_2CF_2CF_3$, $-CF(CF_3)_2$, $-(CF_2)_3CF_3$ and $-C(CF_3)_3$,
$R^{15}$ and $R^{16}$ are independently of each other H, a $C_6$-$C_{18}$aryl group which is unsubstituted or substituted by at least one $C_1$-$C_{18}$alkyl group or at least one $C_1$-$C_{18}$alkoxy group,
$R^{17}$ and $R^{18}$ are independently of each other H, a $C_6$-$C_{18}$aryl group which is unsubstituted or substituted by at least one $C_1$-$C_{18}$alkyl group or at least one $C_1$-$C_{18}$alkoxy group, a $C_1$-$C_{18}$alkyl group or a $C_1$-$C_{18}$alkyl group, which is interrupted by at least one O, or
$R^{17}$ and $R^{18}$ together form a five or six membered aliphatic, aromatic or heteroaromatic ring, which is unsubstituted or substituted by at least one group E,
$R^{19}$ is H, a $C_6$-$C_{18}$aryl group which is unsubstituted or substituted by at least one $C_1$-$C_{18}$alkyl group or at least one $C_1$-$C_{18}$alkoxy group, a $C_1$-$C_{18}$alkyl group or a $C_1$-$C_{18}$alkyl group, which is interrupted by at least one O,
$R^{20}$ is H or a $C_6$-$C_{18}$aryl group which is unsubstituted or substituted by at least one $C_1$-$C_{18}$alkyl group or at least one $C_1$-$C_{18}$alkoxy group, a $C_1$-$C_{18}$ alkyl group or a $C_1$-$C_{18}$alkyl group, which is interrupted by at least one O,
$R^{21}$ is independently of each other H, a $C_6$-$C_{18}$aryl group which is unsubstituted or substituted by at least one $C_1$-$C_{18}$alkyl group or at least one $C_1$-$C_{18}$alkoxy group, a $C_1$-$C_{18}$alkyl group or a $C_1$-$C_{18}$alkyl group, which is interrupted

by at least one O,

$R^{22}$, $R^{23}$ and $R^{24}$ are independently of each other H, a $C_1$-$C_{18}$alkyl group, a $C_6$-$C_{18}$aryl group which is unsubstituted or substituted by at least one $C_1$-$C_{18}$alkyl group, and

$R^{25}$ and $R^{27}$ are independently of each other H, a $C_1$-$C_{18}$alkyl group, a $C_6$-$C_{18}$aryl group which is unsubstituted or substituted by at least one $C_1$-$C_{18}$alkyl group, a $C_7$-$C_{25}$aralkyl which is unsubstituted or substituted by at least one group E, a $C_5$-$C_{12}$cycloalkyl group which is unsubstituted or substituted by at least one group E.

Preferred compounds of formula (I) having the following formula

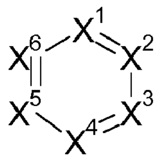

(I*)

wherein

$X^1$ is $CR^{1a}$ or N;
$X^2$ is $CR^{2a}$ or N;
$X^3$ is $CR^{3a}$ or N;
$X^4$ is $CR^{4a}$ or N;
$X^5$ is $CR^{5a}$ or N;
$X^6$ is $CR^{6a}$ or N;

wherein

0, 1 or 2, preferably 0 or 1, more preferably 0 of the groups $X^1$, $X^2$, $X^3$, $X^4$, $X^5$ and $X^6$ are N;
$R^{1a}$, $R^{2a}$, $R^{3a}$, $R^{4a}$, $R^{5a}$ and $R^{6a}$ are each independently CN, $R^A$, $D^1$ or $D^2$;

wherein

1, 2, 3, 4 or 5, preferably 1, 2, 3 or 4, more preferably 1 or 2 of the residues $R^{1a}$, $R^{2a}$, $R^{3a}$, $R^{4a}$, $R^{5a}$ and $R^{6a}$ are CN;
0, 1 or 2 of the residues $R^{1a}$, $R^{2a}$, $R^{3a}$, $R^{4a}$, $R^{5a}$ and $R^{6a}$ are $R^A$;
0 or 1, 2 preferably 0 or 1 of the residues $R^{1a}$, $R^{2a}$, $R^{3a}$, $R^{4a}$, $R^{5a}$ and $R^{6a}$ are $D^2$; and
1 of the residues $R^{1a}$, $R^{2a}$, $R^{3a}$, $R^{4a}$, $R^{5a}$ and $R^{6a}$ is $D^1$.

Definitions of the groups and residues $R^{1a}$, $R^{2a}$, $R^{3a}$, $R^{4a}$, $R^{5a}$, $R^{6a}$, $R^A$, $D^1$ or $D^2$ are mentioned above and preferred definitions are mentioned below.

According to the above aspect of the invention, compounds suitable for use in an organic electroluminescence device capable of prolonging a lifetime, improving a luminous efficiency, and inhibiting a roll-off when driven at a high current density, an organic electroluminescence device, and an electronic device including the organic electroluminescence device can be provided. Especially, the compounds are useful in an organic electroluminescence device capable of improving luminous efficiency, especially at high current.

One key finding of the inventors of the present invention is the relevance of the presence of a condensed benzimidazolo[1,2-a]benzimidazole group. Therefore, the compounds of the present invention are characterized by a good suitability as TADF hosts or emitters in a TADF emitter system.

The compounds of the present invention may be used for electrophotographic photoreceptors, photoelectric converters, organic solar cells (organic photovoltaics), switching elements, such as organic transistors, for example, organic FETs and organic TFTs, organic light emitting field effect transistors (OLEFETs), image sensors, dye lasers and electroluminescence devices, such as, for example, organic light-emitting diodes (OLEDs).

Accordingly, a further subject of the present invention is directed to an electronic device, comprising a compound according to the present invention. The electronic device is preferably an electroluminescence device, such as an organic light-emitting diode (OLED).

The compounds of formula (I) are preferably used as TADF emitter materials, or TADF host materials in combination with at least one fluorescent emitter.

A further subject of the present invention is directed to an emitting layer, comprising a compound of formula (I) according to the present invention. In said embodiment a compound of formula (I) is preferably used as TADF emitter

material, or TADF host material in combination with at least one fluorescent emitter.

The term of "XX to YY carbon atoms" referred to by "a substituted or unsubstituted group ZZ having XX to YY carbon atoms" used herein is the number of carbon atoms of the unsubstituted group ZZ and does not include any carbon atom in the substituent of the substituted group ZZ. "YY" is larger than "XX" and each of "XX" and "YY" represents an integer of 1 or more.

The term of "XX to YY atoms" referred to by "a substituted or unsubstituted group ZZ having XX to YY atoms" used herein is the number of atoms of the unsubstituted group ZZ and does not include any atom in the substituent of the substituted group ZZ. "YY" is larger than "XX" and each of "XX" and "YY" represents an integer of 1 or more.

The term "$C_{ZZ}$-$C_{YY}$-" referred to by "$C_{ZZ}$-$C_{YY}$-ZZ group which is unsubstituted or substituted" used herein is the number of carbon atoms of the unsubstituted group ZZ and does not include any heteroatom or any atom in the substituent of the substituted group ZZ. "YY" is larger than "XX" and each of "XX" and "YY" represents an integer of 1 or more.

The term of "unsubstituted group ZZ" referred to by "a substituted or unsubstituted group ZZ" used herein means the group ZZ wherein no hydrogen atom is substituted by a substituent.

The number of "ring carbon atoms" ("a ring formed of carbon atoms") referred to herein means the number of the carbon atoms included in the atoms which are members forming the ring itself of a compound in which a series of atoms is bonded to form the ring (for example, a monocyclic compound, a fused ring compound, a cross-linked compound, a carbocyclic compound, and a heterocyclic compound). If the ring has a substituent, the carbon atom in the substituent is not included in the ring carbon atom. The same applies to the number of "ring carbon atom" described below, unless otherwise noted. For example, a benzene ring has 6 ring carbon atoms, a naphthalene ring has 10 ring carbon atoms, a pyridinyl group has 5 ring carbon atoms, and a furanyl group has 4 ring carbon atoms. If a benzene ring or a naphthalene ring has, for example, an alkyl substituent, the carbon atom in the alkyl substituent is not counted as the ring carbon atom of the benzene or naphthalene ring. In case of a fluorene ring to which a fluorene substituent is bonded (inclusive of a spirofluorene ring), the carbon atom in the fluorene substituent is not counted as the ring carbon atom of the fluorene ring.

The number of "ring atom" ("a ring formed of atoms") referred to herein means the number of the atoms which are members forming the ring itself (for example, a monocyclic ring, a fused ring, and a ring assembly) of a compound in which a series of atoms is bonded to form the ring (for example, a monocyclic compound, a fused ring compound, a cross-linked compound, a carbocyclic compound, and a heterocyclic compound). The atom not forming the ring (for example, hydrogen atom(s) for saturating the valence of the atom which forms the ring) and the atom in a substituent, if the ring is substituted, are not counted as the ring atom. The same applies to the number of "ring atoms" described below, unless otherwise noted. For example, a pyridine ring has 6 ring atoms, a quinazoline ring has 10 ring atoms, and a furan ring has 5 ring atoms. The hydrogen atom on the ring carbon atom of a pyridine ring or a quinazoline ring and the atom in a substituent are not counted as the ring atom. In case of a fluorene ring to which a fluorene substituent is bonded (inclusive of a spirofluorene ring), the atom in the fluorene substituent is not counted as the ring atom of the fluorene ring.

The definition of "hydrogen atom" used herein includes isotopes different in the neutron numbers, i.e., light hydrogen (protium), heavy hydrogen (deuterium), and tritium.

The residues mentioned in the specification of the present application generally have the following preferred meanings, if said residues are not further specified in specific embodiments mentioned below:

Halogen is fluorine, chlorine, bromine and iodine.

$C_1$-$C_{25}$alkyl ($C_1$-$C_{18}$alkyl) is typically linear or branched, where possible. Examples are methyl, ethyl, n-propyl, isopropyl, n-butyl, sec.-butyl, isobutyl, tert.-butyl, n-pentyl, 2-pentyl, 3-pentyl, 2,2-dimethylpropyl, 1,1,3,3-tetramethylpentyl, n-hexyl, 1-methylhexyl, 1,1,3,3,5,5-hexamethylhexyl, n-heptyl, isoheptyl, 1,1,3,3-tetramethylbutyl, 1-methylheptyl, 3-methylheptyl, n-octyl, 1,1,3,3-tetramethylbutyl and 2-ethylhexyl, n-nonyl, decyl, undecyl, dodecyl, tridecyl, tetradecyl, pentadecyl, hexadecyl, heptadecyl, or octadecyl. $C_1$-$C_8$alkyl is typically methyl, ethyl, n-propyl, isopropyl, n-butyl, sec.-butyl, isobutyl, tert.-butyl, n-pentyl, 2-pentyl, 3-pentyl, 2,2-dimethyl-propyl, n-hexyl, n-heptyl, n-octyl, 1,1,3,3-tetramethylbutyl and 2-ethylhexyl. Preferred alkyl groups are $C_1$-$C_4$alkyl groups. $C_1$-$C_4$alkyl is typically methyl, ethyl, n-propyl, isopropyl, n-butyl, sec.-butyl, isobutyl, tert.-butyl. More preferred alkyl groups are methyl, isopropyl and tert.-butyl.

The alkyl groups may be substituted by one or more halogens and form halogenated alkyl groups (haloalkyl groups). Specific examples of the above halogenated alkyl groups are a fluoromethyl group, difluoromethyl group, trifluoromethyl group, fluoroethyl group, trifluoromethylmethyl group, trifluoroethyl group and pentafluoroethyl group.

$C_1$-$C_{25}$alkoxy groups ($C_1$-$C_{18}$alkoxy groups) are straight-chain or branched alkoxy groups, e.g. methoxy, ethoxy, n-propoxy, isopropoxy, n-butoxy, sec-butoxy, tert-butoxy, amyloxy, isoamyloxy or tert-amyloxy, heptyloxy, octyloxy, isooctyloxy, nonyloxy, decyloxy, undecyloxy, dodecyloxy, tetradecyloxy, pentadecyloxy, hexadecyloxy, heptadecyloxy and octadecyloxy. Examples of $C_1$-$C_8$alkoxy are methoxy, ethoxy, n-propoxy, isopropoxy, n-butoxy, sec.-butoxy, isobutoxy, tert.-butoxy, n-pentyloxy, 2-pentyloxy, 3-pentyloxy, 2,2-dimethylpropoxy, n-hexyloxy, n-heptyloxy, n-octyloxy, 1,1,3,3-tetramethylbutoxy and 2-ethylhexyloxy, preferably $C_1$-$C_4$alkoxy such as typically methoxy, ethoxy, n-propoxy,

isopropoxy, n-butoxy, sec.-butoxy, isobutoxy, tert.-butoxy.

The term "cycloalkyl group" is typically $C_5$-$C_{12}$cycloalkyl, such as cyclopentyl, cyclohexyl, cycloheptyl, cyclooctyl, cyclononyl, cyclodecyl, cycloundecyl, cyclododecyl, preferably cyclopentyl, cyclohexyl, cycloheptyl, or cyclooctyl, which may be unsubstituted or substituted. More preferred cycloalkyl groups are cyclopentyl and cyclohexyl, which may be unsubstituted or substituted.

$C_6$-$C_{24}$aryl (preferably $C_6$-$C_{18}$aryl), which optionally can be substituted, is typically phenyl, 4-methylphenyl, 4-methoxyphenyl, naphthyl, especially 1-naphthyl, or 2-naphthyl, biphenylyl, ter-phenylyl, pyrenyl, 2- or 9-fluorenyl, phenanthryl, or anthryl, which may be unsubstituted or substituted. More preferred aryl groups are $C_6$-$C_{10}$aryl groups which are unsubstituted or substituted. Phenyl, 1-naphthyl and 2-naphthyl are examples of a $C_6$-$C_{10}$aryl group. Most preferred is a phenyl group which is unsubstituted or substituted. Further most preferred is an unsubstituted phenyl group.

$C_6$-$C_{24}$aryloxy, which optionally can be substituted, is typically $C_6$-$C_{10}$aryloxy, which optionally can be substituted by one, or more $C_1$-$C_8$alkyl and/or $C_1$-$C_8$alkoxy groups, such as, for example, phenoxy, 1-naphthoxy, or 2-naphthoxy.

$C_7$-$C_{25}$aralkyl is typically benzyl, 2-benzyl-2-propyl, β-phenyl-ethyl, α,α-dimethylbenzyl, ω-phenyl-butyl, ω,ω-dimethyl-ω-phenyl-butyl, ω-phenyl-dodecyl, ω-phenyl-octadecyl, ω-phenyleicosyl or ω-phenyl-docosyl, preferably $C_7$-$C_{18}$aralkyl such as benzyl, 2-benzyl-2-propyl, β-phenyl-ethyl, α,α-dimethylbenzyl, ω-phenyl-butyl, ω,ω-dimethyl-ω-phenyl-butyl, ω-phenyl-dodecyl or ω-phenyl-octadecyl, and particularly preferred $C_7$-$C_{12}$aralkyl such as benzyl, 2-benzyl-2-propyl, β-phenyl-ethyl, α,α-dimethylbenzyl,ω-phenyl-butyl, or ω,ω-dimethyl-ω-phenyl-butyl, in which both the aliphatic hydrocarbon group and aromatic hydrocarbon group may be unsubstituted or substituted. Preferred examples are benzyl, 2-phenylethyl, 3-phenylpropyl, naphthylethyl, naphthylmethyl, and cumyl.

$C_2$-$C_{30}$heteroaryl (preferably $C_2$-$C_{13}$ heteroarylaryl) represents a ring with five to seven ring atoms or a condensed ring system, wherein nitrogen, oxygen or sulfur are the possible hetero atoms, and is typically a heterocyclic group with five to 30 atoms having at least six conjugated π-electrons such as thienyl, benzothiophenyl, dibenzothiophenyl, thianthrenyl, furyl, furfuryl, 2H-pyranyl, benzofuranyl, isobenzofuranyl, dibenzofuranyl, phenoxythienyl, pyrrolyl, imidazolyl, pyrazolyl, pyridyl, bipyridyl, triazinyl, pyrimidinyl, pyrazinyl, pyridazinyl, indolizinyl, isoindolyl, indolyl, indazolyl, purinyl, quinolizinyl, chinolyl, isochinolyl, phthalazinyl, naphthyridinyl, chinoxalinyl, chinazolinyl, cinnolinyl, pteridinyl, carbazolyl, carbolinyl, benzotriazolyl, benzoxazolyl, phenanthridinyl, acridinyl, pyrimidinyl, phenanthrolinyl, phenazinyl, isothiazolyl, phenothiazinyl, isoxazolyl, furazanyl, 4-imidazo[1,2-a]benzimidazoyl, 5-benzimidazo[1,2-a]benzimidazoyl, benzimidazolo[2,1-b][1,3]benzothiazolyl, carbazolyl, or phenoxazinyl, which can be unsubstituted or substituted. Benzimidazo[1,2-a]benzimidazo-5-yl, benzimidazo[1,2-a]benzimidazo-2-yl, carbazolyl and dibenzofuranyl are examples of a $C_2$-$C_{14}$heteroaryl group.

A $C_2$-$C_{13}$heteroaryl group is for example, benzimidazo[1,2-a]benzimidazo-5-yl (

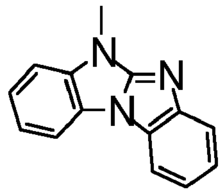

),

benzimidazo[1,2-a]benzimidazo-2-yl (

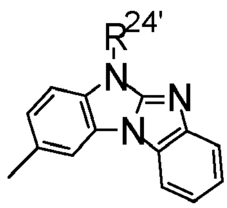

),

benzimidazolo[2,1-b][1,3]benzothiazolyl, benzimidazolo[2,1-b][1,3]benzoxazole, carbazolyl, dibenzofuranyl, or dibenzotihophenyl, which can be unsubstituted or substituted, especially by $C_6$-$C_{10}$aryl, or $C_6$-$C_{10}$aryl, which is substituted by $C_1$-$C_4$alkyl; or $C_2$-$C_{13}$heteroaryl.

$C_2$-$C_{30}$heteroaryl (preferably $C_2$-$C_{13}$ heteroarylaryl) means that the heteroaryl residue comprises at least 2 carbon atoms and at most 30 carbon atoms in the base skeleton (without substituents). The further atoms in the heteroaryl base skeleton are heteroatoms (N, O and/or S).

$R^{24'}$ is in each case independently $C_1$-$C_{18}$alkyl, such as methyl, ethyl, n-propyl, iso-propyl, n-butyl, isobutyl, sec-butyl, hexyl, octyl, or 2-ethyl-hexyl, or $C_6$-$C_{14}$aryl, such as phenyl, tolyl, naphthyl, phenanthronyl, triphenylenyl, fluoranthenyl or biphenylyl

$C_6$-$C_{24}$arylene groups are typically phenylene, 4-methylphenylene, 4-methoxyphenylene, naphthylene, especially 1-naphthylene, or 2-naphthylene, biphenylylene, terphenylylene, pyrenylene, 2- or 9-fluorenylene, phenanthrylene, or anthrylene, which may be unsubstituted or substituted. Preferred $C_6$-$C_{24}$arylene groups are 1,4-phenylene, 1,3-phenylene, 1,2-phenylene, 3,3'-biphenylylene, 3,3'-m-terphenylene, 2- or 9-fluorenylene, phenanthrylene, or 4-t-butylphenylene, which may be unsubstituted or substituted. More preferred arylene groups are 1,4-phenylene, 1,3-phenylene, 1,2-phenylene groups and 4-t-butyl-phenylene.

$C_2$-$C_{30}$heteroarylene groups, which optionally can be substituted, represent a ring with five to seven ring atoms or a condensed ring system comprising at least one heteroatom. Nitrogen, oxygen or sulfur are the possible hetero atoms. Typical heteroarylene groups are heterocyclic groups with five to 30 atoms having at least six conjugated -electrons such as thienylene, benzothiophenylene, dibenzothiophenylene, thianthrenylene, furylene, furfurylene, 2H-pyranylene, benzofuranylene, isobenzofuranylene, dibenzofuranylene, phenoxythienylene, pyrrolylene, imidazolylene, pyrazolylene, pyridylene, bipyridylene, triazinylene, pyrimidinylene, pyrazinylene, pyridazinylene, indolizinylene, isoindolylene, indolylene, indazolylene, purinylene, quinolizinylene, chinolylene, isochinolylene, phthalazinylene, naphthyridinylene, chinoxalinylene, chinazolinylene, cinnolinylene, pteridinylene, carbolinylene, benzotriazolylene, benzoxazolylene, phenanthridinylene, acridinylene, pyrimidinylene, phenanthrolinylene, phenazinylene, isothiazolylene, phenothiazinylene, isoxazolylene, furazanylene, carbazolylene, benzimidazo[1,2-a]benzimidazo-2,5-ylene, or phenoxazinylene, which can be unsubstituted or substituted. Preferred $C_2$-$C_{30}$heteroarylen groups are pyridylene, triazinylene, pyrimidinylene, carbazolylene, dibenzofuranylene and benzimidazo[1,2-a]benzimidazo-2,5-ylene (

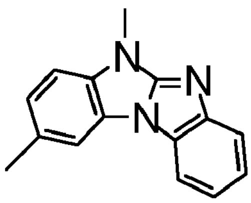

),

which can be unsubstituted or substituted, especially by $C_6$-$C_{10}$aryl, $C_6$-$C_{10}$aryl which is substituted by $C_1$-$C_4$alkyl; or $C_2$-$C_{14}$heteroaryl.

Suitable substituents of the abovementioned groups are the groups E mentioned above and below.

If a substituent occurs more than one time in a group, it can be different in each occurrence.

The wording "substituted by E" means that one or more, especially one, two or three substituents E might be present.

As described above, some of the aforementioned groups may be substituted by E and/or, if desired, interrupted by D. Interruptions are of course possible only in the case of groups containing at least 2 carbon atoms connected to one another by single bonds; $C_6$-$C_{18}$aryl is not interrupted; interrupted arylalkyl contains the unit D in the alkyl moiety. "a $C_1$-$C_{18}$alkyl substituted by one or more E and/or interrupted by one or more units D" or "a $C_1$-$C_{18}$alkyl group, which is interrupted by at least one O" is for example, $(CH_2CH_2O)_{1-9}$-$R^x$, where $R^x$ is H or $C_1$-$C_{10}$alkyl or $C_2$-$C_{10}$alkanoyl (e.g. $CO$-$CH(C_2H_5)C_4H_9$), $CH_2$-$CH(OR^{y'})$-$CH_2$-O-$R^y$, where $R^y$ is $C_1$-$C_{18}$alkyl, $C_5$-$C_{12}$Cycloalkyl, phenyl, $C_7$-$C_{15}$phenylalkyl, and $R^{y'}$ embraces the same definitions as $R^y$ or is H; $C_1$-$C_8$alkylene-COO-$R^z$, e.g. $CH_2COOR_z$, $CH(CH_3)COOR^z$, $C(CH_3)_2COOR^z$, where $R^z$ is H, $C_1$-$C_{18}$alkyl, $(CH_2CH_2O)_{1-9}$-$R^x$, and $R^x$ embraces the definitions indicated above; $CH_2CH_2$-O-CO-$CH$=$CH_2$; $CH_2CH(OH)CH_2$-O-CO-$C(CH_3)$=$CH_2$.

D is -CO-, -COO-, -S-, -SO-, -$SO_2$-, -O-, -$CR^{15}$=$CR^{16}$-, -$NR^{17}$-, -$SiR^{22}R^{23}$-, -$POR^{25}$- or -C≡C-. Preferably, D is -CO-, -COO-, -S-, -SO-, -$SO_2$-, -O-, -$NR^{17}$-, wherein $R^{17}$ is $C_1$-$C_{18}$alkyl, such as methyl, ethyl, n-propyl, iso-propyl, n-butyl, isobutyl, or sec-butyl, or $C_6$-$C_{14}$aryl, such as phenyl, tolyl, naphthyl, or biphenylyl, or $C_2$-$C_{30}$heteroaryl, such as, for example, benzimidazo[1,2-a]benzimidazo-2-yl (

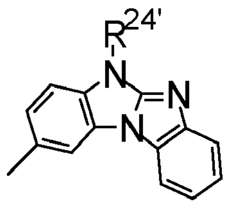

),

carbazolyl, dibenzofuranyl, dibenzothiophenyl, dialkyl fluranthenyl (dimethyl) which can be unsubstituted or substituted by E, especially by $C_6$-$C_{10}$aryl, which may in turn be unsubstituted or substituted by $C_1$-$C_4$alkyl; or $C_2$-$C_{13}$heteroaryl, which can be unsubstituted or substituted by E, which is preferably unsubstituted. More preferably, D is O.

E is $-OR^{21}$, $-SR^{21}$, $-NR^{17}R^{18}$, $-COR^{20}$, $-COOR^{19}$, $-CONR^{17}R^{18}$, -CN, $-SiR^{22}R^{23}R^{24}$, $-POR^{25}R^{27}$, halogen, preferably F,
a $C_6$-$C_{40}$aryl group which is unsubstituted or is substituted by at least one group selected from a $C_1$-$C_{18}$alkyl group, a $C_1$-$C_{18}$alkoxy group, a $C_1$-$C_{18}$alkyl group, which is interrupted by at least one O, a $C_6$-$C_{18}$aryl group, which is in turn unsubstituted or substituted by at least one $C_1$-$C_{18}$alkyl group or at least one $C_1$-$C_{18}$alkoxy group, a $C_5$-$C_{12}$cycloalkyl group, which is in turn unsubstituted or substituted by at least one $C_1$-$C_{18}$alkyl group or at least one $C_1$-$C_{18}$alkoxy group, a $C_1$-$C_{18}$heteroaryl group, which is in turn unsubstituted or substituted by at least one $C_1$-$C_{18}$alkyl group or at least one $C_1$-$C_{18}$alkoxy group, -F, $-CF_3$, $-CF_2CF_3$, $-CF_2CF_2CF_3$, $-CF(CF_3)_2$, $-(CF_2)_3CF_3$ and $-C(CF_3)_3$,
a $C_1$-$C_{18}$alkyl group,
a $C_1$-$C_{18}$ alkoxy group,
a $C_1$-$C_{24}$heteroaryl group which is unsubstituted or substituted by at least one group selected from a $C_1$-$C_{18}$alkyl group, a $C_1$-$C_{18}$alkoxy group, a $C_1$-$C_{18}$alkyl group, which is interrupted by at least one O, a $C_6$-$C_{18}$aryl group, which is in turn unsubstituted or substituted by at least one $C_1$-$C_{18}$alkyl group or at least one $C_1$-$C_{18}$alkoxy group, a $C_5$-$C_{12}$cycloalkyl group, which is in turn unsubstituted or substituted by at least one $C_1$-$C_{18}$alkyl group or at least one $C_1$-$C_{18}$alkoxy group, a $C_1$-$C_{18}$heteroaryl group, which is in
turn unsubstituted or substituted by at least one $C_1$-$C_{18}$alkyl group or at least one $C_1$-$C_{18}$alkoxy group, -F, $-CF_3$, $-CF_2CF_3$, $-CF_2CF_2CF_3$, $-CF(CF_3)_2$, $-(CF_2)_3CF_3$ and $-C(CF_3)_3$.

Preferably, E is CN, F, $CF_3$,
a $C_6$-$C_{18}$aryl group which is unsubstituted or is substituted by at least one group selected from a $C_1$-$C_{18}$alkyl group, a $C_1$-$C_{18}$alkoxy group, an unsubstituted $C_6$-$C_{18}$aryl group,
a $C_1$-$C_{18}$alkyl group,
a $C_1$-$C_{24}$heteroaryl group which is unsubstituted or substituted by at least one group selected from a $C_1$-$C_{18}$alkyl group, a $C_1$-$C_{18}$alkoxy group, an unsubstituted $C_6$-$C_{18}$aryl group.

$R^{15}$ and $R^{16}$ are independently of each other H, a $C_6$-$C_{18}$aryl group which is unsubstituted or substituted by at least one $C_1$-$C_{18}$alkyl group or at least one $C_1$-$C_{18}$alkoxy group, preferably H, a $C_6$-$C_{10}$aryl group which is unsubstituted or substituted by at least one $C_1$-$C_8$alkyl group or at least one $C_1$-$C_8$alkoxy group, more preferably H.

$R^{17}$ and $R^{18}$ are independently of each other H, a $C_6$-$C_{18}$aryl group which is unsubstituted or substituted by at least one $C_1$-$C_{18}$alkyl group or at least one $C_1$-$C_{18}$alkoxy group, a $C_1$-$C_{18}$alkyl group or a $C_1$-$C_{18}$alkyl group, which is interrupted by at least one O, or
$R^{17}$ and $R^{18}$ together form a five or six membered aliphatic, aromatic or heteroaromatic ring, which is unsubstituted or substituted by at least one group E,

Preferably, $R^{17}$ and $R^{18}$ are independently of each other a $C_6$-$C_{10}$aryl group which is unsubstituted or substituted by at least one $C_1$-$C_8$alkyl group or at least one $C_1$-$C_8$alkoxy group, a $C_1$-$C_8$alkyl group or a $C_1$-$C_8$alkoxy group, more preferably a $C_6$-$C_{10}$aryl group which is unsubstituted.

$R^{19}$ is H, a $C_6$-$C_{18}$aryl group which is unsubstituted or substituted by at least one $C_1$-$C_{18}$alkyl group or at least one $C_1$-$C_{18}$alkoxy group, a $C_1$-$C_{18}$alkyl group or a $C_1$-$C_{18}$alkyl group, which is interrupted by at least one O, preferably a $C_6$-$C_{10}$aryl group which is unsubstituted or substituted by at least one $C_1$-$C_8$alkyl group or at least one $C_1$-$C_8$alkoxy group or a $C_1$-$C_8$alkyl group, more preferably a $C_6$-$C_{10}$aryl group which is unsubstituted or a $C_1$-$C_8$alkyl group.

$R^{20}$ is H or a $C_6$-$C_{18}$aryl group which is unsubstituted or substituted by at least one $C_1$-$C_{18}$alkyl group or at least one $C_1$-$C_{18}$alkoxy group, a $C_1$-$C_{18}$ alkyl group or a $C_1$-$C_{18}$alkyl group, which is interrupted by at least one O, preferably a $C_6$-$C_{10}$aryl group which is unsubstituted or substituted by at least one $C_1$-$C_8$alkyl group or at least one $C_1$-$C_8$alkoxy group or a $C_1$-$C_8$alkyl group, more preferably a $C_6$-$C_{10}$aryl group which is unsubstituted or a $C_1$-$C_8$alkyl group.

$R^{21}$ is independently of each other H, a $C_6$-$C_{18}$aryl group which is unsubstituted or substituted by at least one $C_1$-$C_{18}$alkyl group or at least one $C_1$-$C_{18}$alkoxy group, a $C_1$-$C_{18}$alkyl group or a $C_1$-$C_{18}$alkyl group, which is interrupted by at least one O, preferably a $C_6$-$C_{10}$aryl group which is unsubstituted or substituted by at least one $C_1$-$C_8$alkyl group or at least one $C_1$-$C_8$alkoxy group or a $C_1$-$C_8$alkyl group, more preferably a $C_6$-$C_{10}$aryl group which is unsubstituted or

a $C_1$-$C_8$alkyl group.

$R^{22}$, $R^{23}$ and $R^{24}$ are independently of each other H, a $C_1$-$C_{18}$alkyl group, a $C_6$-$C_{18}$aryl group which is unsubstituted or substituted by at least one $C_1$-$C_{18}$alkyl group, preferably a $C_6$-$C_{10}$aryl group which is unsubstituted or substituted by at least one $C_1$-$C_8$alkyl group or at least one $C_1$-$C_8$alkoxy group or a $C_1$-$C_8$alkyl group, more preferably a $C_6$-$C_{10}$aryl group which is unsubstituted or a $C_1$-$C_8$alkyl group.

$R^{25}$ and $R^{27}$ are independently of each other H, a $C_1$-$C_{18}$alkyl group, a $C_6$-$C_{18}$aryl group which is unsubstituted or substituted by at least one $C_1$-$C_{18}$alkyl group, a $C_7$-$C_{25}$aralkyl which is unsubstituted or substituted by at least one group E, a $C_5$-$C_{12}$cycloalkyl group which is unsubstituted or substituted by at least one group E, preferably a $C_6$-$C_{10}$aryl group which is unsubstituted or substituted by at least one $C_1$-$C_8$alkyl group or at least one $C_1$-$C_8$alkoxy group or a $C_1$-$C_8$alkyl group, more preferably a $C_6$-$C_{10}$aryl group which is unsubstituted or a $C_1$-$C_8$alkyl group.

In formula (I), 0, 1 or 2, preferably 0 or 1, more preferably 0 of the groups $X^1$, $X^2$, $X^3$, $X^4$, $X^5$ and $X^6$ are N, and 0, 1 or 2, preferably 0 or 1, more preferably 0 of the groups $X^{13}$, $X^{14}$, $X^{15}$, $X^{16}$, $X^{17}$ and $X^{18}$ are N. z is 0 or 1.

$R^{1a}$, $R^{2a}$, $R^{3a}$, $R^{4a}$, $R^{5a}$ and $R^{6a}$ in formula (I) are each independently CN, $R^A$, $D^1$ or $D^2$;
wherein
1, 2, 3, 4 or 5, preferably 1, 2, 3 or 4, more preferably 1 or 2 of the residues $R^{1a}$, $R^{2a}$, $R^{3a}$, $R^{4a}$, $R^{5a}$ and $R^{6a}$ are CN;
0, 1 or 2 of the residues $R^{1a}$, $R^{2a}$, $R^{3a}$, $R^{4a}$, $R^{5a}$ and $R^{6a}$ are $R^A$;
0 or 1, 2 preferably 0 or 1, more preferably 1 of the residues $R^{1a}$, $R^{2a}$, $R^{3a}$, $R^{4a}$, $R^{5a}$ and $R^{6a}$ are $D^2$; and
1 of the residues $R^{1a}$, $R^{2a}$, $R^{3a}$, $R^{4a}$, $R^{5a}$ and $R^{6a}$ is $D^1$.

In the case that z is 0 in formula (I), the residues CN and $D^1$ are mandatorily present in the compounds of formula (I), preferably, the residues CN and $D^1$ are mandatorily present in the compounds of formula (I) and at least one additional residue $D^2$, CN or $R^A$ are mandatorily present in the compounds of formula (I), and more preferably, the residues CN, $D^1$ and $D^2$ are mandatorily present in the compounds of formula (I).

$R^{13a}$, $R^{14a}$, $R^{15a}$, $R^{16a}$, $R^{17a}$ and $R^{18a}$ in formula (I) are each independently CN, $R^A$, $D^1$ or $D^2$;
wherein
1, 2, 3 or 4, preferably 1 or 2, more preferably 1 of the residues $R^{13a}$, $R^{14a}$, $R^{15a}$, $R^{16a}$, $R^{17a}$ and $R^{18a}$ are CN;
0, 1 or 2 of the residues $R^{13a}$, $R^{14a}$, $R^{15a}$, $R^{16a}$, $R^{17a}$ and $R^{18a}$ are $R^A$;
0 or 1, more preferably 0 of the residues $R^{13a}$, $R^{14a}$, $R^{15a}$, $R^{16a}$, $R^{17a}$ and $R^{18a}$ are $D^1$; and 1 of the residues $R^{13a}$, $R^{14a}$, $R^{15a}$, $R^{16a}$, $R^{17a}$ and $R^{18a}$ is $D^2$.

In the case that z is 1 in formula (I), two residues CN and one residue $D^1$ and one residue $D^2$ are mandatorily present in the compounds of formula (I).

In formula (I), 0, 1 or 2, preferably 0 or 1, more preferably 0 of the groups $X^1$, $X^2$, $X^3$, $X^4$, $X^5$ and $X^6$ are N, and 0, 1 or 2, preferably 0 or 1, more preferably 0 of the groups $X^{13}$, $X^{14}$, $X^{15}$, $X^{16}$, $X^{17}$ and $X^{18}$ are N. z is 0 or 1.

$R^{1a}$, $R^{2a}$, $R^{3a}$, $R^{4a}$, $R^{5a}$ and $R^{6a}$ in formula (I) are each independently CN, $R^A$, $D^1$ or $D^2$;
wherein
1, 2, 3, 4 or 5, preferably 1, 2, 3 or 4, more preferably 1 or 2 of the residues $R^{1a}$, $R^{2a}$, $R^{3a}$, $R^{4a}$, $R^{5a}$ and $R^{6a}$ are CN;
0, 1 or 2 of the residues $R^{1a}$, $R^{2a}$, $R^{3a}$, $R^{4a}$, $R^{5a}$ and $R^{6a}$ are $R^A$;
0 or 1, 2 preferably 0 or 1, more preferably 1 of the residues $R^{1a}$, $R^{2a}$, $R^{3a}$, $R^{4a}$, $R^{5a}$ and $R^{6a}$ are $D^2$; and
1 of the residues $R^{1a}$, $R^{2a}$, $R^{3a}$, $R^{4a}$, $R^{5a}$ and $R^{6a}$ is $D^1$.

In the compounds of formula (I*), the residues CN and $D^1$ are mandatorily, preferably, the residues CN and $D^1$ are mandatorily present in the compounds of formula (I*) and at least one additional residue $D^2$, CN or $R^A$ are mandatorily present in the compounds of formula (I*), and more preferably, the residues CN, $D^1$ and $D^2$ are mandatorily present in the compounds of formula (I*).

*The residue $R^A$*

$R^A$ in the compound of formula (I) is H, a $C_1$-$C_{25}$alkyl group which is unsubstituted or substituted by at least one group E and/or interrupted by at least one group D, a $C_6$-$C_{40}$aryl group which is unsubstituted or substituted by at least one group E, a $C_1$-$C_{24}$heteroaryl group which is unsubstituted or substituted by at least one group E, a $C_7$-$C_{25}$aralkyl group which is unsubstituted or substituted by at least one group E, a $C_5$-$C_{12}$cycloalkyl group which is unsubstituted or substituted by at least one group E, $-OR^{21}$, $-SR^{21}$, $-NR^{17}R^{18}$, $-COR^{20}$, $-COOR^{19}$, $-CONR^{17}R^{18}$,$-SiR^{22}R^{23}R^{24}$, $-POR^{25}R^{27}$, or halogen, or
at least two of the residues $R^A$ if present at adjacent carbon atoms together form at least one $C_6$-$C_{18}$aryl ring or ring system which is unsubstituted or substituted by at least one group E or $C_1$-$C_{24}$heteroaryl ring or ring system which is unsubstituted or substituted by at least one group E.

Preferably, $R^A$ is H, a $C_1$-$C_{25}$alkyl group which is unsubstituted or substituted by at least one group E and/or interrupted by at least one group D, a $C_6$-$C_{40}$aryl group which is unsubstituted or substituted by at least one group E or a $C_1$-$C_{24}$heteroaryl group which is unsubstituted or substituted by at least one group E, more preferably H, a $C_1$-$C_8$alkyl group which is unsubstituted, a $C_6$-$C_{10}$aryl group which is unsubstituted or a $C_1$-$C_{24}$heteroaryl group which is unsubsti-

tuted, most preferably phenyl or H, even more most preferably H.

*The group $D^1$*

$D^1$ in formula (I) is a group of formula (III)

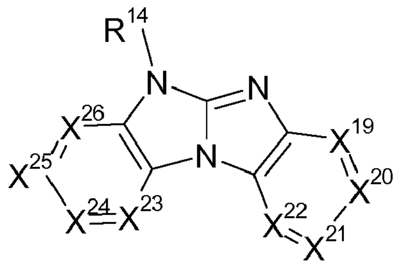

(III)

wherein

$X^{19}$ is $CR^1$ or N;
$X^{20}$ is $CR^2$ or N;
$X^{21}$ is $CR^3$ or N;
$X^{22}$ is $CR^4$ or N;
$X^{23}$ is $CR^5$ or N;
$X^{24}$ is $CR^6$ or N;
$X^{25}$ is $CR^7$ or N;
$X^{26}$ is $CR^8$ or N;

wherein

0, 1 or 2, preferably 0 or 1, more preferably 0 of the groups $X^{19}$, $X^{20}$, $X^{21}$, $X^{22}$, $X^{23}$, $X^{24}$, $X^{25}$ and $X^{26}$ are N;

wherein
at least two adjacent residues $R^1$, $R^2$, $R^3$, $R^4$, $R^5$, $R^6$, $R^7$ and $R^8$ of at least two adjacent groups $CR^1$, $CR^2$, $CR^3$, $CR^4$, $CR^5$, $CR^6$, $CR^7$ and $CR^8$ together form a ring of formula (IV)

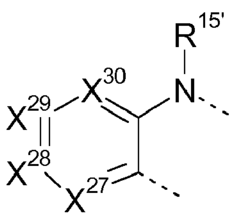

(IV)

wherein

$X^{27}$ is $CR^9$ or N;
$X^{28}$ is $CR^{10}$ or N;
$X^{29}$ is $CR^{11}$ or N;
$X^{30}$ is $CR^{12}$ or N;

wherein
0, 1 or 2, preferably 0 or 1, more preferably 0 of the groups $X^{27}$, $X^{28}$, $X^{29}$ and $X^{30}$ are N; wherein
the dotted lines are bonding sites to the group of formula (III), and
the remaining residues $R^1$ to $R^8$ and $R^9$ to $R^{12}$ are independently of each other H, -CN, a $C_1$-$C_{25}$alkyl group which is unsubstituted or substituted by at least one group E and/or interrupted by at least one group D, a $C_6$-$C_{40}$aryl group which

is unsubstituted or substituted by at least one group E, a $C_1$-$C_{24}$heteroaryl group which is unsubstituted or substituted by at least one group E, a $C_7$-$C_{25}$aralkyl group which is unsubstituted or substituted by at least one group E, a $C_5$-$C_{12}$cycloalkyl group which is unsubstituted or substituted by at least one group E, $-OR^{21}$, $-SR^{21}$,$-NR^{17}R^{18}$, $-COR^{20}$, $-COOR^{19}$, $-CONR^{17}R^{18}$, $-SiR^{22}R^{23}R^{24}$, $-POR^{25}R^{27}$, or halogen, or
at least two of the remaining residues $R^1$ to $R^8$ and $R^9$ to $R^{12}$ if present at adjacent carbon atoms together form at least one $C_6$-$C_{18}$aryl or $C_1$-$C_{24}$heteroaryl ring or ring system.

$R^{14}$ and $R^{15'}$ are each independently a $C_6$-$C_{40}$aryl group which is unsubstituted or substituted by at least one group E or a $C_1$-$C_{24}$heteroaryl group which is unsubstituted or substituted by at least one group E, preferably a $C_6$-$C_{10}$aryl group which is unsubstituted or substituted by at least one group E or a $C_2$-$C_{13}$heteroaryl group which is unsubstituted or substituted by at least one group E, more preferably a $C_6$-$C_{10}$aryl group which is unsubstituted or a $C_2$-$C_{13}$heteroaryl group which is unsubstituted, most preferably phenyl,
wherein one of $R^{14}$ and $R^{15'}$ is a group $-(M)_m$---,
wherein the dotted line is a bonding site to formula (I).

Preferably, $X^{19}$, $X^{20}$, $X^{21}$, $X^{22}$, $X^{23}$, $X^{24}$, $X^{25}$ and $X^{26}$ in the group of formula (III) are $CR^1$, $CR^2$, $CR^3$, $CR^4$, $CR^5$, $CR^6$, $CR^7$ and $CR^8$, and at least two adjacent residues $R^1$, $R^2$, $R^3$, $R^4$, $R^5$, $R^6$, $R^7$ and $R^8$ of at least two adjacent groups $CR^1$, $CR^2$, $CR^3$, $CR^4$, $CR^5$, $CR^6$, $CR^7$ and $CR^8$ together form a ring of formula (IV). More preferably, at least two adjacent residues $R^1$ and $R^2$, $R^5$ and $R^6$ and/or $R^7$ and $R^8$ of at least two adjacent groups $CR^1$ and $CR^2$, $CR^5$ and $CR^6$ and/or $CR^7$ and $CR^8$ together form a ring of formula (IV). Most preferably, at least two adjacent residues $R^1$ and $R^2$ and/or $R^5$ and $R^6$ of at least two adjacent groups $CR^1$ and $CR^2$ and/or $CR^5$ and $CR^6$ together form a ring of formula (IV). Further most preferably, two adjacent residues $R^1$ and of two adjacent groups $CR^1$ and $CR^2$ together form a ring of formula (IV).

Preferably, $X^{27}$, $X^{28}$, $X^{29}$ and $X^{30}$ in the group of formula (IV) are $CR^9$, $CR^{10}$, $CR^{11}$ and $CR^{12}$.

Preferably, the remaining residues $R^1$ to $R^8$ and $R^9$ to $R^{12}$ are independently of each other H, a $C_1$-$C_{25}$alkyl group which is unsubstituted or substituted by at least one group E and/or interrupted by at least one group D, a $C_6$-$C_{40}$aryl group which is unsubstituted or substituted by at least one group E or a $C_1$-$C_{24}$heteroaryl group which is unsubstituted or substituted by at least one group E, more preferably H, a $C_1$-$C_8$alkyl group which is unsubstituted, a $C_6$-$C_{10}$aryl group which is unsubstituted or a $C_1$-$C_{24}$heteroaryl group which is unsubstituted, most preferably H.

Most preferably, $D^1$ is therefore a group of formula (IIIA)

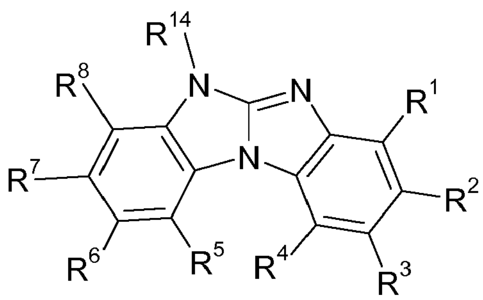

(IIIA)

wherein
at least two adjacent residues $R^1$ to $R^8$ together form a ring of formula (IVA)

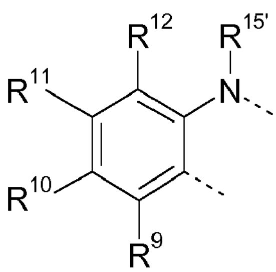

(IVA),

wherein $R^1$ to $R^{12}$, $R^{14}$ and $R^{15'}$ are defined above and below. Further most preferably, $R^1$ to $R^8$ and $R^9$ to $R^{12}$ are H.

Even more most preferably, $D^1$ is selected from the group consisting of

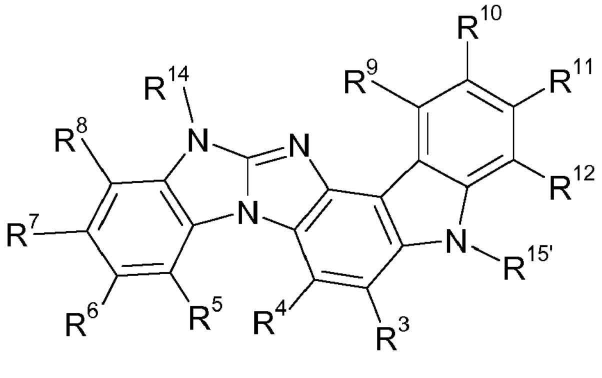

(IIIA-1),

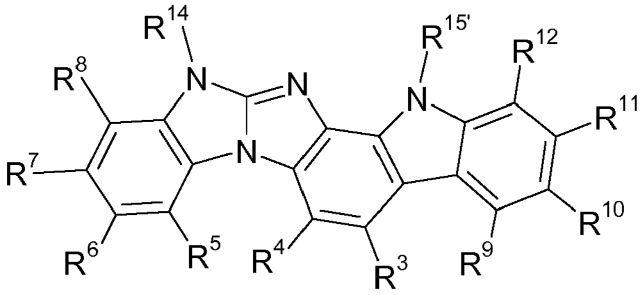

(IIIA-2),

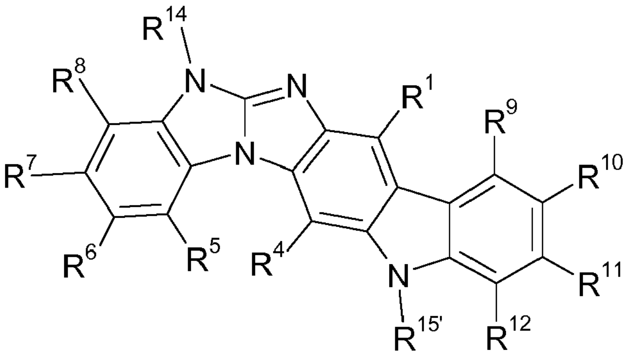

(IIIA-3),

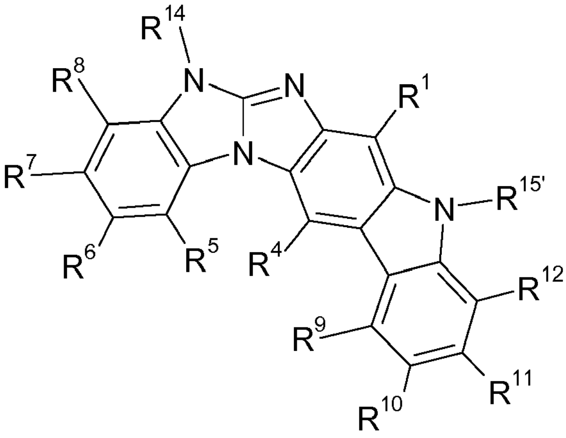

(IIIA-4),

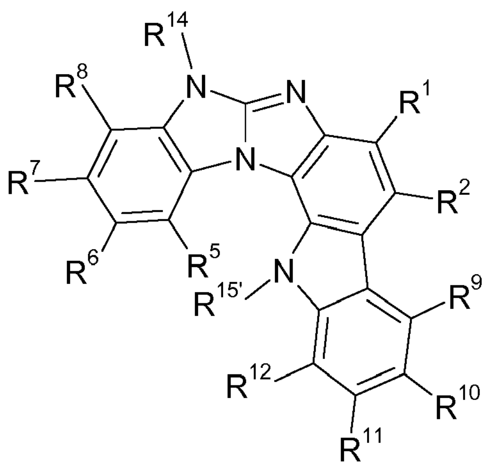

(IIIA-

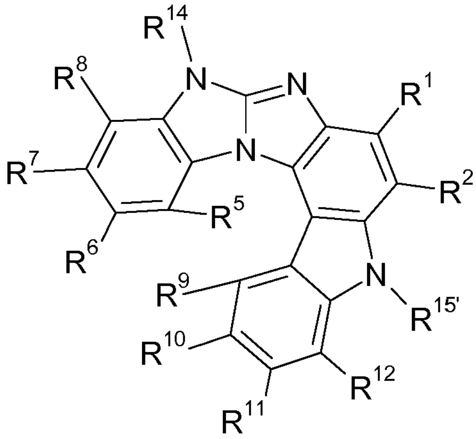

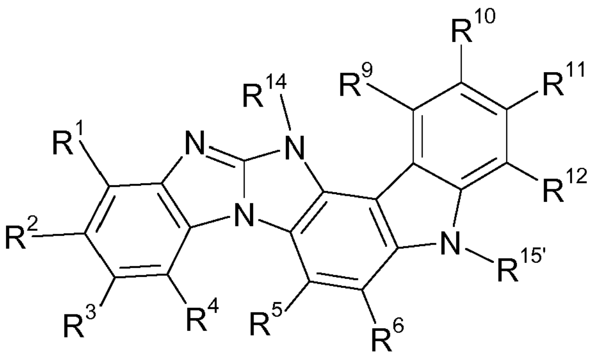

5), (IIIA-6),

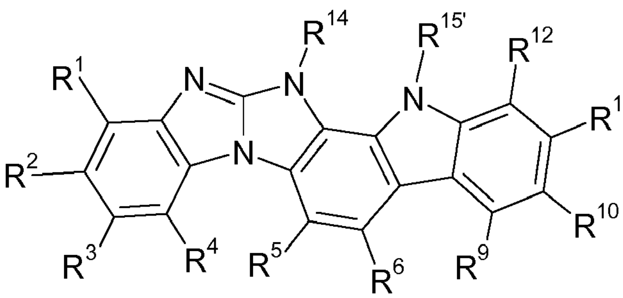

(IIIA-7), (IIIA-8),

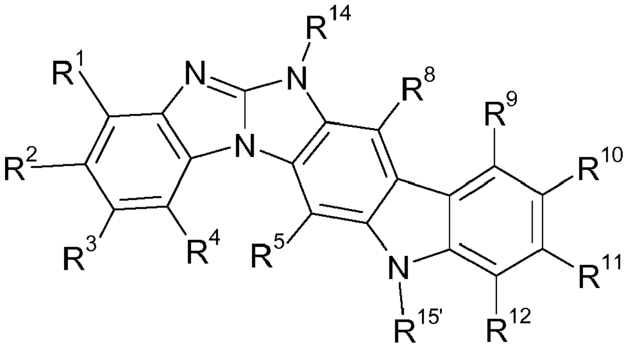

(IIIA-9),

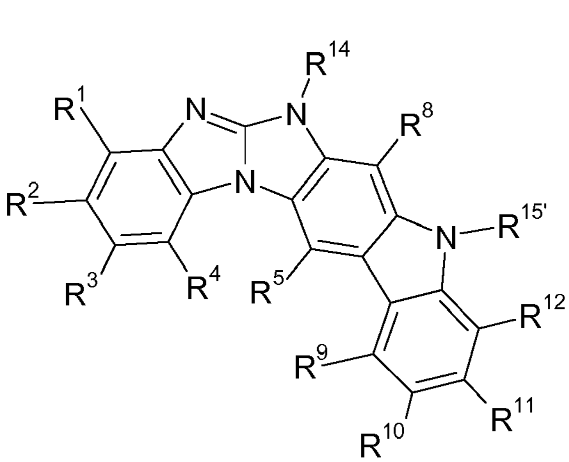

(IIIA-10),

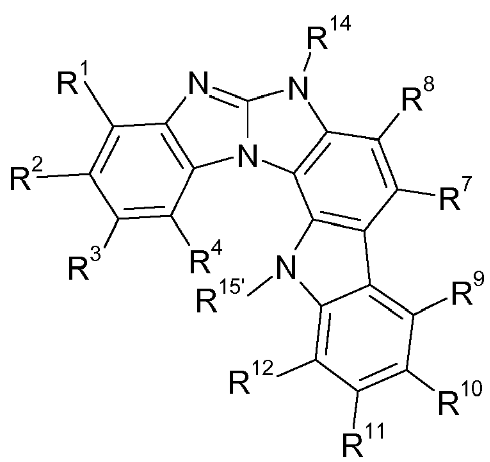

(IIIA-11), and

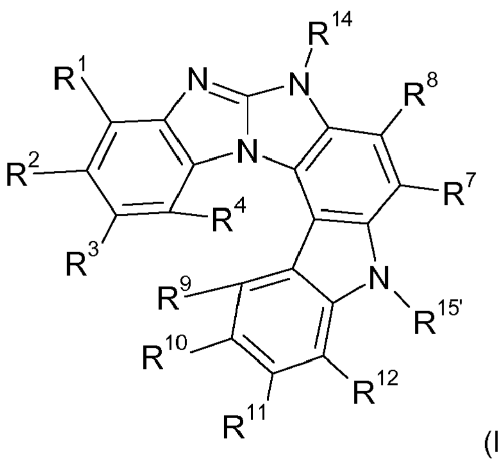

(IIIA-12),

wherein $R^1$ to $R^{12}$, $R^{14}$ and $R^{15'}$ are defined above and below. Further even more most preferably, $R^1$ to $R^8$ and $R^9$ to $R^{12}$ are H.

Most preferred groups $D^1$ are the groups (IIIA-2), (IIIA-10) and (IIIA-11), wherein $R^1$ to $R^{12}$, $R^{14}$ and $R^{15'}$ are defined above and below. Further even more most preferably, $R^1$ to $R^8$ and $R^9$ to $R^{12}$ are H.

Most preferably, $D^1$ is a group of formula (IIIA-2), wherein $R^1$ to $R^{12}$, $R^{14}$ and $R^{15'}$ are defined above and below. Further even more most preferably, $R^1$ to $R^8$ and $R^9$ to $R^{12}$ are H.

$R^{14}$ in the group of formula (III) and $R^{15'}$ in the group of formula (IV) are each independently a $C_6$-$C_{40}$aryl group which is unsubstituted or substituted by at least one group E or a $C_1$-$C_{24}$heteroaryl group which is unsubstituted or substituted by at least one group E, preferably a $C_6$-$C_{10}$aryl group which is unsubstituted or substituted by at least one group E or a $C_1$-$C_{14}$heteroaryl group which is unsubstituted or substituted by at least one group E, more preferably a $C_6$-$C_{10}$aryl group which is unsubstituted or a $C_1$-$C_{14}$heteroaryl group which is unsubstituted, most preferably phenyl,
wherein one of $R^{14}$ and $R^{15'}$ is a group -$(M)_m$---, preferably $R^{15'}$ is a group -$(M)_m$---,
wherein the dotted line is a bonding site to formula (I).

Most preferably, $R^{14}$ is phenyl and $R^{15'}$ is a group -$(M)_m$---.

M is a $C_6$-$C_{40}$arylene group which is unsubstituted or substituted by at least one group E, a $C_1$-$C_{24}$heteroarylene group which is unsubstituted or substituted by at least one group E, a $C_1$-$C_{25}$alkylene group which is unsubstituted or substituted by at least one group E and/or interrupted by at least one group D, or a $C_2$-$C_{25}$alkenylene group which is unsubstituted or substituted by at least one group E and/or interrupted by at least one group D, preferably a $C_6$-$C_{10}$arylene group which is unsubstituted or substituted by at least one group E, a $C_1$-$C_{14}$heteroarylene group which is unsubstituted or substituted by at least one group E, a $C_1$-$C_8$alkylene group which is unsubstituted or substituted by at least one group E and/or interrupted by at least one group D, or a $C_2$-$C_8$alkenylene group which is unsubstituted or substituted by at least one group E and/or interrupted by at least one group D, more preferably a $C_6$-$C_{10}$arylene group which is unsubstituted or substituted by at least one group E or a $C_1$-$C_{14}$heteroarylene group which is unsubstituted or substituted by at least one group E, most preferably a $C_6$-$C_{10}$arylene group which is unsubstituted or a $C_1$-$C_{14}$heteroarylene group which is unsubstituted, further most preferably dibenzofuran-diyl, 1,2-phenylene, 1,3-phenylene or 1,4-phenylene, even further most preferably1,2-phenylene.

m is 0, 1 or 2, preferably 0 or 1, more preferably 0,

*Compounds of formula (I)*

Preferred compounds of formula (I) are represented by the following formula:

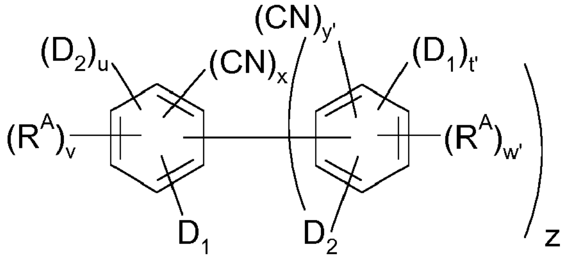

(IA),

preferably formula (I*A),

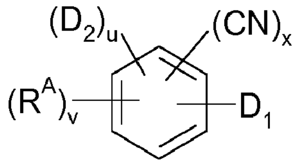

(I*A)

wherein

$D^2$ and $R^A$ and z are defined above and below, and
wherein
x is 1, 2, 3, 4 or 5, preferably 1, 2, 3 or 4, more preferably 1 or 2,
v is 0, 1 or 2,
u is 0 or 1, 2 preferably 0 or 1,
wherein the sum of x, v and u is 1, 2, 3, 4 or 5, preferably 1, 2, 3 or 4, more preferably 1, 2 or 3,
y' is 1, 2, 3 or 4, preferably 1 or 2,
w' is 0, 1 or 2,
t' is 0 or 1,
wherein the sum of y', w' and t' is 1, 2, 3 or 4, preferably 1, 2 or 3,
$D^1$ is a group of formula (IIIA)

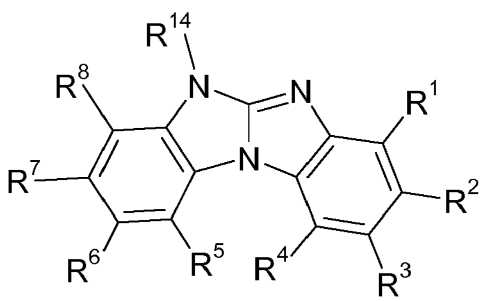

(IIIA)

wherein
at least two adjacent residues $R^1$ to $R^8$ together form a ring of formula (IVA)

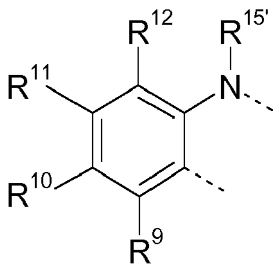

(IVA)

wherein
the dotted lines are bonding sites to the group of formula (IIIA).

The residues $R^1$, $R^2$, $R^3$, $R^4$, $R^5$, $R^6$, $R^7$, $R^8$, $R^9$, $R^{10}$, $R^{11}$, $R^{12}$, $R^{14}$ and $R^{15'}$ are defined above and below.

More preferably, in the case of z = 0 in formula (IA),

x is 1 or 2, preferably 1;
u is 1;
v is 0, 1 or 2, preferably 0 or 1, more preferably 0.

Most preferably, in the case of z = 0 in formula (IA),
x is 1;
u is 1; and
v is 0.

In a further embodiment, in the case of z = 0 in formula (IA),
x is 2;
u is 0; and
v is 0.

In a further embodiment, in the case of z = 0 in formula (IA),
x is 1;
u is 0; and
v is 2.

More preferably, in the case of z = 1 in formula (IA),
x and y' are each independently 1 or 2, preferably 1;
u and t' are 0 or 1; preferably 0,
v and w' are each independently 0, 1 or 2, preferably 0 or 1, more preferably 0.

More preferably, in formula (I*A),
x is 1 or 2, preferably 1;
u is 1;
v is 0, 1 or 2, preferably 0 or 1, more preferably 0.

Most preferably, in formula (I*A),
x is 1;
u is 1; and
v is 0.

More preferred compounds of formula (I) are represented by formulae (IA), preferably by formula (I*A) as mentioned above, wherein
$D^1$ is a group of formula (IIIA)

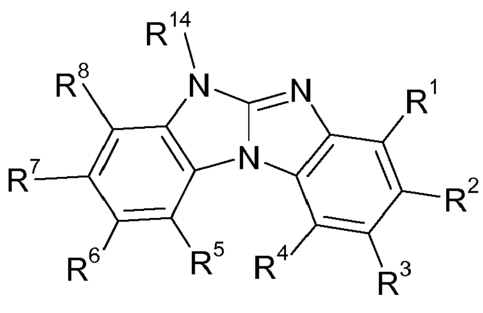

(IIIA)

wherein
at least two adjacent residues $R^1$ to $R^8$ together form a ring of formula (IVA)

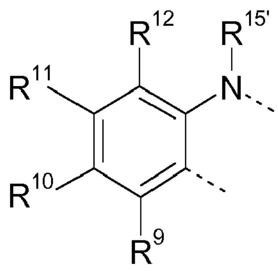

(IVA)

wherein

the dotted lines are bonding sites to the group of formula (IIIA),
wherein $R^1$ to $R^{12}$, $R^{14}$ and $R^{15'}$ are defined above and below. Further even more most preferably, $R^1$ to $R^8$ and $R^9$ to $R^{12}$ are H.

More preferred compounds of formula (I) are represented by formulae (IA), preferably by formula (I*A) as mentioned above, wherein $D^1$ is a group selected from formulae (IIIA-1) to (IIIA-12) as defined above. Preferably, $D^1$ is a group (IIIA-2), (IIIA-10) and (IIIA-11). Most preferably, $D^1$ is a group of formula (IIIA-2).

More preferably, the compound of formula (I*A) is represented by a compound of formula (I*AA)

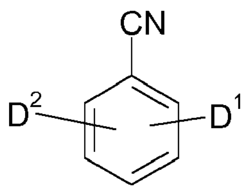

(I*AA),

wherein the groups $D^1$ and $D^2$ are defined above and below.

Most preferably, the compound of formula (I*AA) is selected from the group consisting of the following formulae

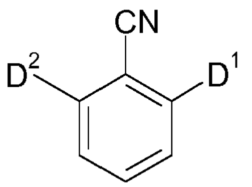
(I*AA-1),
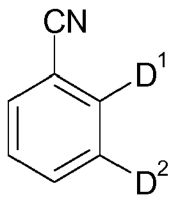
(I*AA-2),
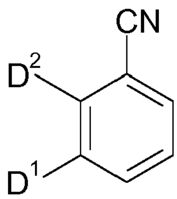
(I*AA-3),

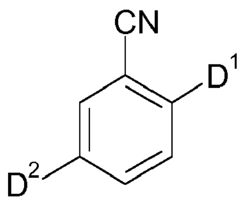
(I*AA-4),
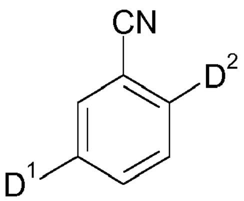
(I*AA-5),
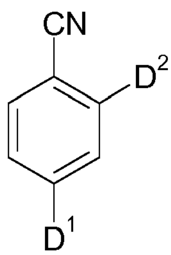
(I*AA-6),

and

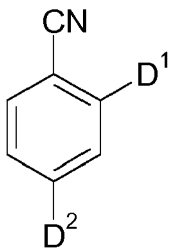

(I*AA-7),

preferably (I*AA-1), (I*AA-3), (I*AA-6) and (I*AA-7), more preferably (I*AA-1), wherein the groups $D^1$ and $D^2$ are defined above and below.

More preferably, the compound of formula (IA), wherein z = 1 is represented by a compound of formula (IIA*), preferably a compound of formula (IIAA)

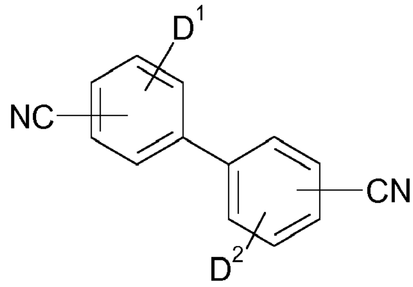

(IIA*),

preferably

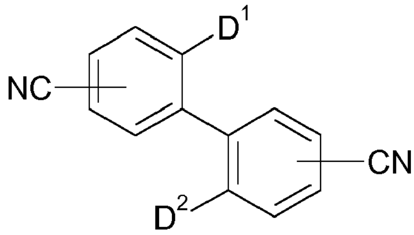

(IIAA),

wherein the groups $D^1$ and $D^2$ are defined above and below.

Most preferably, the compound of formula (IIAA) is selected from the group consisting of the following formulae

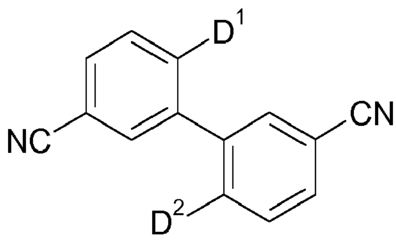

(IIAA-1),

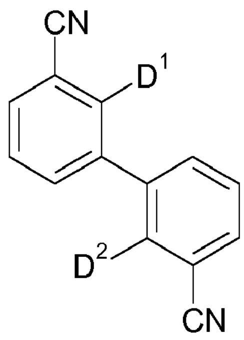

(IIAA-2),

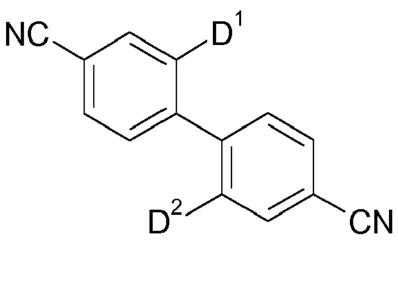

(IIAA-3),

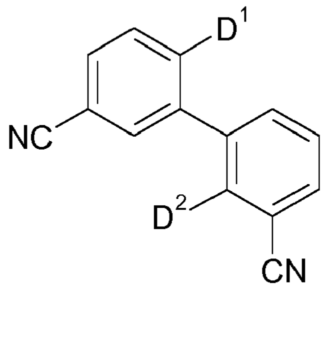

(IIAA-4),

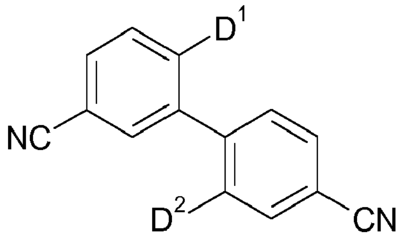 (IIAA-5), 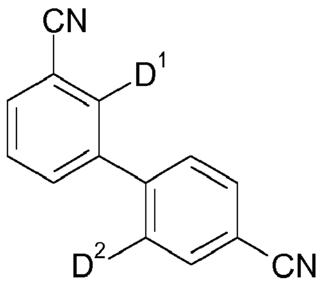 (IIAA-6),

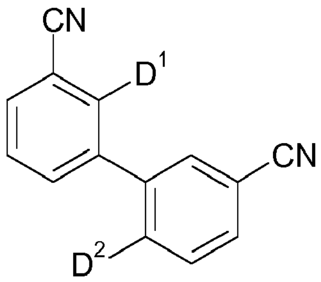 (IIAA-7), 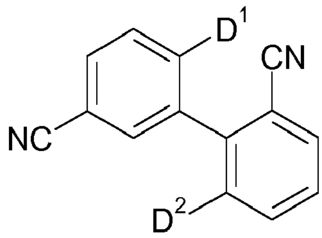 (IIAA-8) and 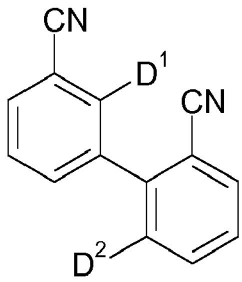

(IIAA-9), preferably (IIAA-1) and (IIAA-2),
wherein the groups $D^1$ and $D^2$ are defined above and below.

Further most preferred compounds of formula (IIAA) are the compounds (IIAA-1) and (IIAA-2).

*The group $D^2$*

The group $D^2$ in the compounds of formula (I) is preferably selected from the groups consisting of (i), (ii), (iii) and (iv):

(i) A group of formula (V),

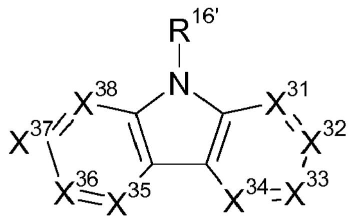 (V)

wherein

$X^{31}$ is $CR^{31}$ or N;
$X^{32}$ is $CR^{32}$ or N;
$X^{33}$ is $CR^{33}$ or N;
$X^{34}$ is $CR^{34}$ or N;
$X^{35}$ is $CR^{35}$ or N;
$X^{36}$ is $CR^{36}$ or N;
$X^{37}$ is $CR^{37}$ or N;
$X^{38}$ is $CR^{38}$ or N;

wherein

0, 1 or 2, preferably 0 or 1, more preferably 0 of the groups $X^{31}$, $X^{32}$, $X^{33}$, $X^{34}$, $X^{35}$, $X^{36}$, $X^{37}$ and $X^{38}$ are N;
or

at least two adjacent residues $R^{31}$, $R^{32}$, $R^{33}$, $R^{34}$, $R^{35}$, $R^{36}$, $R^{37}$ and $R^{38}$ of at least two adjacent groups $CR^{31}$, $CR^{32}$, $CR^{33}$, $CR^{34}$, $CR^{35}$, $CR^{36}$, $CR^{37}$ and $CR^{38}$ together may form a ring of formula (VI)

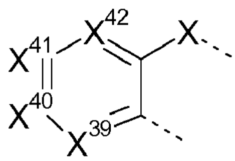

(VI)

wherein

$X^{39}$ is $CR^{39}$ or N;
$X^{40}$ is $CR^{40}$ or N;
$X^{41}$ is $CR^{41}$ or N;
$X^{42}$ is $CR^{42}$ or N;

wherein

0, 1 or 2, preferably 0 or 1, more preferably 0 of the groups $X^{39}$, $X^{40}$, $X^{41}$ and $X^{42}$ are N;
X is O, S, $NR^{17'}$, $CR^{18'}R^{19'}$, $SiR^{18'}R^{19'}$ or $PO(OR^{18'})$; preferably O, S, $NR^{17'}$ or $CR^{18'}R^{19'}$,

wherein
the dotted lines are bonding sites to the group of formula (V), and
the residues $R^{31}$ to $R^{38}$ and $R^{39}$ to $R^{42}$ are independently of each other H, -CN, a $C_1$-$C_{25}$alkyl group which is unsubstituted or substituted by at least one group E and/or interrupted by at least one group D, a $C_6$-$C_{40}$aryl group which is unsubstituted or substituted by at least one group E, a $C_1$-$C_{24}$heteroaryl group which is unsubstituted or substituted by at least one group E, a $C_7$-$C_{25}$aralkyl group which is unsubstituted or substituted by at least one group E, a $C_5$-$C_{12}$cycloalkyl group which is unsubstituted or substituted by at least one group E, $-OR^{21}$, $-SR^{21}$, - $NR^{17}R^{18}$, $-COR^{20}$, $-COOR^{19}$, $-CONR^{17}R^{18}$, $-SiR^{22}R^{23}R^{24}$, $-POR^{25}R^{27}$, or halogen, or
at least two of the remaining residues $R^{31}$ to $R^{38}$ and $R^{39}$ to $R^{42}$ if present at adjacent carbon atoms together may form at least one $C_6$-$C_{18}$aryl ring, which is unsubstituted or substituted by at least one group E or at least one $C_1$-$C_{24}$heteroaryl ring, which is unsubstituted or substituted by at least one group E,
$R^{16'}$ and $R^{17'}$ are each independently a $C_6$-$C_{40}$aryl group which is unsubstituted or substituted by at least one group E or a $C_1$-$C_{24}$heteroaryl group which is unsubstituted or substituted by at least one group E,
$R^{18'}$ and $R^{19'}$ are each independently hydrogen, a $C_1$-$C_{25}$alkyl group which is unsubstituted or substituted by at least one group E and/or interrupted by at least one group D, a $C_6$-$C_{40}$aryl group which is unsubstituted or substituted by at least one group E or a $C_1$-$C_{24}$heteroaryl group which is unsubstituted or substituted by at least one group E,
wherein one of $R^{16'}$ and $R^{17'}$ is a group $-(M)_m$---,
wherein the dotted line is a bonding site to formula (I).

Preferably, $X^{31}$, $X^{32}$, $X^{33}$, $X^{34}$, $X^{35}$, $X^{36}$, $X^{37}$ and $X^{38}$ in formula (V) are $CR^{31}$, $CR^{32}$, $CR^{33}$, $CR^{34}$, $CR^{35}$, $CR^{36}$, $CR^{37}$ and $CR^{38}$, and $X^{39}$, $X^{40}$, $X^{41}$ and $X^{42}$ in formula (VI) are preferably $CR^{39}$, $CR^{40}$, $CR^{41}$ and $CR^{42}$.

Preferably, the residues $R^{31}$ to $R^{38}$ and $R^{39}$ to $R^{42}$ are independently of each other H, -CN, a $C_1$-$C_{25}$alkyl group which is unsubstituted or substituted by at least one group E and/or interrupted by at least one group D, a $C_6$-$C_{40}$aryl group which is unsubstituted or substituted by at least one group E, a $C_1$-$C_{24}$heteroaryl group which is unsubstituted or substituted by at least one group E, a $C_7$-$C_{25}$aralkyl group which is unsubstituted or substituted by at least one group E, a $C_5$-$C_{12}$cycloalkyl group which is unsubstituted or substituted by at least one group E, $-OR^{21}$, $-SR^{21}$, - $NR^{17}R^{18}$, $-COR^{20}$, $-COOR^{19}$, $-CONR^{17}R^{18}$, $-SiR^{22}R^{23}R^{24}$, $-POR^{25}R^{27}$, or halogen, or
at least two of the remaining residues $R^{31}$ to $R^{38}$ and $R^{39}$ to $R^{42}$ if present at adjacent carbon atoms together may form at least one $C_6$-$C_{18}$aryl ring, which is unsubstituted or substituted by at least one group E or at least one $C_1$-$C_{24}$heteroaryl ring, which is unsubstituted or substituted by at least one group E,
or at least two adjacent residues $R^{31}$, $R^{32}$, $R^{33}$, $R^{34}$, $R^{35}$, $R^{36}$, $R^{37}$ and $R^{38}$ of at least two adjacent groups $CR^{31}$, $CR^{32}$, $CR^{33}$, $CR^{34}$, $CR^{35}$, $CR^{36}$, $CR^{37}$ and $CR^{38}$ together may form a ring of formula (VI) as defined above.
More preferably, $R^{31}$ to $R^{38}$ and $R^{39}$ to $R^{42}$ are independently of each other H, -CN, a $C_1$-$C_8$alkyl group which is unsubstituted or substituted by at least one group E and/or interrupted by at least one group D, a $C_6$-$C_{10}$aryl group which is unsubstituted or substituted by at least one group E or a $C_1$-$C_{14}$heteroaryl group which is unsubstituted or substituted by at least one group E,

or at least two adjacent residues $R^{31}$, $R^{32}$, $R^{33}$, $R^{34}$, $R^{35}$, $R^{36}$, $R^{37}$ and $R^{38}$ of at least two adjacent groups $CR^{31}$, $CR^{32}$, $CR^{33}$, $CR^{34}$, $CR^{35}$, $CR^{36}$, $CR^{37}$ and $CR^{38}$ together may form a ring of formula (VI) as defined above.

Most preferably, $R^{31}$ to $R^{38}$ and $R^{39}$ to $R^{42}$ are independently of each other H, a $C_1$-$C_8$alkyl group which is unsubstituted or substituted by at least one group E and/or interrupted by at least one group D, a $C_6$-$C_{10}$aryl group which is unsubstituted or substituted by at least one group E or a $C_1$-$C_{14}$heteroaryl group which is unsubstituted or substituted by at least one group E,

or at least two adjacent residues $R^{31}$, $R^{32}$, $R^{33}$, $R^{34}$, $R^{35}$, $R^{36}$, $R^{37}$ and $R^{38}$ of at least two adjacent groups $CR^{31}$, $CR^{32}$, $CR^{33}$, $CR^{34}$, $CR^{35}$, $CR^{36}$, $CR^{37}$ and $CR^{38}$ together may form a ring of formula (VI) as defined above.

Further most preferably, $R^{31}$ to $R^{38}$ and $R^{39}$ to $R^{42}$ are independently of each other H, a $C_1$-$C_8$alkyl group which is unsubstituted, for example methyl or tert.-butyl, a $C_6$-$C_{10}$aryl group which is unsubstituted, for example phenyl, or a $C_4$-$C_{10}$heteroaryl group which is unsubstituted, for example carbazolyl.

or at least two adjacent residues $R^{31}$, $R^{32}$, $R^{33}$, $R^{34}$, $R^{35}$, $R^{36}$, $R^{37}$ and $R^{38}$ of at least two adjacent groups $CR^{31}$, $CR^{32}$, $CR^{33}$, $CR^{34}$, $CR^{35}$, $CR^{36}$, $CR^{37}$ and $CR^{38}$ together may form a ring of formula (VI) as defined above.

Even further most preferably, $R^{31}$, $R^{32}$, $R^{34}$, $R^{35}$, $R^{37}$ and $R^{38}$ and $R^{39}$ to $R^{42}$ are H, and $R^{33}$ and $R^{36}$ are independently of each other as defined above,

or at least two adjacent residues $R^{31}$, $R^{32}$, $R^{33}$, $R^{34}$, $R^{35}$, $R^{36}$, $R^{37}$ and $R^{38}$ of at least two adjacent groups $CR^{31}$, $CR^{32}$, $CR^{33}$, $CR^{34}$, $CR^{35}$, $CR^{36}$, $CR^{37}$ and $CR^{38}$ together may form a ring of formula (VI) as defined above.

Preferably, $R^{16'}$ and $R^{17'}$ are each independently a $C_6$-$C_{10}$aryl group which is unsubstituted or substituted by at least one group E or a $C_2$-$C_{13}$heteroaryl group which is unsubstituted or substituted by at least one group E, more preferably a $C_6$-$C_{10}$aryl group which is unsubstituted or a $C_2$-$C_{13}$heteroaryl group which is unsubstituted, most preferably phenyl.

Preferably, $R^{18'}$ and $R^{19'}$ are each independently hydrogen, a $C_1$-$C_{18}$alkyl group which is unsubstituted or substituted by at least one group E, a $C_6$-$C_{10}$aryl group which is unsubstituted or substituted by at least one group E or a $C_2$-$C_{13}$heteroaryl group which is unsubstituted or substituted by at least one group E, more preferably a $C_1$-$C_8$alkyl group which is unsubstituted, a $C_6$-$C_{10}$aryl group which is unsubstituted or a $C_2$-$C_{13}$heteroaryl group which is unsubstituted.

Preferred groups of formula (V) are represented by the following formulae:

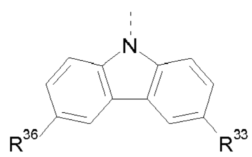 (VA-1), 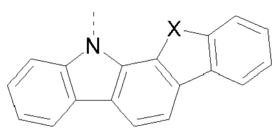 (VA-2), 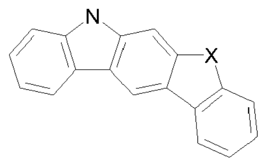 (VA-3),

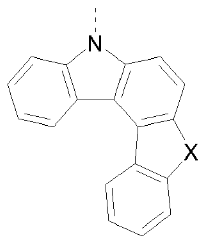 (VA-4), 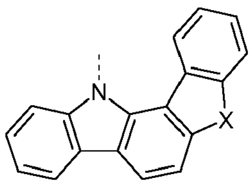 (VA-5), 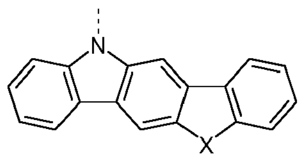 (VA-6),

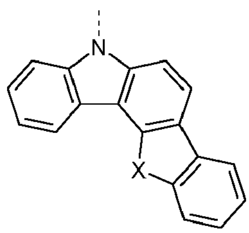 (VA-7),

wherein

$R^{33}$ and $R^{36}$ are independently of each other H, a $C_1$-$C_8$alkyl group which is unsubstituted, for example methyl or tert.-butyl, a $C_6$-$C_{10}$aryl group which is unsubstituted, for example phenyl, or a $C_4$-$C_{10}$heteroaryl group which is unsubstituted, for example carbazolyl,

X is O, S, $NR^{17'}$ or $CR^{18'}R^{19'}$,

$R^{17'}$ is a $C_6$-$C_{10}$aryl group which is unsubstituted or a $C_2$-$C_{13}$heteroaryl group which is unsubstituted, preferably phenyl, and

$R^{18'}$ and $R^{19'}$ are each independently hydrogen, a $C_1$-$C_8$alkyl group which is unsubstituted, a $C_6$-$C_{10}$aryl group which is unsubstituted or a $C_2$-$C_{13}$heteroaryl group which is unsubstituted, preferably methyl, ethyl or phenyl,

wherein
the dotted lines are bonding sites.

Most preferred groups of formula (V) are the following groups:

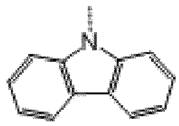 , 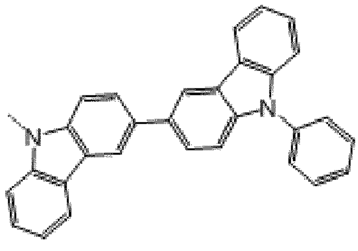 , 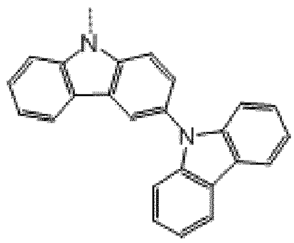 ,

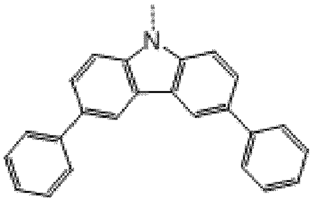 , 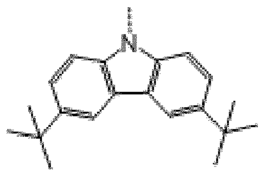 , 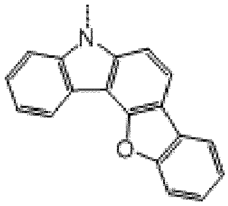 , 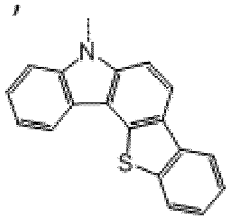 ,

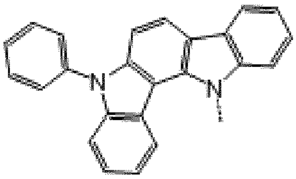 , 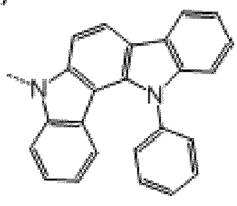 , 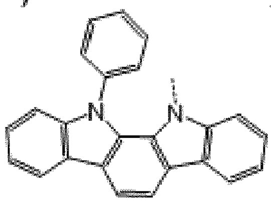 , 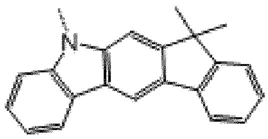

(ii) A group of formula (VII),

$$---(M)_m\text{-}NAr_1Ar_2 \qquad (VII)$$

wherein

$Ar_1$ and $Ar_2$ are each independently a $C_6$-$C_{40}$aryl group which is unsubstituted or substituted by at least one group E or a $C_1$-$C_{24}$heteroaryl group which is unsubstituted or substituted by at least one group E; or

$Ar_1$ and $Ar_2$ may be connected together to form a ring,

wherein the dotted line is a bonding site to formula (I).

Preferably, $Ar_1$ and $Ar_2$ are each independently a $C_6$-$C_{10}$aryl group which is unsubstituted or substituted by at least one group E or a $C_2$-$C_{13}$heteroaryl group which is unsubstituted or substituted by at least one group E, more preferably a $C_6$-$C_{10}$aryl group which is unsubstituted or a $C_2$-$C_{13}$heteroaryl group which is unsubstituted, most preferably phenyl.

Most preferred groups of formula (VII) are the following groups:

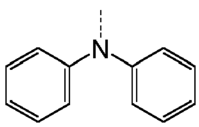 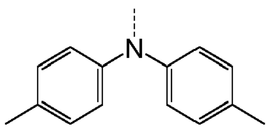 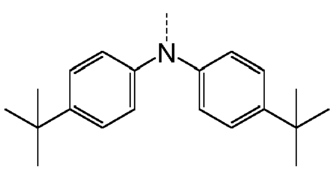 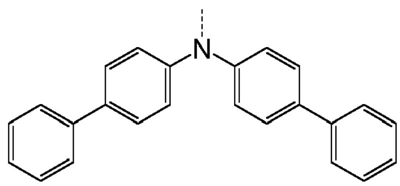

(iii) A group ---$(M)_m$-$D_1$,

preferably a group of formula (IIIA), more preferably a group of formula of formula (IIIA-1), (IIIA-2), (IIIA-3), (IIIA-4), (IIIA-5), (IIIA-6), (IIIA-7), (IIIA-8), (IIIA-9), (IIIA-10), (IIIA-11), (IIIA-12), wherein the dotted line is a bonding site to formula (I). And

(iv) a group of formula (VIII),

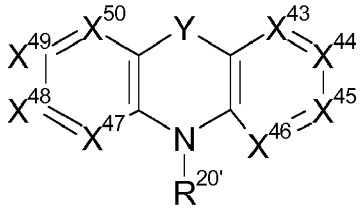

(VIII)

wherein

Y is $CR^{51}R^{52}$, O or S,
$X^{43}$ is $CR^{43}$ or N;
$X^{44}$ is $CR^{44}$ or N;
$X^{45}$ is $CR^{45}$ or N;
$X^{46}$ is $CR^{46}$ or N;
$X^{47}$ is $CR^{47}$ or N;
$X^{48}$ is $CR^{48}$ or N;
$X^{49}$ is $CR^{49}$ or N;
$X^{50}$ is $CR^{50}$ or N;

wherein

0, 1 or 2, preferably 0 or 1, more preferably 0 of the groups $X^{43}$, $X^{44}$, $X^{45}$, $X^{46}$, $X^{47}$, $X^{48}$, $X^{49}$ and $X^{50}$ are N;
$R^{43}$ to $R^{50}$ are independently of each other H, -CN, a $C_1$-$C_{25}$alkyl group which is unsubstituted or substituted by at least one group E and/or interrupted by at least one group D, a $C_6$-$C_{40}$aryl group which is unsubstituted or substituted by at least one group E, a $C_1$-$C_{24}$heteroaryl group which is unsubstituted or substituted by at least one group E, a $C_7$-$C_{25}$aralkyl group which is unsubstituted or substituted by at least one group E, a $C_5$-$C_{12}$cycloalkyl group which is unsubstituted or substituted by at least one group E, $-OR^{21}$, $-SR^{21}$, $-NR^{17}R^{18}$, $-COR^{20}$, $-COOR^{19}$, - $CONR^{17}R^{18}$, $-SiR^{22}R^{23}R^{24}$, $-POR^{25}R^{27}$, or halogen, or
at least two of the residues $R^{43}$ to $R^{50}$ if present at adjacent carbon atoms together may form at least one $C_6$-$C_{18}$aryl ring, which is unsubstituted or substituted by at least one group E or at least one $C_1$-$C_{24}$heteroaryl ring, which is unsubstituted or substituted by at least one group E, wherein $R^{20'}$ is a group $-(M)_m$---, wherein the dotted line is a bonding site to formula (I);
$R^{51}$ and $R^{52}$ are each independently hydrogen, a $C_1$-$C_{25}$alkyl group which is unsubstituted or substituted by at least one group E and/or interrupted by at least one group D, a $C_6$-$C_{40}$aryl group which is unsubstituted or substituted by at least one group E or a $C_1$-$C_{24}$heteroaryl group which is unsubstituted or substituted by at least one group E,
or $R^{51}$ and $R^{52}$ may togetherform a ring.
$X^{43}$, $X^{44}$, $X^{45}$, $X^{46}$, $X^{47}$, $X^{48}$, $X^{49}$ and $X^{50}$ are preferably $CR^{43}$, $CR^{44}$, $CR^{45}$, $CR^{46}$, $CR^{47}$, $CR^{48}$, $CR^{49}$ and $CR^{50}$.

Preferably, $R^{43}$ to $R^{50}$ are independently of each other H, a $C_1$-$C_8$alkyl group which is unsubstituted or substituted by at least one group E and/or interrupted by at least one group D, a $C_6$-$C_{10}$aryl group which is unsubstituted or substituted by at least one group E or a $C_2$-$C_{13}$heteroaryl group which is unsubstituted or substituted by at least one group E, more preferably H, a $C_1$-$C_8$alkyl group which is unsubstituted or a $C_6$-$C_{10}$aryl group which is unsubstituted, most preferably H.
Preferably, $R^{51}$ and $R^{52}$ are each independently a $C_1$-$C_8$alkyl group which is unsubstituted or substituted by at least one group E and/or interrupted by at least one group D, a $C_6$-$C_{10}$aryl group which is unsubstituted or substituted by at least one group E or a $C_2$-$C_{13}$heteroaryl group which is unsubstituted or substituted by at least one group E,
or
$R^{51}$ and $R^{52}$ may togetherform a five or six membered ring.
Most preferably, $R^{51}$ and $R^{52}$ are each independently a $C_1$-$C_8$alkyl group which is unsubstituted or a $C_6$-$C_{10}$aryl group which is unsubstituted.

Most preferred groups of formula (VIII) are represented by formula (VIIIA)

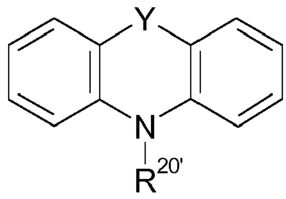

(VIIIA)

wherein

Y is $CR^{51}R^{52}$, O or S,

$R^{51}$ and $R^{52}$ are each independently a $C_1$-$C_8$alkyl group which is unsubstituted or substituted by at least one group E and/or interrupted by at least one group D, a $C_6$-$C_{10}$aryl group which is unsubstituted or substituted by at least one group E or a $C_2$-$C_{13}$heteroaryl group which is unsubstituted or substituted by at least one group E,

or

$R^{51}$ and $R^{52}$ may togetherform a five or six membered ring;

preferably a $C_1$-$C_8$alkyl group which is unsubstituted or a $C_6$-$C_{10}$aryl group which is unsubstituted,

$R^{20'}$ is a group -(M)$_m$---, preferably a bonding site, i.e. m is 0, wherein the dotted line is a bonding site to formula (I).

Most preferred groups of formula (VIII) are the following groups:

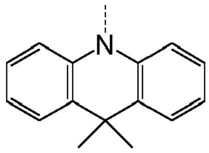 , 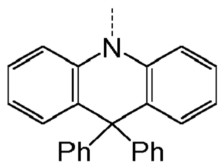 , 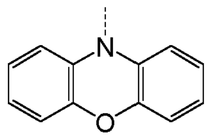 , 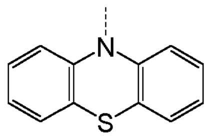

The groups and residues D, E, M, m, $R^{17}$ to $R^{25}$ and $R^{27}$ in the groups mentioned under (i), (ii), (iii) and (iv) are defined above.

Preferably, $D^2$ is a group (i), i.e. $D^2$ is a group of formula (V), more preferably a group of formula (VA-1), (VA-2), (VA-3), (VA-4), (VA-5), (VA-6) or (VA-7).

A particularly preferred compound of formula (I) is the compound of formula (IAA-1), represented by a compound of the following formula

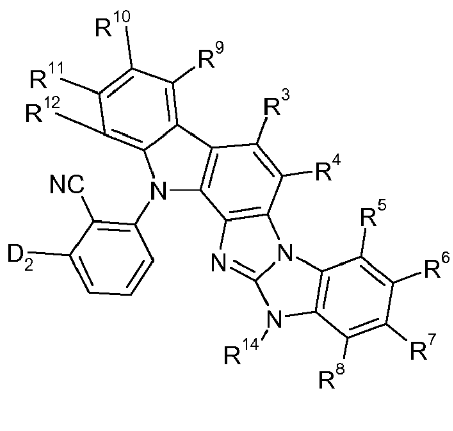

wherein

$D^2$ is a group of formula (V),

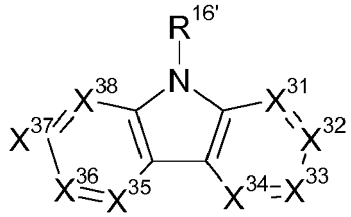

(V).

Preferred groups of formula (V) and preferred residues $R^3$ to $R^{12}$ and $R^{14}$ are mentioned above. Most preferably, $R^3$ to $R^{12}$ are hydrogen and $R^{14}$ is phenyl and the group of formula (V) is a group of formula (VA-1), (VA-2), (VA-3), (VA-4), (VA-5), (VA-6) or (VA-7).

Examples for preferred compounds of formula (I) are the following compounds:

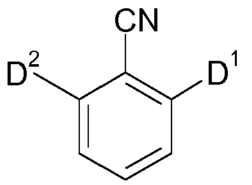

(IAA-1),

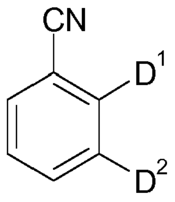

(IAA-2),

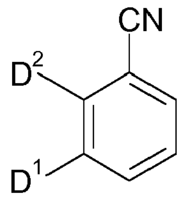

(IAA-3),

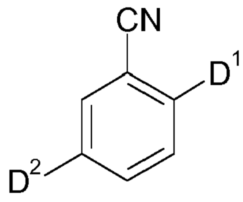

(IAA-4),

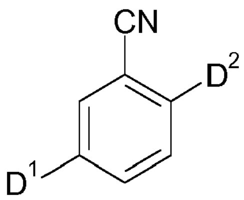

(IAA-5),

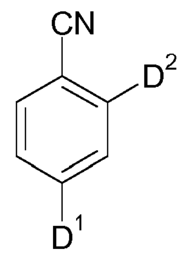

(IAA-6), and

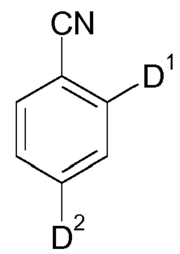

(IAA-7),

wherein $D^1$ is selected from the following groups (IIIA-1), (IIIA-2), (IIIA-3), (IIIA-4), (IIIA-5), (IIIA-6), (IIIA-7), (IIIA-8), (IIIA-9), (IIIA-10), (IIIA-11) and (IIIA-12), preferably (IIIA-2), (IIIA-10) or (IIIA-11), more preferably (IIIA-2);
wherein $R^1$ to $R^{12}$ in the groups (IIIA-1), (IIIA-2), (IIIA-3), (IIIA-4), (IIIA-5), (IIIA-6), (IIIA-7), (IIIA-8), (IIIA-9), (IIIA-10), (IIIA-11) and (IIIA-12) are H, $R^{14}$ is phenyl and $R^{15'}$ is a group $-(M)_m$---, wherein m is 0, i.e. the group $-(M)_m$--- is a bonding site; most preferred groups $D^1$ are therefore:

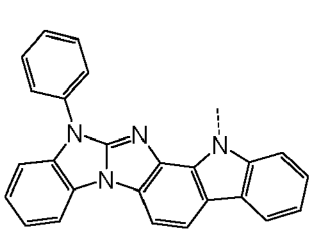

,

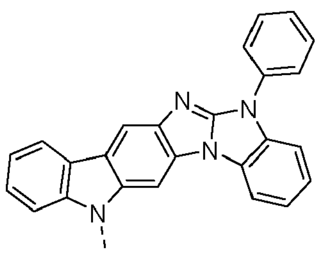

,

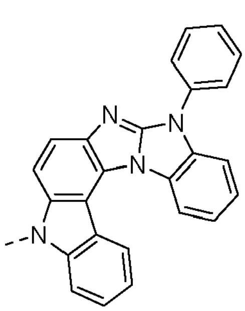

,

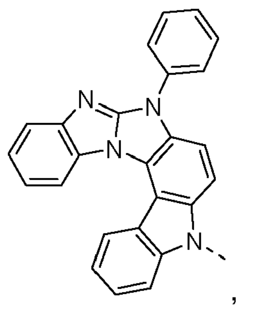

,

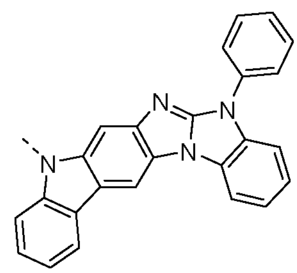

,

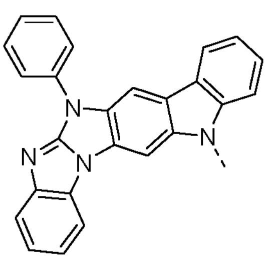

,

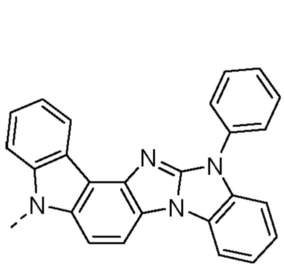

,

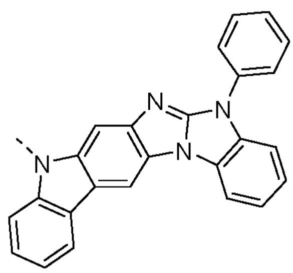

,

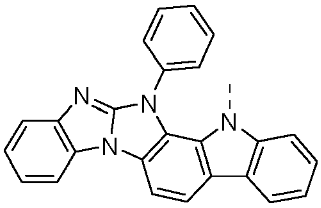,
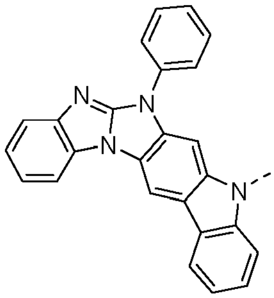,
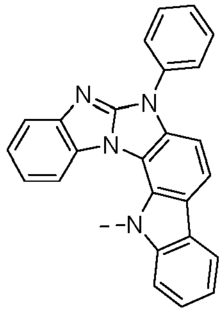

and

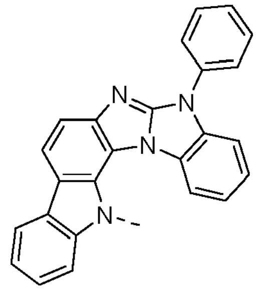,

wherein the dotted lines are bonding sites. and
$D^2$ is one of the following groups:

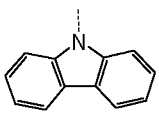,
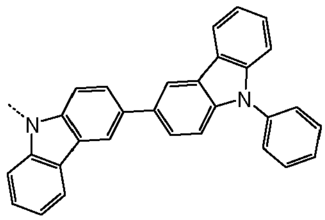,
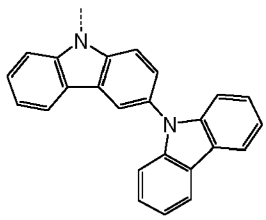,
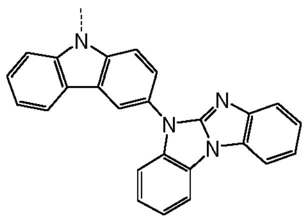,

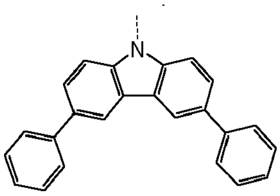,
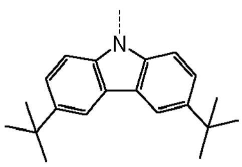,
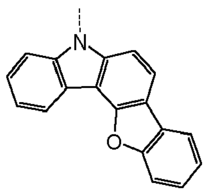,
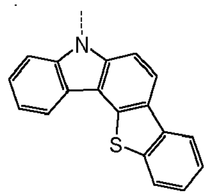,

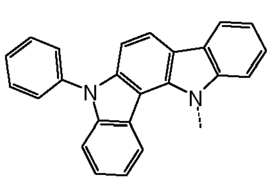,
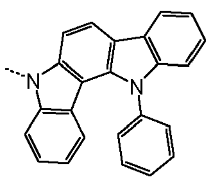,
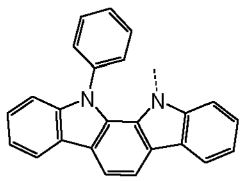,
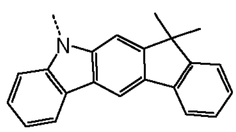,
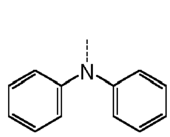

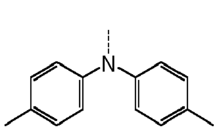
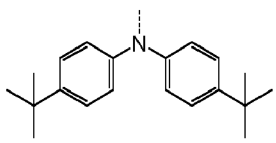
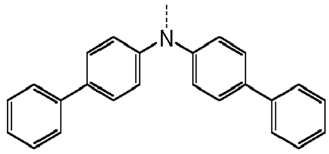,

(IIIA-1), (IIIA-2), (IIIA-3), (IIIA-4), (IIIA-5), (IIIA-6), (IIIA-7), (IIIA-8), (IIIA-9), (IIIA-10), (IIIA-11), (IIIA-12),

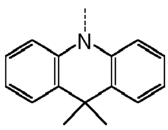,

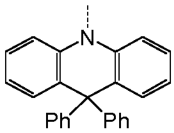, 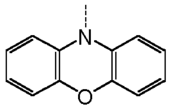, or 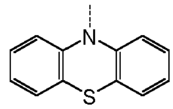,

preferably

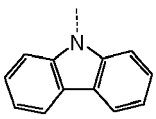, 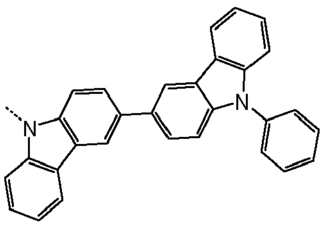,

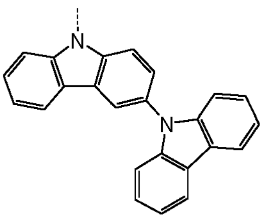, 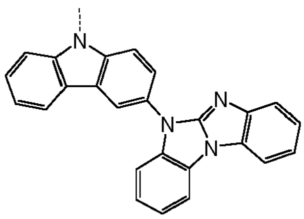, 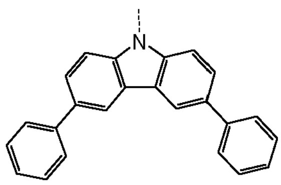, 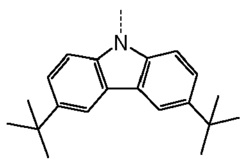,

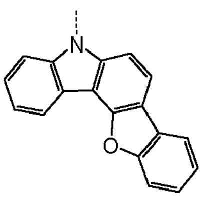, 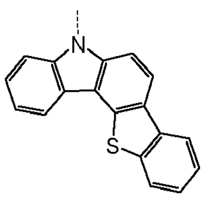, 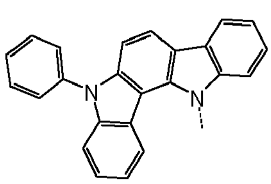, 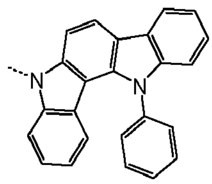, 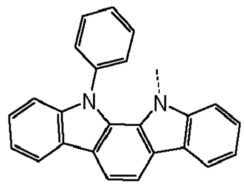,

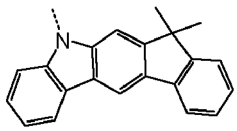.

Most preferred are compounds of formula (IAA-1), (IAA-2), (IAA-3), (IAA-4), (IAA-5), (IAA-6) and (IAA-7) as defined above, wherein $D^1$ and $D^2$ are defined in the following table:

| number. | $D^1$ | $D^2$ |
|---|---|---|
| a-1 | 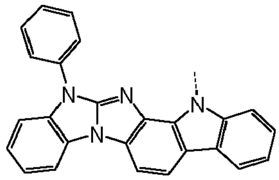 | 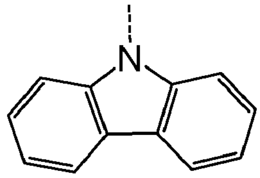 |
| a-2 | 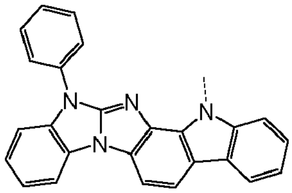 | 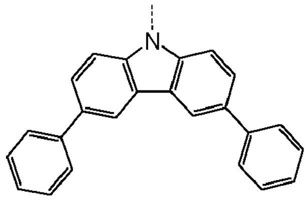 |
| a-3 | 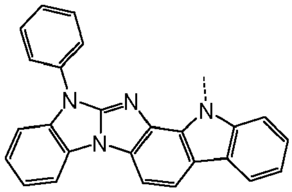 | 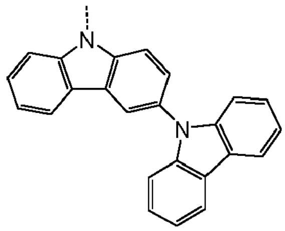 |
| a-4 | 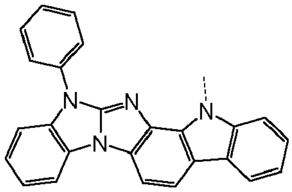 | 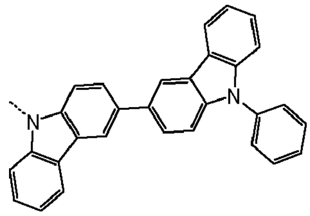 |
| a-5 | 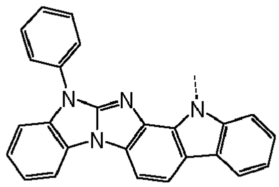 | 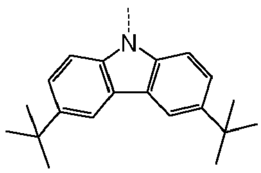 |
| a-6 | 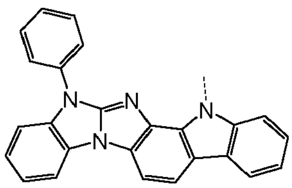 | 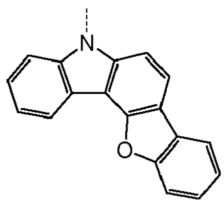 |
| a-7 | 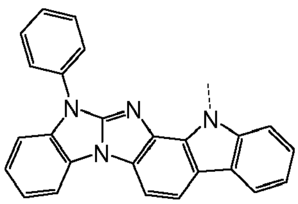 | 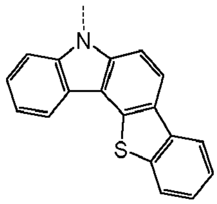 |

(continued)

| number. | D1 | D2 |
|---|---|---|
| a-8 | 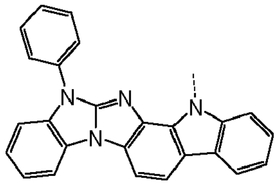 | 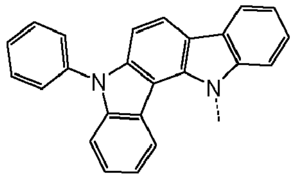 |
| a-9 | 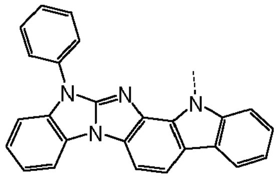 | 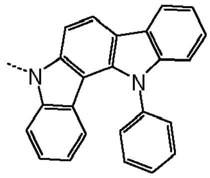 |
| a-10 | 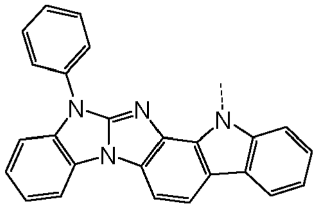 | 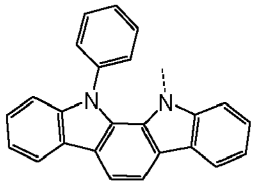 |
| a-11 | 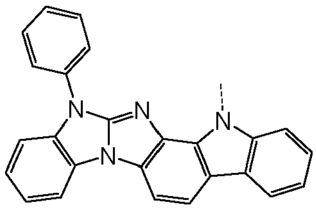 | 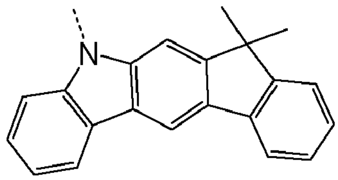 |
| a-12 | 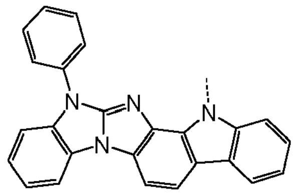 | 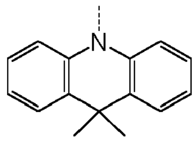 |
| a-13 | 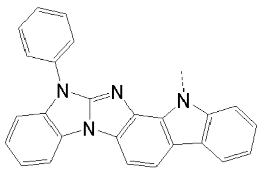 | 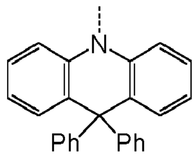 |
| a-14 | 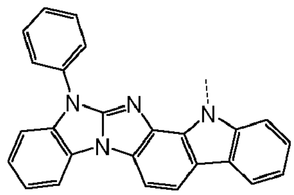 | 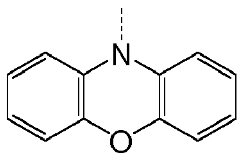 |

(continued)

| number. | $D^1$ | $D^2$ |
|---|---|---|
| a-15 | 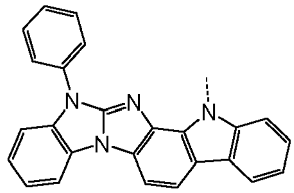 | 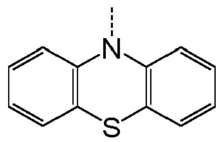 |
| a-16 | 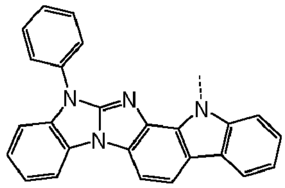 | 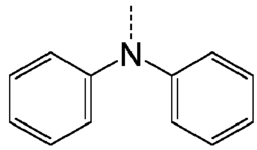 |
| a-17 | 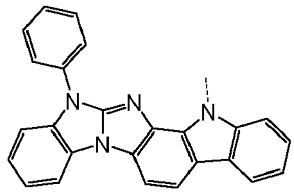 | 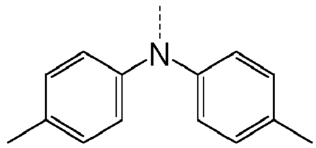 |
| a-18 | 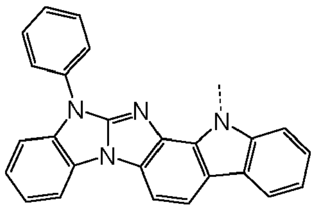 | 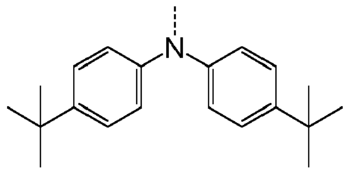 |
| a-19 | 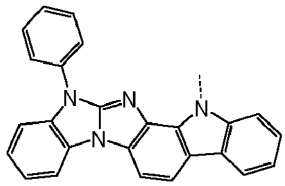 | 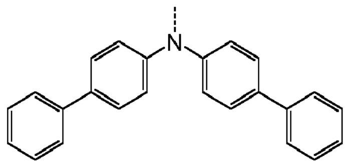 |
| a-20 | 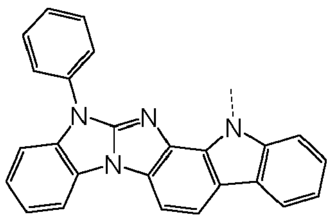 | 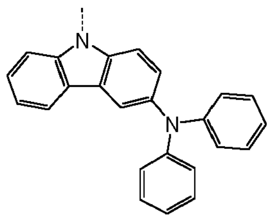 |
| a-21 | 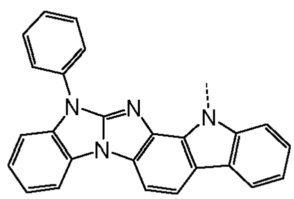 | 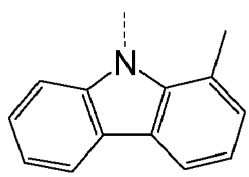 |

(continued)

| number. | $D^1$ | $D^2$ |
|---|---|---|
| a-22 | 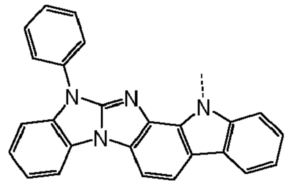 | 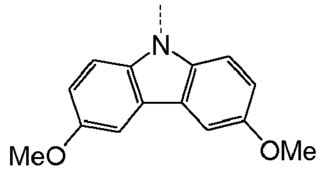 |
| a-23 | 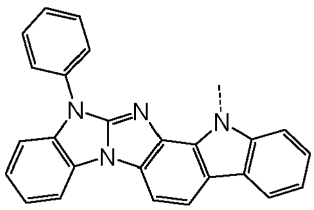 | 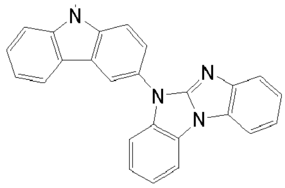 |

More preferred compounds of formula (I) are compounds (IAA-1)-(a-1), (IAA-1)-(a-2), (IAA-1)-(a-3), (IAA-1)-(a-4), (IAA-1)-(a-5), (IAA-1)-(a-6), (IAA-1)-(a-7), (IAA-1)-(a-8), (IAA-1)-(a-9), (IAA-1)-(a-10), (IAA-1)-(a-11), (IAA-1)-(a-23), (IAA-2)-(a-1), (IAA-2)-(a-2), (IAA-2)-(a-3), (IAA-2)-(a-4), (IAA-2)-(a-5), (IAA-2)-(a-6), (IAA-2)-(a-7), (IAA-2)-(a-8), (IAA-2)-(a-9), (IAA-2)-(a-10), (IAA-2)-(a-11), (IAA-2)-(a-23), (IAA-3)-(a-1), (IAA-3)-(a-2), (IAA-3)-(a-3), (IAA-3)-(a-4), (IAA-3)-(a-5), (IAA-3)-(a-6), (IAA-3)-(a-7), (IAA-3)-(a-8), (IAA-3)-(a-9), (IAA-3)-(a-10), (IAA-3)-(a-11), (IAA-3)-(a-23), (IAA-4)-(a-1), (IAA-4)-(a-2), (IAA-4)-(a-3), (IAA-4)-(a-4), (IAA-4)-(a-5), (IAA-4)-(a-6), (IAA-4)-(a-7), (IAA-4)-(a-8), (IAA-4)-(a-9), (IAA-4)-(a-10), (IAA-4)-(a-11), (IAA-4)-(a-23), (IAA-5)-(a-1), (IAA-5)-(a-2), (IAA-5)-(a-3), (IAA-5)-(a-4), (IAA-5)-(a-5), (IAA-5)-(a-6), (IAA-5)-(a-7), (IAA-5)-(a-8), (IAA-5)-(a-9), (IAA-5)-(a-10), (IAA-5)-(a-11), (IAA-5)-(a-23), (IAA-6)-(a-1), (IAA-6)-(a-2), (IAA-6)-(a-3), (IAA-6)-(a-4), (IAA-6)-(a-5), (IAA-6)-(a-6), (IAA-6)-(a-7), (IAA-6)-(a-8), (IAA-6)-(a-9), (IAA-6)-(a-10), (IAA-6)-(a-11), (IAA-6)-(a-23), (IAA-7)-(a-1), (IAA-7)-(a-2), (IAA-7)-(a-3), (IAA-7)-(a-4), (IAA-7)-(a-5), (IAA-7)-(a-6), (IAA-7)-(a-7), (IAA-7)-(a-8), (IAA-7)-(a-9), (IAA-7)-(a-10), (IAA-7)-(a-11) and (IAA-7)-(a-23).

Most preferred compounds of formula (I) are compounds (IAA-1)-(a-1), (IAA-1)-(a-2), (IAA-1)-(a-3), (IAA-1)-(a-4), (IAA-1)-(a-5), (IAA-1)-(a-6), (IAA-1)-(a-7), (IAA-1)-(a-8), (IAA-1)-(a-9), (IAA-1)-(a-10) and (IAA-1)-(a-11).

Further exemplary embodiments for compounds of formula (I) are the following compounds:

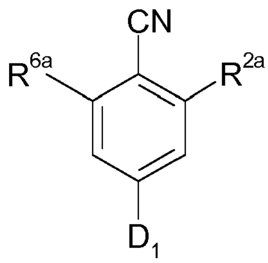

(IAB)

wherein $D^1$ is selected from the following groups

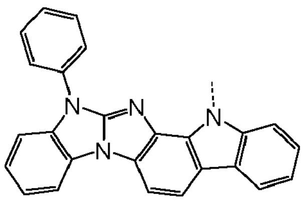

,

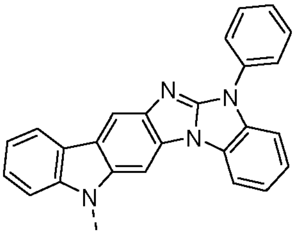, 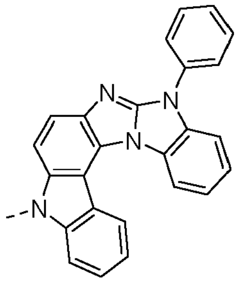, 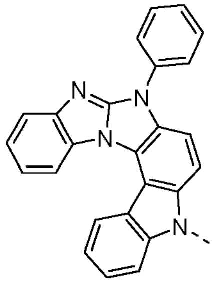, 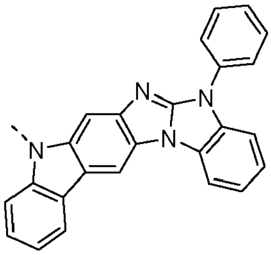,

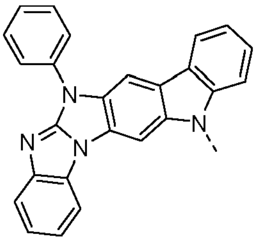, 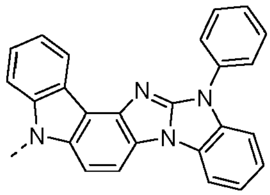, 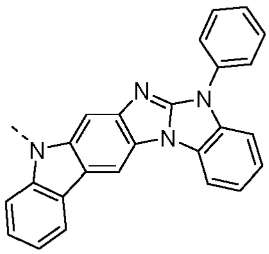, 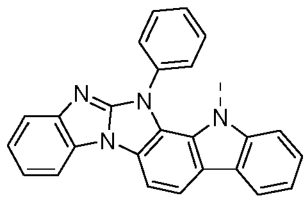,

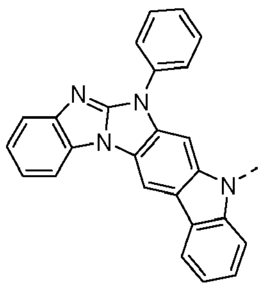, 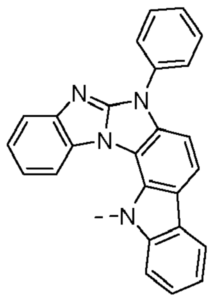 and 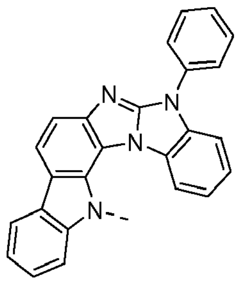;

and

$R^{2a}$, $R^{6a}$ are each independently H, a $C_1$-$C_8$alkyl group which is unsubstituted, a $C_6$-$C_{10}$aryl group which is unsubstituted or a $C_1$-$C_{24}$heteroaryl group which is unsubstituted, preferably phenyl or H, more preferably phenyl.

Most preferred are compounds of formula (IAB) as defined above, wherein $D_1$ and $R^{2a}$, $R^{6a}$ are defined in the following table:

| number | $D^1$ | $R^{2a}$, $R^{6a}$ |
|---|---|---|
| b-1 | 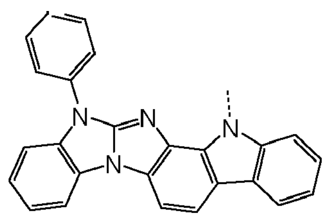 | Phenyl |
| b-2 | 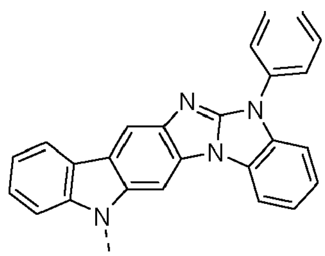 | Phenyl |

(continued)

| number | $D^1$ | $R^{2a}$, $R^{6a}$ |
|---|---|---|
| b-3 | 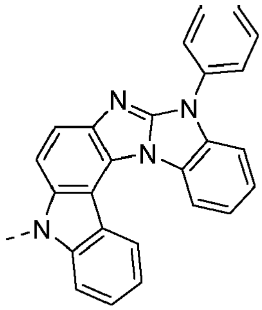 | Phenyl |
| b-4 | 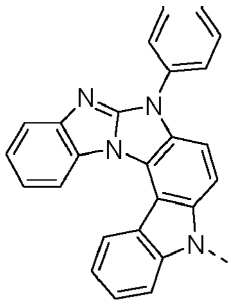 | Phenyl |
| b-5 | 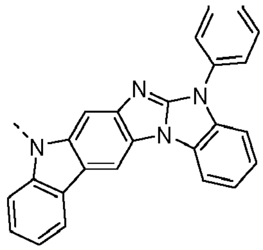 | Phenyl |
| b-6 | 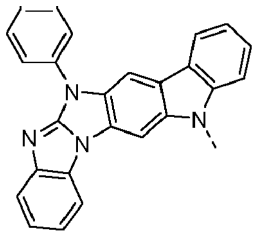 | Phenyl |
| b-7 | 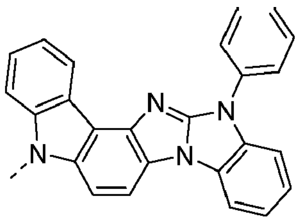 | Phenyl |
| b-8 | 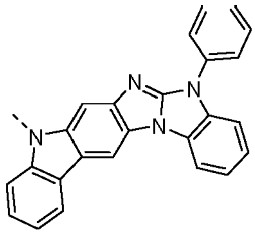 | Phenyl |

(continued)

| number | $D^1$ | $R^{2a}$, $R^{6a}$ |
|---|---|---|
| b-9 | 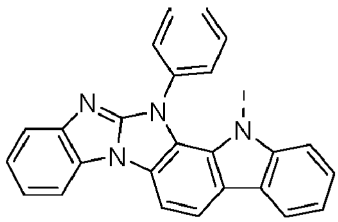 | Phenyl |
| b-10 | 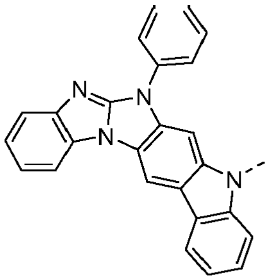 | Phenyl |
| b-11 | 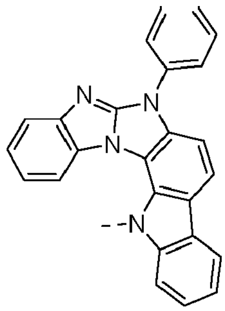 | Phenyl |
| b-12 | 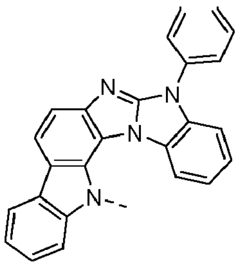 | Phenyl |

Further exemplary embodiments for compounds of formula (I) are the following compounds:

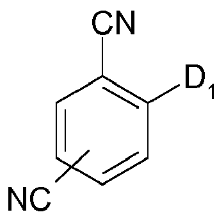 (IAC), preferably 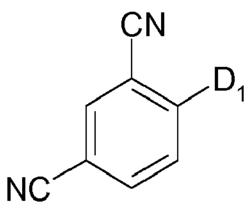 (IAC-1),

wherein
$D^1$ is selected from the following groups

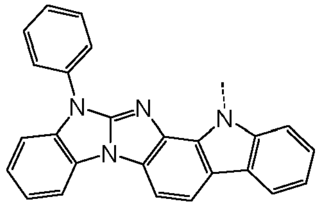,
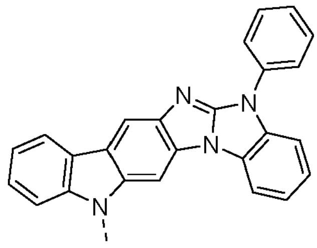,

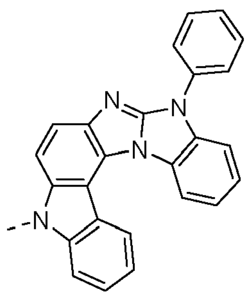,
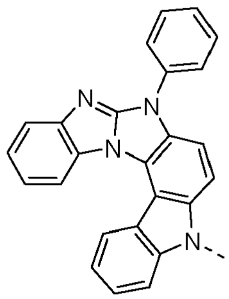,
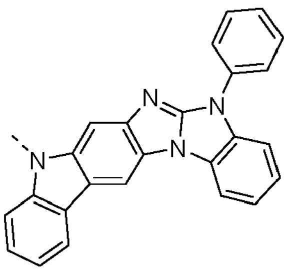,
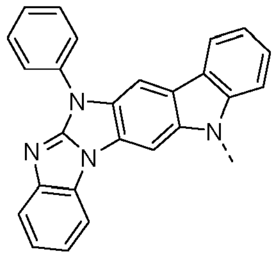,

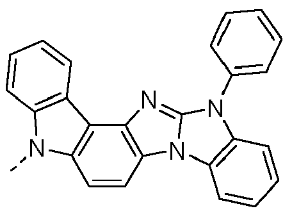,
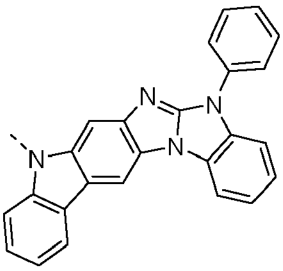,
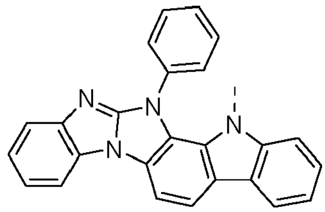,
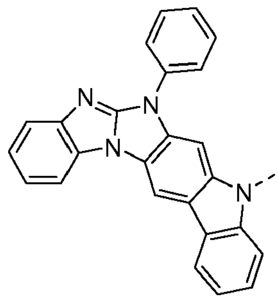,

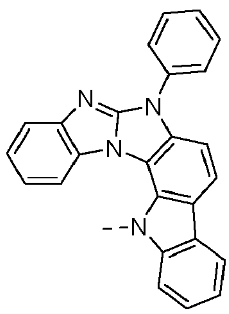.

*Preparation of the compounds of formula (I)*

The compounds of formula (I) can for example be prepared by reacting at least one intermediate (IIIa) or (III'a) and optionally at least one intermediate (V*), (VII*) or (VIII*)

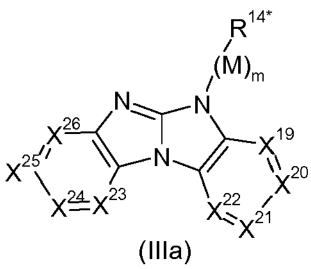
(IIIa)

or

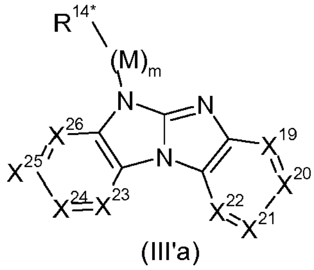
(III'a)

,

wherein $X^{19}$ to $X^{26}$, m and M are defined as in the group of formula (III) and $R^{14*}$ is H, a $C_1$-$C_{25}$alkyl group which is unsubstituted or substituted by at least one group E and/or interrupted by at least one group D, a $C_6$-$C_{40}$aryl group which is unsubstituted or substituted by at least one group E or a $C_1$-$C_{24}$heteroaryl group which is unsubstituted or substituted by at least one group E, preferably H, a $C_1$-$C_8$alkyl group which is unsubstituted or substituted by at least one group E and/or interrupted by at least one group D, a $C_6$-$C_{10}$aryl group which is unsubstituted or substituted by at least one group E or a $C_2$-$C_{13}$heteroaryl group which is unsubstituted or substituted by at least one group E, more preferably H, a $C_1$-$C_8$alkyl group which is unsubstituted, a $C_6$-$C_{10}$aryl group which is unsubstituted or a $C_2$-$C_{13}$heteroaryl group which is unsubstituted, most preferably H;

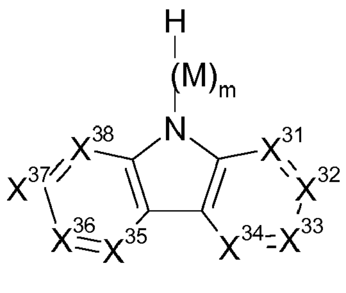
(V*),

wherein $X^{31}$ to $X^{38}$, M and m are defined as in the group of formula (V),
H-$(M)_m$-$NAr_1Ar_2$ (VII*), wherein $Ar^1$, $Ar^2$, M and m are defined as in the group of formula (VII), or

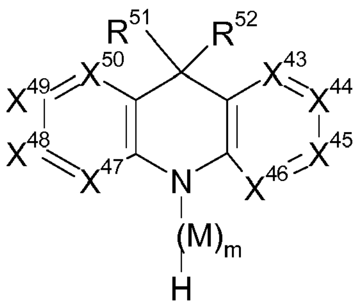
(VIII*),

wherein $X^{43}$ to $X^{50}$, $R^{51}$, $R^{52}$, M and m are defined as in the group of formula (VIII), successively with a compound of formula

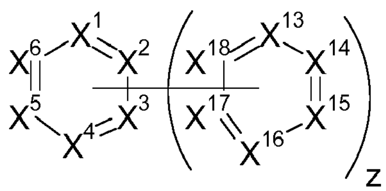
(I')

wherein

z is 0 or 1, preferably 0;
$X^1$ is $CR^{1a}$ or N;
$X^2$ is $CR^{2a}$ or N;
$X^3$ is $CR^{3a}$ or N;
$X^4$ is $CR^{4a}$ or N;
$X^5$ is $CR^{5a}$ or N;
$X^6$ is $CR^{6a}$ or N;

wherein

0, 1 or 2, preferably 0 or 1, more preferably 0 of the groups $X^1$, $X^2$, $X^3$, $X^4$, $X^5$ and $X^6$ are N;
$R^{1a}$, $R^{2a}$, $R^{3a}$, $R^{4a}$, $R^{5a}$ and $R^{6a}$ are each independently CN, $R^A$ or F;

wherein

1, 2, 3, 4 or 5, preferably 1, 2, 3 or 4, more preferably 1 or 2 of the residues $R^{1a}$, $R^{2a}$, $R^{3a}$, $R^{4a}$, $R^{5a}$ and $R^{6a}$ are CN;
0, 1 or 2 of the residues $R^{1a}$, $R^{2a}$, $R^{3a}$, $R^{4a}$, $R^{5a}$ and $R^{6a}$ are $R^A$;
1, 2 or 3 preferably 1 or 2 of the residues $R^{1a}$, $R^{2a}$, $R^{3a}$, $R^{4a}$, $R^{5a}$ and $R^{6a}$ are F;
$X^{13}$ is $CR^{13a}$ or N;
$X^{14}$ is $CR^{14a}$ or N;
$X^{15}$ is $CR^{15a}$ or N;
$X^{16}$ is $CR^{16a}$ or N;
$X^{17}$ is $CR^{17a}$ or N;
$X^{18}$ is $CR^{18a}$ or N;

wherein

0, 1 or 2, preferably 0 or 1, more preferably 0 of the groups $X^{13}$, $X^{14}$, $X^{15}$, $X^{16}$, $X^{17}$ and $X^{18}$ are N;
$R^{13a}$, $R^{14a}$, $R^{15a}$, $R^{16a}$, $R^{17a}$ and $R^{18a}$ are each independently CN, $R^A$ or F;

wherein

1, 2, 3 or 4, preferably 1 or 2 of the residues $R^{13a}$, $R^{14a}$, $R^{15a}$, $R^{16a}$, $R^{17a}$ and $R^{18a}$ are CN;
0, 1 or 2 of the residues $R^{13a}$, $R^{14a}$, $R^{15a}$, $R^{16a}$, $R^{17a}$ and $R^{18a}$ are $R^A$;
1 or 2 of the residues $R^{13a}$, $R^{14a}$, $R^{15a}$, $R^{16a}$, $R^{17a}$ and $R^{18a}$ are F;

and the groups and residues are defined as in formula (I), in the presence of a base, preferably, $K_3PO_4$, $K_2CO_3$, $Cs_2CO_3$, NaOtBu or NaH.

The reaction may be carried out in a solvent. Suitable solvents are dimethylsulfoxide (DMSO),dimethylformamide (DMF), dimethylacetamide (DMA) and N-methyl-2-pyrrolidone (NMP).

Suitable reaction conditions are described in the examples.

The condensed benzimidazolo[1,2-a]benzimidazole intermediate (IIia) or (III'a) may for example prepared as follows:

(ia) Preparation of a nitro group comprising intermediate (C) by reacting a ortho-nitro halobenzene (A) with a benzimidazole derivative (B)

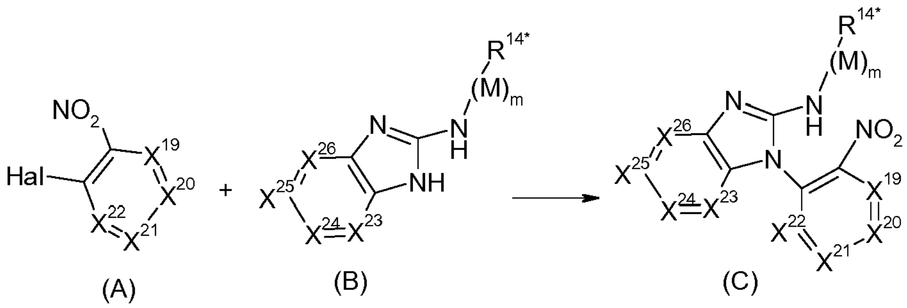

(A) (B) (C)

or

(I'a) Preparation of a nitro group comprising intermediate (C') by reacting a ortho-nitro halobenzene (A) with a benzimidazole derivative (B')

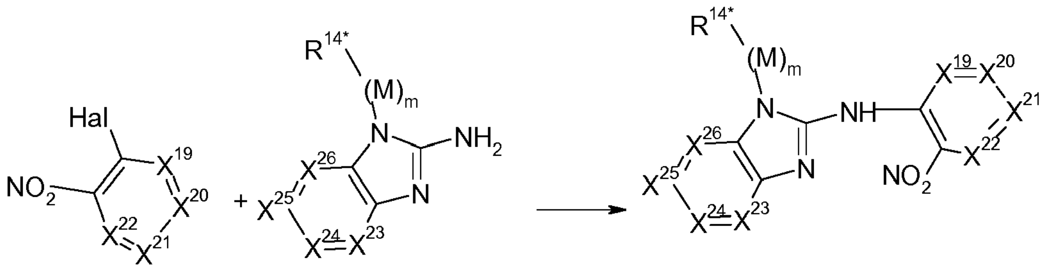

(A) (B') (C')

and

(ib) Cyclization of the intermediate (C) to the benzimidazolo[1,2-a]benzimidazole intermediate (IIIa)

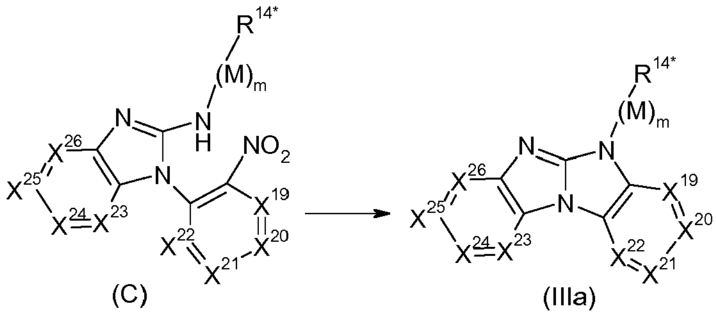

(C) (IIIa)

or

(I'b) Cyclization of the intermediate (C') to the benzimidazolo[1,2-a]benzimidazole intermediate (III'a)

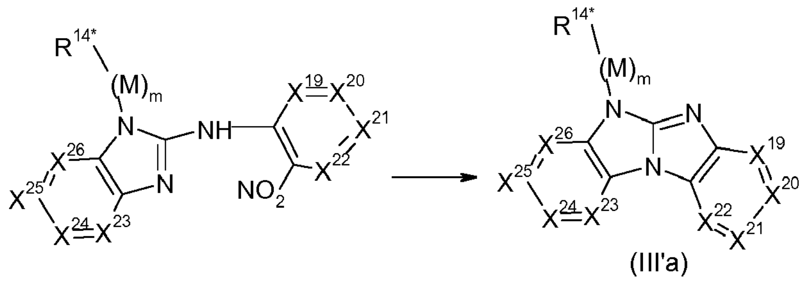

(III'a)

(C')

wherein

Hal is F, Cl or Br, preferably F or Cl, more preferably F;

$X^{19}$ to $X^{26}$, m and M are defined as in the group of formula (III), and

$R^{14*}$ is H, a $C_1$-$C_{25}$alkyl group which is unsubstituted or substituted by at least one group E and/or interrupted by at least one group D, a $C_6$-$C_{40}$aryl group which is unsubstituted or substituted by at least one group E or a $C_1$-$C_{24}$heteroaryl group which is unsubstituted or substituted by at least one group E, preferably H, a $C_1$-$C_8$alkyl group which is unsubstituted or substituted by at least one group E and/or interrupted by at least one group D, a $C_6$-$C_{10}$aryl group which is unsubstituted or substituted by at least one group E or a $C_2$-$C_{13}$heteroaryl group which is unsubstituted or substituted by at least one group E, more preferably H, a $C_1$-$C_8$alkyl group which is unsubstituted, a $C_6$-$C_{10}$aryl group which is unsubstituted or a $C_2$-$C_{13}$heteroaryl group which is unsubstituted, most preferably H.

**Device**

TADF (thermally activated delayed fluorescence) generally involves an emitter system of emitter and host and

optionally a third material. The compounds of formula (I) are useful for organic electroluminescence devices (organic EL devices), preferably as TADF host or as TADF emitter in a TADF emitter system. In case of a TADF emitter, one or more host materials, usually one host material, is used and the TADF emitter emits light. In case of a TADF host, the TADF host material transfers excitons to one or more, usually one, fluorescence emitter. Preferably, a third material is used for diluting the TADF host material.

The present invention therefore relates to an organic electroluminescence device comprising at least one compound of formula (I) according to the present application.

The compound of formula (I) is capable of prolonging a lifetime, improving a luminous efficiency, and/or inhibiting a roll-off when driven at a high current density of organic electroluminescence devices comprising the same.

Usually, the inventive organic electroluminescence device comprises a cathode, an anode, and a plurality of organic layers provided between the cathode and the anode, wherein at least one of the plurality of organic layers is an emitting layer, wherein at least one organic layer comprises at least one compound of formula (I) according to the present application.

Preferably, the emitting layer of the organic electroluminescence device comprises the at least one compound of formula (I) as a delayed-fluorescent emitter (TADF emitter) and/or as a host material (TADF host).

In addition to the emitting layer, the inventive organic electroluminescence device may further comprise a hole transporting layer between the anode and the emitting layer and/or an electron transporting layer between the cathode and the emitting layer. Examples of organic EL devices comprising additional layers are described below.

The compound of formula (I) is preferably used in the organic electroluminescence device, more preferably in the emitting layer, as TADF emitter materials, or TADF host materials in combination with at least one fluorescent emitter.

A further subject of the present invention is directed to an emitting layer, comprising a compound of formula (I) according to the present invention. In said embodiment a compound of formula (I) is preferably used as TADF emitter material, or TADF host material in combination with at least one fluorescent emitter.

Arrangement(s) of an organic EL device of the invention will be described below.

The organic EL device includes an anode, a cathode and an organic layer disposed therebetween. The organic layer includes one or more layers formed of an organic compound. The organic layer may further contain an inorganic compound. The organic layer of the organic EL device of the exemplary embodiment includes at least one emitting layer. For instance, the organic layer may consist of the emitting layer, or may include one of layers usable in a typical organic EL device, such as a hole injecting layer, a hole transporting layer, an electron injecting layer, an electron transporting layer, a hole blocking layer and an electron blocking layer.

**Fig. 1** schematically shows an exemplary arrangement of the organic EL device according to the exemplary embodiment. An organic EL device (1) includes a light-transmissive substrate (2), an anode (3), a cathode (4), and an organic layer (10) provided between the anode (3) and the cathode (4). The organic layer (10) include a hole injecting layer (6), a hole transporting layer (7), an emitting layer (5), an electron transporting layer (8), and an electron injecting layer (9) which are laminated on the anode (3) in this order.

### Emitting Layer

The emitting layer of the organic EL device according to the present invention comprises a first compound and a second compound, wherein at least one of the first and the second compound is a compound of formula (I) according to the present invention. The first compound is a host compound and the second compound is an emitter compound.

In the following exemplary embodiments of suitable devices according to the present invention are disclosed. It should be noted that the invention is not limited to the embodiments mentioned below. The invention may include any modification and improvement compatible with the invention.

### First Embodiment

### First Compound

The first compound in the first embodiment is a delayed fluorescent compound (TADF host).

The first compound is represented by a compound of formula (I).

### Delayed Fluorescence

Delayed fluorescence (thermally activated delayed fluorescence) is explained in " ADACHI, Chihaya, ed., "Yuki Hando-tai no Debaisu Bussei (Device Physics of Organic Semiconductors)", Kodansha, pp. 261-268 ." According to this literature, when an energy gap $\Delta E_{13}$ ($\Delta E_{S1T1}$) between the singlet state and the triplet state of a fluorescent material can be reduced, inverse energy transfer from the triplet state to the singlet state, which usually occurs at a low transition

probability, occurs at a high transition probability to cause thermally activated delayed fluorescence (TADF). Further, Fig. 10.38 in this literature illustrates an occurrence mechanism of the delayed fluorescence. The first compound of the exemplary embodiment is a compound exhibiting thermally activated delayed fluorescence caused by this mechanism. Occurrence of delayed fluorescence emission can be determined by transient PL (Photo Luminescence) measurement.

The behavior of delayed fluorescence can be analyzed based on the decay curve obtained by the transient PL measurement. The transient PL measurement is a process where a sample is irradiated with a pulse laser to be excited, and a decay behavior (transient characteristics) of PL emission after the irradiation is stopped is measured. PL emission using a TADF material is divided into an emission component from singlet excitons generated by the first PL excitation and an emission component from singlet excitons generated via triplet excitons. The lifetime of the singlet excitons generated by the first PL excitation is in a nano-second order and considerably short. Emission from these singlet excitons thus decays immediately after the irradiation with the pulse laser.

In contrast, delayed fluorescence, which is emission from the singlet excitons generated via long-life triplet excitons, decays slowly. There is thus a large difference in time between emission from the singlet excitons generated by the first PL excitation and emission from the singlet excitons generated via triplet excitons. Therefore, a luminous intensity resulting from the delayed fluorescence can be obtained.

**Fig. 2** schematically shows an exemplary device for measuring transient PL.

A transient PL measuring device 100 of the exemplary embodiment includes: a pulse laser 101 capable of emitting light with a predetermined wavelength; a sample chamber 102 configured to house a measurement sample; a spectrometer 103 configured to disperse light emitted from the measurement sample; a streak camera 104 configured to form a two-dimensional image; and a personal computer 105 configured to analyze the two-dimensional image imported thereinto. It should be noted that transient PL may be measured by a device different from one described in the exemplary embodiment.

The sample to be housed in the sample chamber 102 is prepared by forming a thin film, which is made of a matrix material doped with a doping material at a concentration of 12 mass%, on a quartz substrate.

The thus-obtained thin film sample is housed in the sample chamber 102, and is irradiated with a pulse laser emitted from the pulse laser 101 to excite the doping material. The emitted excitation light is taken in a 90-degree direction with respect to the irradiation direction of the excitation light, and is dispersed by the spectrometer 103. A two-dimensional image of the light is formed through the streak camera 104. In the thus-obtained two-dimensional image, an ordinate axis corresponds to time, an abscissa axis corresponds to wavelength, and a bright spot corresponds to luminous intensity. The two-dimensional image is taken at a predetermined time axis, thereby obtaining an emission spectrum with an ordinate axis representing luminous intensity and an abscissa axis representing wavelength. Further, the two-dimensional image is taken at a wavelength axis, thereby obtaining a decay curve (transient PL) with an ordinate axis representing the logarithm of luminous intensity and an abscissa axis representing time.

For instance, a thin film sample A was prepared using a reference compound M1 below as a matrix material and a reference compound DP1 below as a doping material, and transient PL was measured.

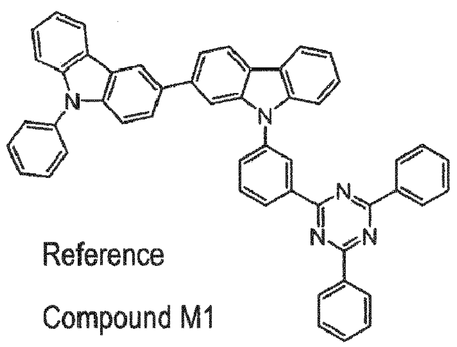

Reference Compound M1

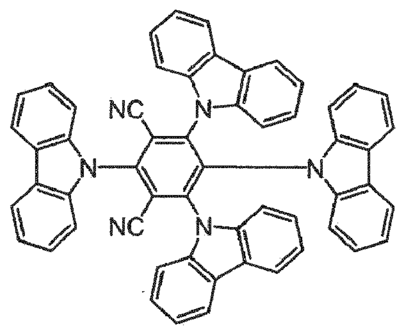

Reference Compound DP1

Respective decay curves of the thin film sample A and a thin film sample B were analyzed. The thin film sample B was prepared in the same manner as described above using a reference compound M2 below as a matrix material and the reference compound DP1 as a doping material.

**Fig. 3** shows a decay curve obtained from transient PL measured using each of the thin film samples A and B.

*In Fig. 3:*

A is thin film sample A
B is thin film sample B

t is the time in (μs)

$I_V$ is the luminous intensity in (cd) (logarithmic scale)

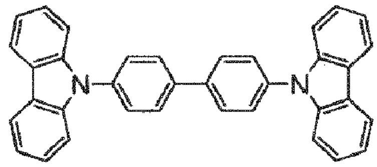

Reference Compound M2

As described above, an emission decay curve with an ordinate axis representing luminous intensity and an abscissa axis representing time can be obtained by the transient PL measurement. Based on the emission decay curve, a fluorescence intensity ratio between fluorescence emitted from a singlet state generated by photo-excitation and delayed fluorescence emitted from a singlet state generated by inverse energy transfer via a triplet state can be estimated. In a delayed fluorescent material, a ratio of the intensity of the slowly decaying delayed fluorescence to the intensity of the promptly decaying fluorescence is relatively large.

In the first embodiment, the luminescence amount of the delayed fluorescence can be obtained using the device shown in **Fig. 2**. Emission from the first compound includes: Prompt emission observed immediately when the excited state is achieved by exciting the first compound with a pulse beam (i.e., a beam emitted from a pulse laser) having an absorbable wavelength; and Delay emission observed not immediately when but after the excited state is achieved. In the exemplary embodiment, the amount of Delay emission is preferably 5% or more relative to the amount of Prompt emission.

The amount of Prompt emission and the amount of Delay emission can be obtained in the same method as a method described in "Nature 492, 234-238, 2012". The amount of Prompt emission and the amount of Delay emission may be calculated using a device different from one described in the above Literature.

For instance, a sample usable for measuring the delayed fluorescence may be prepared by codepositing the first compound and a compound TH-2 below on a quartz substrate at a ratio of the first compound being 12 mass% to form a 100-nm-thick thin film.

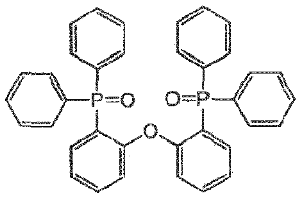

TH-2

**Second Compond**

The second compound in the first embodiment is a fluorescent compound (emitter).

The second compound preferably emits fluorescence having a main peak wavelength (occasionally referred to as an emission peak wavelength) of 430 to 780 nm (blue, green, red). The main peak wavelength means a peak wavelength of luminescence spectrum exhibiting a maximum luminous intensity among luminous spectra measured using a toluene solution where the second compound is dissolved at a concentration from $10^{-6}$ mol/l to $10^{-5}$ mol/l.

The second compound preferably emits a red, green or blue fluorescence. The second compound is preferably a material exhibiting a high emission quantum efficiency.

Suitable fluorescent emitters emitting a red, green or blue fluorescence are known to a person skilled in the art.

Examples of the fluorescent emitter include a bisarylamino naphthalene derivative, an aryl-substituted naphthalene derivative, a bisarylamino anthracene derivative, an aryl-substituted anthracene derivative, a bisarylamino pyrene derivative, an aryl-substituted pyrene derivative, a bisarylamino chrysene derivative, an aryl-substituted chrysene derivative, a bisarylamino fluoranthene derivative, an aryl-substituted fluoranthene derivative, an indenoperylene derivative, a pyrromethene boron complex compound, a compound having a pyrromethene skeleton or a metal complex thereof, a diketopyrrolopyrrole derivative, and a perylene derivative.

Further suitable fluorescence emitters are for example described in US 2016/0172601 A1, especially the compounds of formula (2) and preferred compounds described in US 2016/0172601 A1

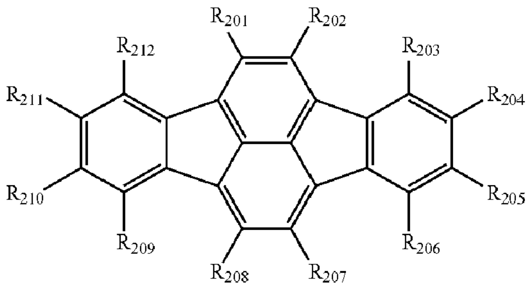

(2)

In the formula (2), $R_{201}$ to $R_{212}$ are each independently a hydrogen atom or a substituent.

When $R_{201}$ to $R_{212}$ are a substituent, the substituent is selected from the group consisting of a substituted or unsubstituted alkyl group having 1 to 30 carbon atoms, a substituted or unsubstituted aryl group having 6 to 30 ring carbon atoms, a substituted or unsubstituted heterocyclic group having 5 to 30 ring atoms, a substituted or unsubstituted alkoxy group having 1 to 30 carbon atoms, a substituted or unsubstituted aryloxy group having 6 to 30 ring carbon atoms, a substituted or unsubstituted aralkyl group having 6 to 30 ring carbon atoms, a substituted amino group, a cyano group and a halogen atom. The substituents of $R_{201}$ to $R_{212}$ may be mutually bonded to form a cyclic structure.

Further suitable fluorescence emitters are for example described in US 2016/0190469 A1, especially the compounds of formula (2) and preferred compounds described in US 2016/0190469 A1

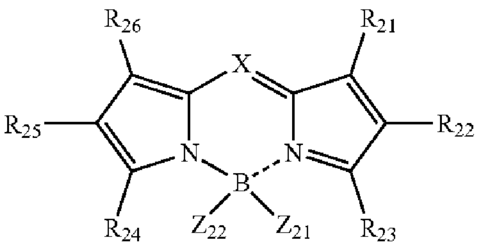

(2)

In the formula (2): X is a nitrogen atom or a carbon atom to be bonded to Y; Y is a hydrogen atom or a substituent; $R_{21}$ to $R_{26}$ are each independently a hydrogen atom or a substituent; when Y and $R_{21}$ to $R_{26}$ are substituents, the substituents are selected from the group consisting of a substituted or unsubstituted alkyl group, a substituted or unsubstituted cycloalkyl group, a substituted or unsubstituted aromatic hydrocarbon group, a substituted or unsubstituted alkoxy group, a substituted or unsubstituted alkylthio group, a substituted or unsubstituted aryloxy group, a substituted or unsubstituted arylthio group, a substituted or unsubstituted alkenyl group, a substituted or unsubstituted aralkyl group, a substituted or unsubstituted heterocyclic group, a halogen atom, a haloalkyl group, a carboxy group, an ester group, a carbamoyl group, an amino group, a nitro group, a cyano group, a silyl group, and a siloxanyl group; $Z_{21}$ and $Z_{22}$ are each independently selected from the group consisting of a halogen atom, a substituted or unsubstituted aromatic hydrocarbon group, a substituted or unsubstituted alkoxy group, and a substituted or un substituted aryloxy group.

Two or more of $R_{21}$ to $R_{26}$ in the formula (2) may be bonded to each other to form a cyclic structure.

Further suitable fluorescence emitters are for example described in WO 2017/146397 and US 20170141322, especially the compounds of formula (4),

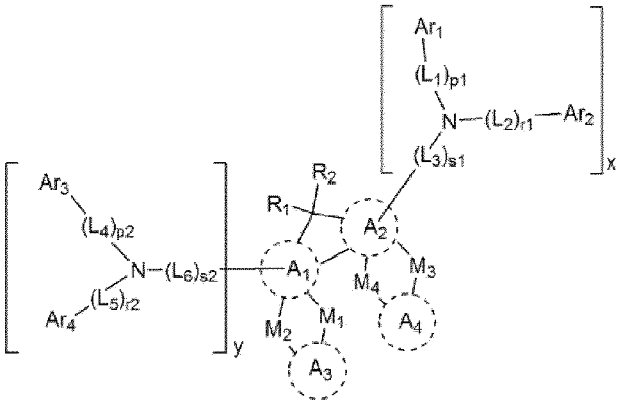

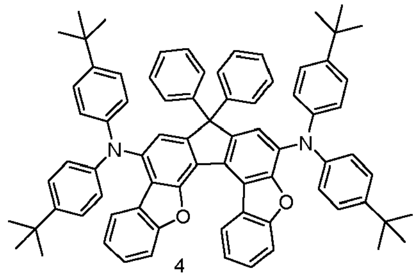

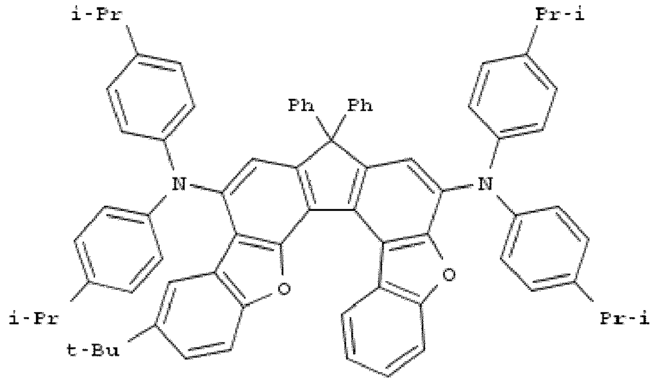

II

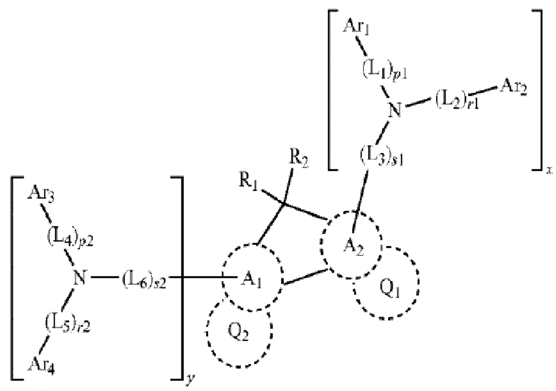

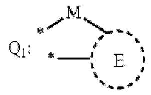

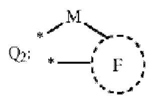

Further suitable fluorescence emitters are for example described in EP 3 109 253 A1, Advanced Materials (Weinheim, Germany), 28(14), 2777-2781; 2016, especially the compounds of formula (3)

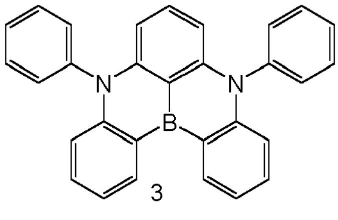

3

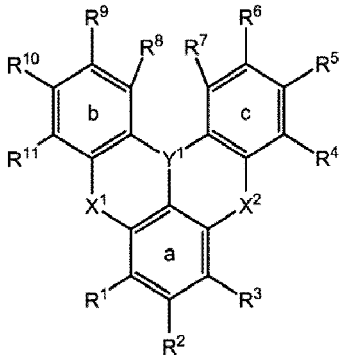

(2)

## Combination of first compound and second compound

The organic EL device in the first embodiment is capable of prolonging a lifetime, improving a luminous efficiency, and inhibiting a roll-off when driven at a high current density. The roll-off refers to a phenomenon in which a luminous efficiency is decreased when the organic EL device is driven at a high current density.

The first compound contained in the emitting layer in the exemplary embodiment is a delayed fluorescent compound, a compound of formula (I). Since the delayed fluorescent compound has a donor (electron-donating) moiety and an acceptor (electron accepting) moiety in a molecular structure, electric charges exist in a localized state in a molecule of the delayed fluorescent compound when the delayed fluorescent compound is excited. Since such a localized state of the electric charges possibly causes various side reactions, the delayed fluorescent compound having a long excited lifetime tends to exhibit a low stability especially in the excited state. Moreover, a half bandwidth of the luminescence spectrum of the delayed fluorescent compound is increased. Accordingly, it is difficult to use the delayed fluorescent compound as a luminescent material for a display unit requiring a high definition display in a large-sized TV set and the like.

Accordingly, an organic EL device including an emitting layer containing a delayed fluorescent compound and a fluorescent compound has been studied. In the organic EL device with the above arrangement, the delayed fluorescent compound is used as a sensitizer in the emitting layer and the fluorescent compound is used as the luminescent material. According to the organic EL device with the above arrangement, since the fluorescent compound emits light, it is expected that the half bandwidth of the luminescence spectrum is decreased. Moreover, since emission from the delayed fluorescent compound having a low excitation stability can be avoided, it is expected that a lifetime of the organic EL device is prolonged. Further, according to the organic EL device with this arrangement, it is theoretically expected that the same luminous efficiency as that of an organic EL device including an emitting layer containing a delayed fluorescent compound as a luminescent material is obtained.

The inventors have found that a combination of the delayed fluorescent compound that is the first compound and the fluorescent compound that is the second compound, wherein the first compound (delayed fluorescent compound (TADF host)) is a compound of formula (I) according to the present invention, can provide advantages of prolonging a lifetime, improving a luminous efficiency, and inhibiting a roll-off in a high current density region.

The organic EL device in the first embodiment preferably emits light exhibiting a peak in a wavelength range of 430 to 780 nm. In other words, the main peak wavelength of the light emitted from the organic EL device preferably falls within a range of 430 to 780 nm. When the organic EL device in the exemplary embodiment emits light, it is preferable that the second compound mainly emits light in the emitting layer (5). The wavelength range of the light emitted by the organic EL device in the first embodiment is more preferably from 430 nm to 570 nm.

It is therefore preferable that the organic EL device in the first embodiment emits blue or green fluorescence.

## TADF Mechanism

In the organic EL device of the first embodiment, the first compound is preferably a compound having a small $\Delta ST(M1)$ so that inverse intersystem crossing from the triplet energy level of the first compound to the singlet energy level thereof is easily caused by a heat energy given from the outside. An energy state conversion mechanism to perform spin exchange from the triplet state of electrically excited excitons within the organic EL device to the singlet state by inverse intersystem crossing is referred to as a TADF mechanism.

**Fig. 4** exemplarily shows a relationship in energy levels (EnL in Fig. 4 is the energy level) between the first compound ($1^{st}$) and the second compound ($2^{nd}$) in the emitting layer. In Fig. 4, S0 represents a ground state, S1(M1) represents a lowest singlet state of the first compound, T1(M1) represents a lowest triplet state of the first compound, S1(M2) represents a lowest singlet state of the second compound, and T1(M2) represents a lowest triplet state of the second compound. A dashed arrow directed from S1(M1) to S1(M2) in Fig. 4 represents Forster energy transfer from

the lowest singlet state of the first compound to the lowest singlet state of the second compound. A difference between the lowest singlet state S1 and the lowest triplet state T1 is defined as ΔST.

As shown in Fig. 4, when a compound having a small ΔST(M1) is used as the first compound, inverse intersystem crossing from the lowest triplet state T1(M1) to the lowest singlet state S1(M1) can be caused by a heat energy. Accordingly, Forster energy transfer from the lowest singlet state S1(M1) of the first compound to the lowest singlet state S1(M2) of the second compound is caused. As a result, fluorescence from the lowest singlet state S1(M2) of the second compound can be observed. It is considered that the internal quantum efficiency can be theoretically raised up to 100% also by using delayed fluorescence by the TADF mechanism.

In the first embodiment, a singlet energy S(M1) of the first compound is preferably larger than a singlet energy S(M2) of the second compound.

In the first embodiment, an energy gap T77K(M1) at 77[K] of the first compound is preferably larger than an energy gap T77K(M2) at 77[K] of the second compound. T77K(M1) is preferably 2.0 eV or more, more preferably 2.2 eV or more.

**Relationship between Triplet Energy and Energy Gap at 77 [K]**

Description will be made on a relationship between a triplet energy and an energy gap at 77 [K]. In the exemplary embodiment, the energy gap at 77 [K] is different from a typical triplet energy in some aspects.

The triplet energy is measured as follows. Firstly, a target compound for measurement is deposited on a quartz substrate to prepare a sample. Alternatively, the target compound is dissolved in an appropriate solvent to prepare a solution and the solution is encapsulated in a quartz glass pipe to prepare a sample. A phosphorescent spectrum (ordinate axis: phosphorescent luminous intensity, abscissa axis: wavelength) of the sample is measured at a low temperature (77K). A tangent is drawn to the rise of the phosphorescent spectrum on the short-wavelength side. The triplet energy is calculated by a predetermined conversion equation based on a wavelength value at an intersection of the tangent and the abscissa axis.

The first compound usable in the exemplary embodiment is preferably a compound having a small ΔST. When ΔST is small, intersystem crossing and inverse intersystem crossing are likely to occur even at a low temperature (77K), so that the singlet state and the triplet state coexist. As a result, the spectrum to be measured in the same manner as the above includes emission from both the singlet state and the triplet state. Although it is difficult to distinguish the emission from the singlet state from the emission from the triplet state, the value of the triplet energy is basically considered dominant.

Accordingly, in the first embodiment, the triplet energy is measured by the same method as a typical triplet energy T, but a value measured in the following manner is referred to as an energy gap $T_{77K}$ in order to differentiate the measured energy from the typical triplet energy in a strict meaning. In the measurement using a thin film, the target compound for the measurement is deposited at a film thickness of 100 nm on a quartz substrate to form a sample. A phosphorescent spectrum (ordinate axis: phosphorescent luminous intensity, abscissa axis: wavelength) of the sample is measured at a low temperature (77K). A tangent is drawn to the rise of the phosphorescent spectrum on the short-wavelength side. An energy amount is calculated by the following conversion equation 1 based on a wavelength value $\lambda_{edge}$[nm] at an intersection of the tangent and the abscissa axis and defined as an energy gap $T_{77K}$.

$$\text{Conversion Equation 1: } T_{77K}\ [\text{eV}] = 1239.85/\lambda_{dge}$$

The tangent to the rise of the phosphorescence spectrum on the short-wavelength side is drawn as follows. While moving on a curve of the phosphorescence spectrum from the short-wavelength side to the maximum spectral value closest to the short-wavelength side among the maximum spectral values, a tangent is checked at each point on the curve toward the long-wavelength of the phosphorescence spectrum. An inclination of the tangent is increased as the curve rises (i.e., a value of the ordinate axis is increased). A tangent drawn at a point of the maximum inclination (i.e., a tangent at an inflection point) was defined as the tangent to the rise of the phosphorescence spectrum on the short-wavelength side.

The maximum with peak intensity being 15% or less of the maximum peak intensity of the spectrum is not included in the above-mentioned maximum closest to the short-wavelength side of the spectrum. The tangent drawn at a point of the maximum spectral value being closest to the short-wavelength side and having the maximum inclination is defined as a tangent to the rise of the phosphorescence spectrum on the short-wavelength side.

For phosphorescence measurement, a spectrophotofluorometer body F-4500 (manufactured by Hitachi High-Technologies Corporation) is usable. The measurement instrument is not limited to this arrangement. A combination of a cooling unit, a low temperature container, an excitation light source and a light-receiving unit may be used for measurement.

**Singlet Energy S**

The singlet energy S is measured as follows. 10 μmol/L of a toluene solution containing the target compound for the measurement was prepared and put in a quartz cell to prepare a sample. An absorption spectrum (ordinate axis: luminous intensity, abscissa axis: wavelength) of the sample was measured at a normal temperature (300K). A tangent was drawn to the fall of the absorption spectrum on the long-wavelength side, and a wavelength value $\lambda_{edge}$[nm] at an intersection of the tangent and the abscissa axis was substituted in the following conversion equation to calculate a singlet energy.

$$\text{Conversion Equation 2: } S\ [eV] = 1239.85/\lambda_{edge}$$

In Example, the absorption spectrum was measured using a spectrophotometer manufactured by Hitachi, Ltd. (device name: U3310). It should be noted that the absorption spectrum measuring device may be different from the above device.

The tangent to the fall of the absorption spectrum on the long-wavelength side is drawn as follows. While moving on a curve of the absorption spectrum from the maximum spectral value closest to the long-wavelength side in a long-wavelength direction, a tangent at each point on the curve is checked. An inclination of the tangent is decreased and increased in a repeated manner as the curve falls (i.e., a value of the ordinate axis is decreased). A tangent drawn at a point of the minimum inclination closest to the long-wavelength side (except when absorbance is 0.1 or less) is defined as the tangent to the fall of the absorption spectrum on the long-wavelength side.

The maximum absorbance of 0.2 or less is not included in the above-mentioned maximum absorbance on the long-wavelength side.

**Film Thickness of Emitting Layer**

A film thickness of the emitting layer (5) of the organic EL device (1) of the exemplary embodiment is preferably in a range from 5 nm to 100 nm, more preferably in a range from 7 nm to 100 nm, and most preferably in a range from 10 nm to 100 nm. The thickness of less than 5 nm may cause difficulty in forming the emitting layer (5) and in controlling chromaticity, while the thickness of more than 100 nm may raise drive voltage.

**Content Ratio of Compounds in Emitting Layer**

In the emitting layer (5) of the organic EL device (1) according to the first embodiment, a content ratio of the first compound is preferably 5 mass% or more, more preferably in a range from 10 mass% to 80 mass%, further preferably in a range from 40 mass% to 60 mass%. A content ratio of the second compound is preferably in a range from 1 mass% to 10 mass%. An upper limit of the total of the respective content ratios of the first and second compounds in the emitting layer 5 is 100 mass%. It should be noted that the emitting layer 5 of the exemplary embodiment may further contain another material in addition to the first and second compounds.

**Substrate**

A substrate (2) is used as a support for the organic EL device (1). For instance, glass, quartz, plastics and the like are usable as the substrate (2). A flexible substrate is also usable. The flexible substrate is a bendable substrate. The flexible substrate is exemplified by a plastic substrate formed of polycarbonate, polyarylate, polyethersulfone, polypropylene, polyester, polyvinyl fluoride, polyvinyl chloride, polyimide, polyethylene naphthalate or the like. Moreover, an inorganic vapor deposition film is also usable as the substrate (2).

**Anode**

Metal, alloy, an electrically conductive compound, a mixture thereof and the like, which have a large work function (specifically, of 4.0 eV or more) is preferably usable as the anode (3) formed on the substrate (2). Specific examples of the material for the anode include indium tin oxide (ITO), indium tin oxide containing silicon or silicon oxide, indium zinc oxide, tungsten oxide, indium oxide containing zinc oxide and graphene. In addition, gold (Au), platinum (Pt), nickel (Ni), tungsten (W), chrome (Cr), molybdenum (Mo), iron (Fe), cobalt (Co), copper (Cu), palladium (Pd),

titanium (Ti), or nitrides of a metal material (e.g., titanium nitride) are usable.

The above materials are typically deposited as a film by sputtering. For instance, indium zinc oxide can be deposited as a film by sputtering using a target that is obtained by adding zinc oxide in a range from 1 mass% to 10 mass% to indium oxide. Moreover, for instance, indium oxide containing tungsten oxide and zinc oxide can be deposited as a film by sputtering using a target that is obtained by adding tungsten oxide in a range from 0.5 mass% to 5 mass% and zinc oxide in a range from 0.1 mass% to 1 mass% to indium oxide. In addition, vapor deposition, coating, ink jet printing, spin coating and the like may be used for forming the anode (3).

Among the organic layers formed on the anode (3), a hole injecting layer (6) formed adjacent to the anode (3) is formed of a composite material that facilitates injection of holes irrespective of the work function of the anode (3). Accordingly, a material usable as an electrode material (e.g., metal, alloy, an electrically conductive compound, a mixture thereof, and elements belonging to Groups 1 and 2 of the periodic table of the elements) is usable as the material for the anode (3).

The elements belonging to Groups 1 and 2 of the periodic table of the elements, which are materials having a small work function, namely, an alkali metal such as lithium (Li) and cesium (Cs) and an alkaline earth metal such as magnesium (Mg), calcium (Ca) and strontium (Sr), alloy thereof (e.g., MgAg, AlLi), a rare earth metal such as europium (Eu) and ytterbium (Yb), and alloy thereof are also usable as the material for the anode. When the anode (3) is formed of the alkali metal, alkaline earth metal and alloy thereof, vapor deposition and sputtering are usable. Further, when the anode (3) is formed of silver paste and the like, coating, ink jet printing and the like are usable.

## Hole Injecting Layer

A hole injecting layer (6) is a layer containing a highly hole-injectable substance. Examples of the highly hole-injectable substance include molybdenum oxide, titanium oxide, vanadium oxide, rhenium oxide, ruthenium oxide, chromium oxide, zirconium oxide, hafnium oxide, tantalum oxide, silver oxide, tungsten oxide, and manganese oxide.

In addition, the examples of the highly hole-injectable substance further include: an aromatic amine compound, which is a low-molecule compound, such that 4,4',4"-tris(N,N-diphenylamino)triphenylamine (abbreviation: TDATA), 4,4',4"-tris[N-(3-methylphenyl)-N-phenylamino]triphenylamine (abbreviation: MTDATA), 4,4'-bis[N-(4-diphenylaminophenyl)-N-phenylamino]biphenyl(abbreviation: DPAB), 4,4'-bis(N-{4-[N'-(3-methylphenyl)-N'-phenylamino]phenyl} -N-phenylamino)biphenyl (abbreviation: DNTPD), 1,3,5-tris[N-(4-diphenylaminophenyl)-N-phenylamino]benzene (abbreviation: DPA3B), 3-[N-(9-phenylcarbazole-3-yl)-N-phenylamino]-9-phenylcarbazole (abbreviation: PCzPCA1), 3,6-bis[N-(9-phenylcarbazole-3-yl)-N-phenylamino]-9-phenylcarbazole (abbreviation: PCzPCA2), and 3-[N-(1-naphthyl)-N-(9-phenylcarbazole-3-yl)amino]-9-phenylcarbazole (abbreviation: PCzPCNI); and dipyrazino[2,3-f:20,30-h]quinoxaline-2,3,6,7,10,11-hexacarbonitrile (HAT-CN).

Moreover, a high-molecule compound (e.g., an oligomer, dendrimer and polymer) is also usable as the highly hole-injectable substance. Examples of the high-molecule compound include poly(N-vinylcarbazole) (abbreviation: PVK), poly(4-vinyltriphenylamine) (abbreviation: PVTPA), poly[N-(4-{N'-[4-(4-diphenylamino)phenyl]phenyl-N' -phenylamino} phenyl)methacrylamido] (abbreviation: PTPDMA), and poly[N,N'-bis(4-butylphenyl)-N,N'-bis(phenyl)benzidine] (abbreviation: Poly-TPD). Furthermore, the examples of the high-molecule compound include a high-molecule compound added with an acid such as poly(3,4-ethylene dioxythiophene)/poly(styrene sulfonic acid) (PEDOT/PSS), and polyaniline/poly(styrene sulfonic acid) (PAni/PSS).

## Hole Transporting Layer

A hole transporting layer (7) is a layer containing a highly hole-transportable substance. An aromatic amine compound, carbazole derivative, anthracene derivative and the like are usable for the hole transporting layer (7). Specific examples of a material for the hole transporting layer 7 include 4,4'-bis[N-(1-naphthyl)-N-phenylamino]biphenyl (abbreviation: NPB), N,N'-bis(3-methylphenyl)-N,N'-diphenyl-[1,1'-biphenyl]-4,4'-diamine (abbreviation: TPD), 4-phenyl-4'-(9-phenylfluorene-9-yl)triphenylamine (abbreviation: BAFLP), 4,4'-bis[N-(9,9-dimethylfluorene-2-yl)-N-phenylamino]biphenyl (abbreviation: DFLDPBi), 4,4',4"-tris(N,N-diphenylamino)triphenylamine (abbreviation: TDATA), 4,4',4"-tris[N-(3-methylphenyl)-N-phenylamino]triphenylamine (abbreviation: MTDATA), and 4,4'-bis[N-(spiro-9,9'-bifluorene-2-yl)-N-phenylamino]biphenyl (abbreviation: BSPB). The above-described substances mostly have a hole mobility of $10^{-6}$ $cm^2$ /(V•S) or more.

A carbazole derivative such as CBP, 9-[4-(N-carbazolyl)]phenyl-10-phenyl anthracene (CzPA) and 9-phenyl-3-[4-(10-phenyl-9-anthryl)phenyl]-9H-carbazole (PCzPA); an anthracene derivative such as t-BuDNA, DNA, and DPAnth; and a high-molecular weight compound such as poly(N-vinylcarbazole)(abbreviation: PVK) and poly(4-vinyltriphenylamine)(abbreviation: PVTPA) are usable for the hole transporting layer (7).

However, any substance having a hole transporting performance higher than an electron transporting performance may be used in addition to the above substances. A highly hole-transportable substance may be provided in the

form of a single layer or a laminated layer of two or more layers of the above substance.

When the hole transporting layer includes two or more layers, one of the layers with a larger energy gap is preferably provided closer to the emitting layer (5).

In the first embodiment, the hole transporting layer (7) preferably has a function of preventing triplet excitons generated in the emitting layer (5) from diffusing into the hole transporting layer to trap the triplet excitons within the emitting layer (5).

**Electron Transporting Layer**

An electron transporting layer (8) is a layer containing a highly electron-transportable substance. As the electron transporting layer, 1) a metal complex such as an aluminum complex, beryllium complex and zinc complex, 2) heteroaromatic compound such as an imidazole derivative, benzimidazole derivative, azine derivative, carbazole derivative, and phenanthroline derivative, and 3) a high-molecule compound are usable. Specifically, as a low-molecule organic compound, a metal complex such as Alq, tris(4-methyl-8-quinolinato)aluminum (abbreviation: $Almq_3$), bis(10-hydroxybenzo[h]quinolinato)beryllium (abbreviation: $BeBq_2$), BAlq, Znq, ZnPBO and ZnBTZ are usable. In addition to the metal complex, a heteroaromatic compound such as 2-(4-biphenyl)-5-(4-tert-butylphenyl)-1,3,4-oxadiazole (abbreviation: PBD), 1,3-bis[5-(ptert-butylphenyl)-1,3,4-oxadiazole-2-yl]benzene (abbreviation: OXD-7), 3-(4-tert-butylphenyl)-4-phenyl-5-(4-biphenyl)-1,2,4-triazole (abbreviation: TAZ), 3-(4-tert-butylphenyl)-4-(4-ethylphenyl)-5-(4-biphenyl)-1,2,4-triazole (abbreviation: p-EtTAZ), bathophenanthroline (abbreviation: BPhen), bathocuproine (abbreviation: BCP), and 4,4'-bis(5-methylbenzoxazole-2-yl)stilbene (abbreviation: BzOs) are usable. In the first embodiment, a benzimidazole compound is suitably usable. The above-described substances mostly have an electron mobility of $10^{-6}$ $cm^2$ /(V•s) or more. However, any substance having an electron transporting performance higher than a hole transporting performance may be used for the electron transporting layer (8) in addition to the above substances. The electron transporting layer (8) may be provided in the form of a single layer or a laminated layer of two or more layers of the above substance(s).

Moreover, a high-molecule compound is also usable for the electron transporting layer (8). For instance, poly[(9,9-dihexylfluorene-2,7-diyl)-co-(pyridine-3,5-diyl)] (abbreviation: PF-Py), poly[(9,9-dioctylfluorene-2,7-diyl)-co-(2,2'-bipyridine-6,6'-diyl)](abbreviation: PF-BPy) and the like are usable.

In the first embodiment, the hole transporting layer (8) preferably has a function of preventing triplet excitons generated in the emitting layer (5) from diffusing into the hole transporting layer (8) and the electron injecting layer (9) to trap the triplet excitons within the emitting layer (5).

**Electron Injecting Layer**

An electron injecting layer (9) is a layer containing a highly electron-injectable substance. Examples of a material for the electron injecting layer include an alkali metal, alkaline earth metal and a compound thereof, examples of which include lithium (Li), cesium (Cs), calcium (Ca), lithium fluoride (LiF), cesium fluoride (CsF), calcium fluoride (CaF2), and lithium oxide (LiOx). In addition, a substance obtained by containing an alkali metal, alkaline earth metal or a compound thereof in the electron transportable substance, specifically, for instance, a substance obtained by containing magnesium (Mg) in Alq may be used. With this substance, electrons can be more efficiently injected from the cathode (4).

Alternatively, a composite material provided by mixing an organic compound with an electron donor may be used for the electron injecting layer (9). The composite material exhibits excellent electron injecting performance and electron transporting performance since the electron donor generates electron in the organic compound. In this arrangement, the organic compound is preferably a material exhibiting an excellent transforming performance of the generated electrons. Specifically, for instance, the above-described substance for the electron transporting layer (8) (e.g., the metal complex and heteroaromatic compound) is usable. The electron donor may be any substance exhibiting an electron donating performance to the organic compound. Specifically, an alkali metal, alkaline earth metal and a rare earth metal are preferable, examples of which include lithium, cesium, magnesium, calcium, erbium and ytterbium. Moreover, an alkali metal oxide and alkaline earth metal oxide are preferable, examples of which include lithium oxide, calcium oxide, and barium oxide. Further, Lewis base such as magnesium oxide is also usable. Furthermore, tetrathiafulvalene (abbreviation: TTF) is also usable.

**Cathode**

Metal, alloy, an electrically conductive compound, a mixture thereof and the like, which have a small work function, specifically, of 3.8 eV or less, is preferably usable as a material for the cathode (4). Specific examples of the material for the cathode 4 include: the elements belonging to Groups 1 and 2 of the periodic table of the elements, namely, an alkali metal such as litium (Li) and cesium (Cs) and an alkaline earth metal such as magnesium (Mg), calcium (Ca) and strontium (Sr); alloy thereof (e.g., MgAg, AlLi); a rare earth metal such as europium (Eu) and ytterbium (Yb);

and alloy thereof.

When the cathode (4) is formed of the alkali metal, alkaline earth metal and alloy thereof, vapor deposition and sputtering are usable. Moreover, when the cathode (4) is formed of silver paste and the like, coating, ink jet printing and the like are usable.

By providing the electron injecting layer (9), various conductive materials such as Al, Ag, ITO, graphene and indium tin oxide containing silicon or silicon oxide are usable for forming the cathode (4) irrespective of the magnitude of the work function. The conductive materials can be deposited as a film by sputtering, ink jet printing, spin coating and the like.

### Layer Formation Method(s)

There is no restriction except for the above particular description for a method for forming each layer of the organic EL device (1) in the exemplary embodiment. Known methods such as dry film-forming and wet film-forming are applicable. Examples of the dry film-forming include vacuum deposition, sputtering, plasma and ion plating. Examples of the wet film-forming include spin coating, dipping, flow coating and ink-jet printing.

### Film Thickness

There is no restriction except for the above particular description for a film thickness of each of the organic layers of the organic EL device (1) in the exemplary embodiment. The film thickness is typically preferably in a range of several nanometers to 1 μm because an excessively-thinned film is likely to cause defects such as a pin hole while an excessively-thickened film requires high voltage to be applied and deteriorates efficiency.

### Electronic Device

The organic EL device (1) according to the first embodiment of the invention is usable in an electronic device such as a display unit and a light-emitting unit. Examples of the display unit include display components such as an organic EL panel module, TV, mobile phone, tablet, and personal computer. Examples of the light-emitting unit include an illuminator and a vehicle light.

The present invention therefore further relates to an electronic device comprising the organic electroluminescence device according to the present application and the use of the compound of formula (I) according to the present invention in an electronic device.

### Second Embodiment

Arrangement(s) of an organic EL device according to a second embodiment will be described below. In the description of the second embodiment, the same components as those in the first embodiment are denoted by the same reference signs and names to simplify or omit an explanation of the components. In the second embodiment, the same materials and compounds as described in the first embodiment are usable for a material and a compound which are not particularly described.

The organic EL device according to the second embodiment is different from the organic EL device according to the first embodiment in that the emitting layer comprises a first compound and a second compound, wherein the second compound is a delayed fluorescent compound (TADF emitter). The first compound is - in the second embodiment - not a delayed fluorescent compound.

### Second compound

The second compound in the third embodiment is represented by a compound of formula (I).

The inventors have found that a combination of the delayed fluorescent compound that is the second compound and a host that is the first compound, wherein the second compound is a delayed fluorescent compound (TADF emitter) is a compound of formula (I) according to the present invention, can provide advantages of prolonging a lifetime, improving a luminous efficiency, and inhibiting a roll-off in a high current density region.

The organic EL device in the second embodiment preferably emits light exhibiting a peak in a wavelength range of 430 to 780 nm. In other words, the main peak wavelength of the light emitted from the organic EL device preferably falls within a range of 430 to 780 nm. When the organic EL device in the exemplary embodiment emits light, it is preferable that the second compound mainly emits light in the emitting layer (5). The wavelength range of the light emitted by the organic EL device in the first embodiment is more preferably from 430 nm to 570 nm.

It is therefore preferable that the organic EL device in the first embodiment emits blue or green fluorescence.

**First compound**

Host materials for TADF emitters are for example described in Chem. Soc. Rev 2017, 46, 915 (start page 974), Adv. Mater. 2017, 1605444, Volume 29, Issue 22, June 13, 2017 1605444 (Start page 39), J. Mater. Chem. C, 2016, 4, 11355-11381 (start page 11355), J. Mater. Chem. C, 2017,5, 8622-8653.

The first compound is a host compound, whereby suitable host compounds are known to a person skilled in the art.

Examples for suitable hosts are described in the following:

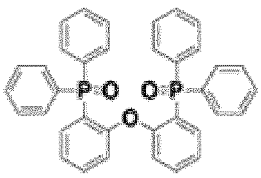

DPEPO

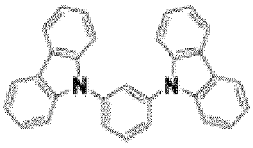

mCP

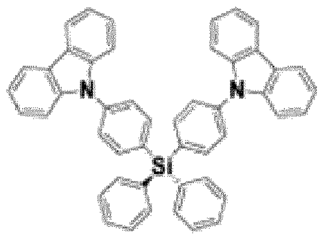

SiCz

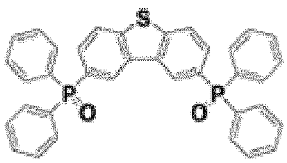

PPT

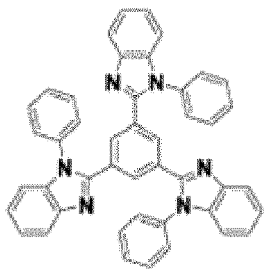

TPBI

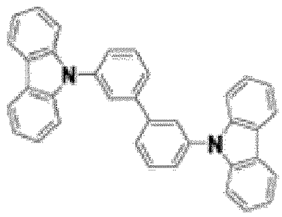

mCBP

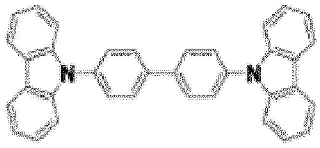

CBP

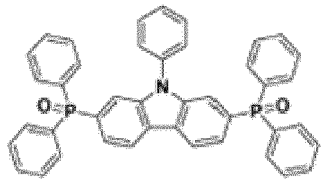

PPO27

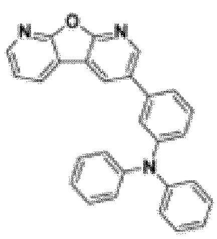

3TPAPFP

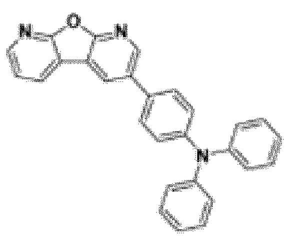

4TPAPFP

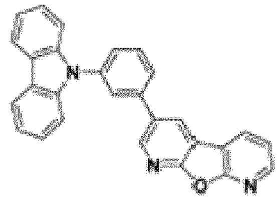

3CzPFP

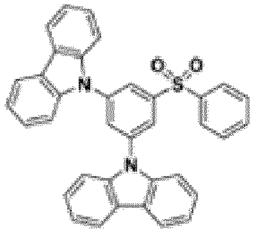

mCPSOB

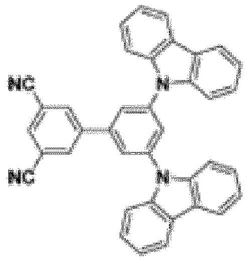

DCzDCN

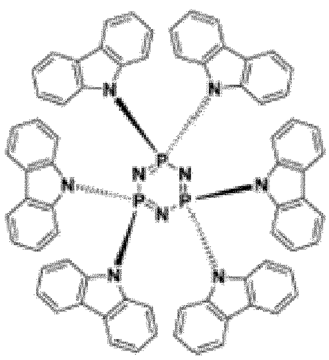

PzCz

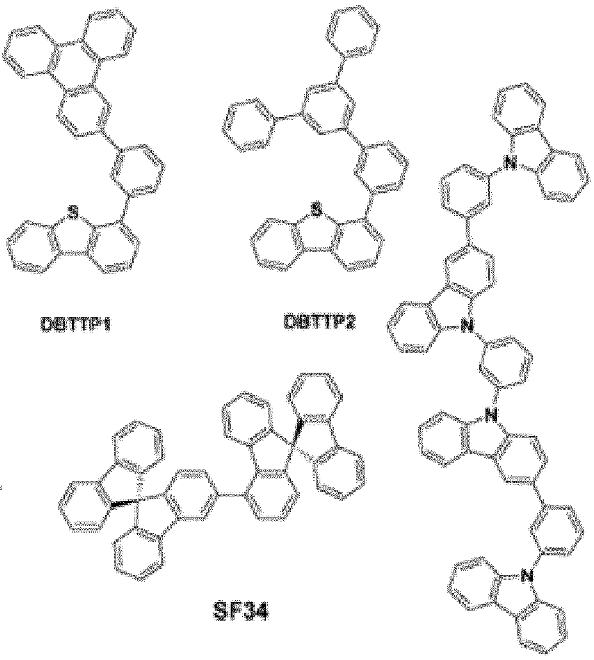

DBTTP1

DBTTP2

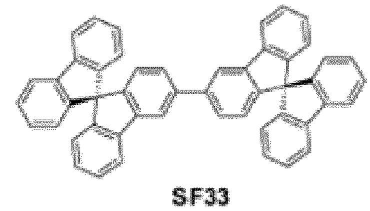

SF33

SF34

CPCB

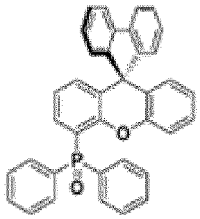

SFXSPO

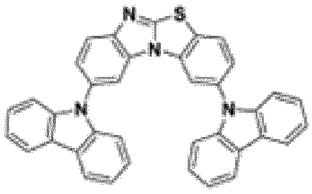

29Cz-BID-BT

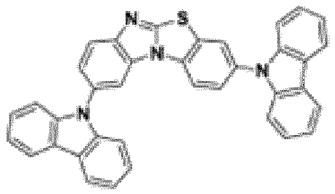

39Cz-BID-BT

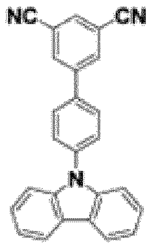

*p*CzB-2CN

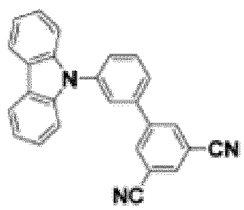

*m*CzB-2CN

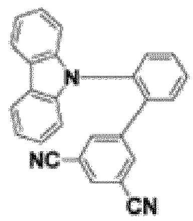

*o*CzB-2CN

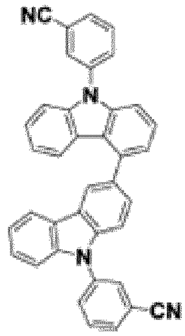

3CN34BCz

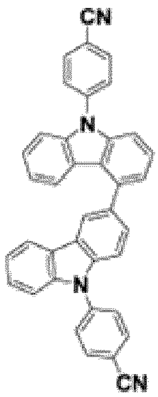

4CN34BCz

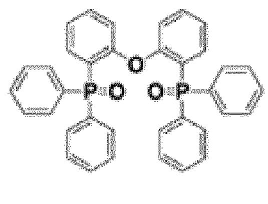

DPEPO

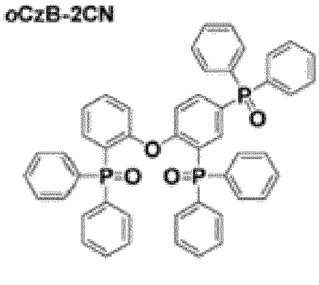

DPETPO

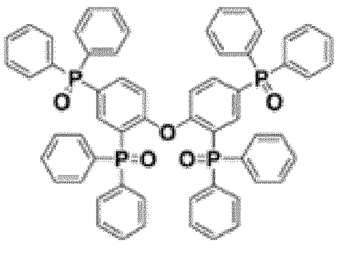

DPEQPO

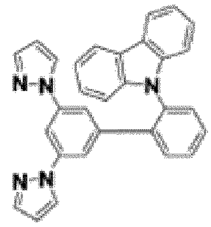

*o*-CzDPz

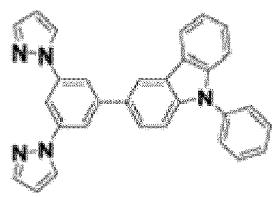

3-CzDPz

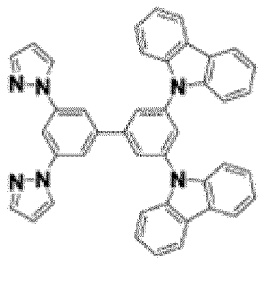

mCPDPz

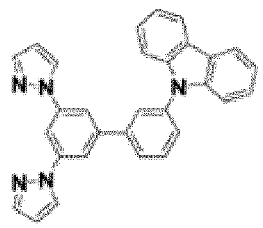

*m*-CzDPz

**Content Ratio of Compounds in Emitting Layer**

In the emitting layer (5) of the organic EL device (1) according to the second embodiment, a content ratio of the first compound (host) is preferably 5 mass% or more, more preferably in a range from 10 mass% to 80 mass%, further preferably in a range from 40 mass% to 60 mass%. A content ratio of the second compound is preferably in a range from 1 mass% to 50 mass%. An upper limit of the total of the respective content ratios of the first and second compounds in the emitting layer (5) is 100 mass%. It should be noted that the emitting layer (5) of the exemplary embodiment may further contain another material in addition to the first and second compounds.

**Third Embodiment**

Arrangement(s) of an organic EL device according to a third embodiment will be described below. In the

description of the third embodiment, the same components as those in the first embodiment are denoted by the same reference signs and names to simplify or omit an explanation of the components. In the third embodiment, the same materials and compounds as described in the first embodiment are usable for a material and a compound which are not particularly described.

The organic EL device according to the third embodiment is different from the organic EL device according to the first embodiment in that the emitting layer further includes a third compound. Other components are the same as those in the first embodiment. As in the first embodiment, at least one compound of formula (I) is present as first (TADF host) compound.

### Third Compound

The singlet energy of the third compound is larger than the singlet energy of the first compound.

**Fig. 5** exemplarily shows a relationship in energy levels (EnL in Fig. 4 is the enery level) between the first compound (1st), the second compound (2nd) and the third compound (3rd) in the emitting layer. In Fig. 5, S0 represents a ground state, S1(M1) represents the lowest singlet state of the first compound, T1(M1) represents the lowest triplet state of the first compound, S1(M2) represents the lowest singlet state of the second compound, T1(M2) represents the lowest triplet state of the second compound, S1(M3) represents a lowest singlet state of the third compound, and T1(M3) represents a lowest triplet state of the third compound. A dashed arrow directed from S1(M1) to S1(M2) in Fig. 5 represents Forster energy transfer from the lowest singlet state of the first compound to the lowest singlet state of the second compound.

As shown in Fig. 5, when a compound having a small ΔST(M1) is used as the first compound, inverse intersystem crossing from the lowest triplet state T1(M1) to the lowest singlet state S1(M1) can be caused by a heat energy. Accordingly, Forster energy transfer from the lowest singlet state S1(M1) of the first compound to the lowest singlet state S1(M2) of the second compound is caused. As a result, fluorescence from the lowest singlet state S1(M2) of the second compound can be observed. It is considered that the internal quantum efficiency can be theoretically raised up to 100% also by using delayed fluorescence by the TADF mechanism.

### Ratio of Three Components

In the emitting layer of the organic EL device of the third embodiment, it is preferable that the content ratio of the first compound is in a range from 10 mass% to 80 mass%, the content ratio of the second compound is in a range from 1 mass% to 10 mass%, and the content ratio of the third compound is in a range from 10 mass% to 80 mass%. The content ratio of the first compound is more preferably in a range from 20 mass% to 80 mass%, further preferably in a range from 40 mass% to 60 mass%. An upper limit of the total of the respective content ratios of the first to third compounds in the emitting layer is 100 mass%. It should be noted that the emitting layer of the exemplary embodiment may further contain another material in addition to the first to third compounds.

Although the third compound is not particularly limited, the third compound is preferably a compound other than an amine compound. For instance, carbazole derivatives, dibenzofuran derivatives, Si containing derivatives, phosphorous oxide derivatives and dibenzothiophene derivatives are usable as the third compound. However, the third compound is not limited thereto.

The third compound preferably has at least one of a partial structure represented by a formula (31) below and a partial structure represented by a formula (32) below in one molecule.

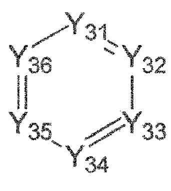
(31)

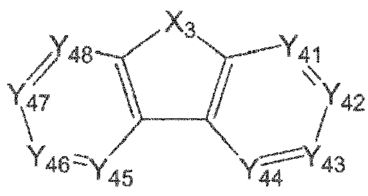
(32)

In the formula (31): $Y_{31}$ to $Y_{36}$ each independently represent a nitrogen atom or a carbon atom bonded to another atom in the molecule of the third compound.

However, at least one of $Y_{31}$ to $Y_{36}$ is a carbon atom bonded to another atom in the molecule of the third compound.

In the formula (32): $Y_{41}$ to $Y_{48}$ each independently represent a nitrogen atom or a carbon atom bonded to another atom in the molecule of the third compound.

However, at least one of $Y_{41}$ to $Y_{48}$ is a carbon atom bonded to another atom in the molecule of the third compound.

$X_3$ represents a nitrogen atom, an oxygen atom or a sulfur atom.

In the formula (32), it is also preferable that at least two of $Y_{41}$ to $Y_{48}$ are carbon atoms bonded to another atom in the molecule of the third compound and a ring structure is formed including the carbon atoms.

Specific partial structures represented by the formula (32) and by the formula (31) are described in EP 3 255 693 A1, especially in paragraphs [0189] to [0212].

The third compound is also preferably an aromatic hydrocarbon compound or an aromatic heterocyclic compound. It is also preferable that the third compound has no fused aromatic hydrocarbon ring in a molecule.

Examples of substituents of the third compound are mentioned in EP 3 255 693 A1, especially in paragraphs [0214] to [0226].

Further suitable third compounds are

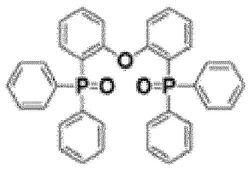

DPEPO and

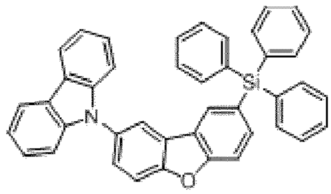

.

The third compound may be prepared by a method described in WO 2012/153780, WO 2013/038650 and EP 3 255 693.

The organic EL device in the third embodiment is capable of prolonging a lifetime, improving a luminous efficiency, and inhibiting a roll-off when driven at a high current density.

The emitting layer of the organic EL device according to the third embodiment includes the delayed fluorescent first compound, the fluorescent second compound, and the third compound having a larger singlet energy than the first compound, whereby the luminous efficiency of the organic EL device is further improved. It is deduced that the luminous efficiency is improved because a carrier balance in the emitting layer is improved by containing the third compound.

**Modification of Embodiments**

It should be noted that the invention is not limited to the embodiments mentioned above. The invention may include any modification and improvement compatible with the invention.

For instance, the emitting layer is not limited to a single layer, but may be provided by laminating a plurality of emitting layers. When the organic EL device has the plurality of emitting layers, it is only required that at least one of the emitting layers contains the first and second compounds. For instance, the rest of the emitting layers may be a fluorescent emitting layer or a phosphorescent emitting layer with use of emission caused by electron transfer from the triplet state directly to the ground state.

When the organic EL device includes the plurality of emitting layers, the plurality of emitting layers may be adjacent to each other, or provide a so-called tandem-type organic EL device in which a plurality of emitting units are layered through an intermediate layer.

For instance, a blocking layer may be provided in contact with an anode-side or a cathode-side of the emitting layer. The blocking layer is preferably provided in contact with the emitting layer to block at least ones of holes, electrons and excitons.

For instance, when the blocking layer is provided in contact with the cathode-side of the emitting layer, the blocking layer permits transport of electrons, but prevents holes from reaching a layer provided near the cathode (e.g., the electron transporting layer) beyond the blocking layer. When the organic EL device includes the electron transporting layer, the blocking layer is preferably interposed between the emitting layer and the electron transporting layer.

When the blocking layer is provided in contact with the anode-side of the emitting layer, the blocking layer permits transport of holes, but prevents electrons from reaching a layer provided near the anode (e.g., the hole transporting layer) beyond the blocking layer. When the organic EL device includes the hole transporting layer, the blocking layer is preferably interposed between the emitting layer and the hole transporting layer.

Further, the blocking layer may be provided in contact with the emitting layer to prevent an excitation energy from leaking from the emitting layer into a layer in the vicinity thereof. Excitons generated in the emitting layer are prevented from moving into a layer provided near the electrode (e.g., the electron transporting layer and the hole transporting layer) beyond the blocking layer.

The emitting layer and the blocking layer are preferably bonded to each other.

Further, the materials and treatments for practicing the invention may be altered to other arrangements and treatments as long as such other arrangements and treatments are compatible with the invention.

## Examples

Examples of the invention will be described below. However, the invention is not limited to Examples.

### I. Synthetic examples

### Synthetic example 1

a.) 2-(9H-Carbazol-9-yl)-6-(13-phenylbenzo[4',5']imidazo[1',2':1,2]imidazo[4,5-a]carbazol-1(13H)-yl)benzonitrile

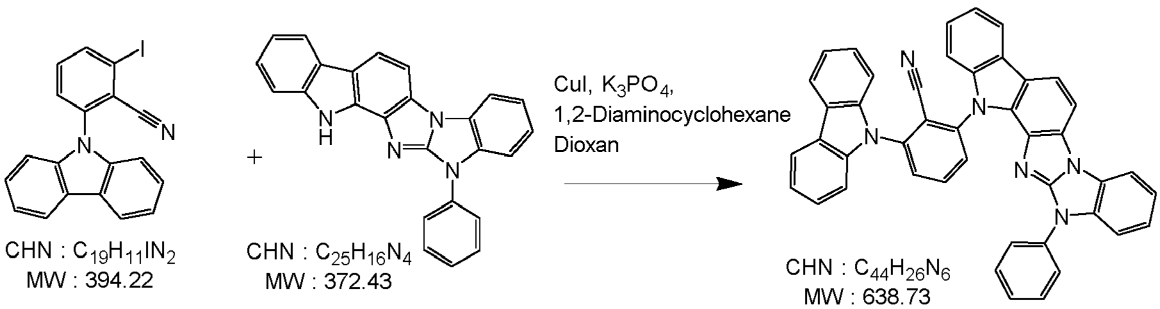


CHN : $C_{19}H_{11}IN_2$
MW : 394.22

CHN : $C_{25}H_{16}N_4$
MW : 372.43

CHN : $C_{44}H_{26}N_6$
MW : 638.73

To 1.69 g (4.54 mmol) 13-phenyl-1,13-dihydrobenzo[4',5'] imidazo[1',2':1,2]imidazo [4,5-a] carbazole, 1.79 g (4.54 mmol) 2-(9H-carbazol-9-yl)-6-iodobenzonitrile, 2.89 g (13.6 mmol) potassium carbonate tribasic, 1.04 g (9.08 mmol) 1,2-cis,trans-diaminocyclohexane and 173 mg (0.908 mmol) copper (I) iodide were added. 20 ml dioxane were added under nitrogen and the reaction mixture was stirred at 100 °C for 27 h.

The reaction mixture was poured on water and the product was filtered off. Column chromatography on silica gel with toluene/heptane 3/2 gave the product.
400 mg (17%)
$^1$H MMR (400 MHz, $CDCl_3$): 8.25 (d, 1H), 8.07-8.15 (m, 3H), 8.03 (t, 1H), 7.98 (d, 1H), 7.87-7.91 (m, 4H), 7.80 (d, 1H), 7.71 (d, 1H), 7.32-7.52 (m, 11H), 7.19-7.27 (m, 2H), 6,99 (td, 1H).

b.) 2-(9H-Carbazol-9-yl)-6-iodobenzonitrile

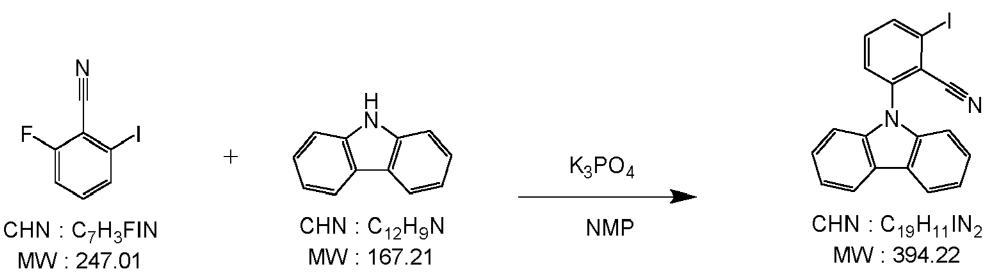


CHN : $C_7H_3FIN$
MW : 247.01

CHN : $C_{12}H_9N$
MW : 167.21

CHN : $C_{19}H_{11}IN_2$
MW : 394.22

To 20 g (81.0 mmol) 2-fluoro-6-iodobenzonitrile, 12.9 g (76.9 mmol) carbazol and 51.6 g (0.243 mmol) potassium carbonate tribasic 100 ml NMP were added under nitrogen. The reaction mixture was stirred under nitrogen at 30 °C for 26 h and 20 h at 45 °C.

The reaction mixture was poured on water and the product was filtered off. The reaction mixture was washed with diethyl ether. Yield 30.0 g (99 %)
$^1$H MMR (400 MHz, $CDCl_3$): 8.16 (d, 2H), 8.12 (d, 1H), 7.63 (d, 1H), 7.52 (t, 1H), 7.46 (t, 2H), 7.36 (t, 1H), 7.21 (d, 1H).

c.) 13-Phenyl-1,13-dihydrobenzo[4',5'] imidazo[1',2':1,2]imidazo [4,5-a] carbazole

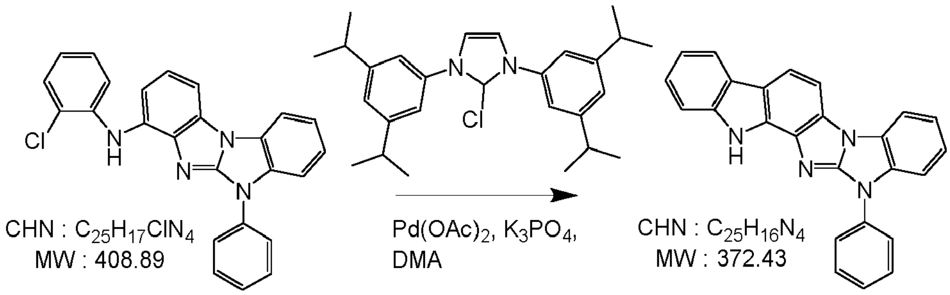


18.0 g (44.0 mmol) 2-chloro-1,3-bis(3,5-diisopropylphenyl)-2,3-dihydro-1H-imidazole and 18.2 g (132 mmol) $K_2CO_3$ in DMA (water free) was degassed 5 times with argon. 494 mg (2.20 mmol) $Pd(OAc)_2$ and 1.87 g (4.40 mmol) N-(2-chlorophenyl)-5-phenyl-5H-benzo[d]benzo[4,5]imida-zo[1,2-a]imidazol-7-amine (CAS: 250285-32-6) was added and the reaction mixture was 5 times degassed with argon. The reaction mixture was stirred under argon at 155 °C for 2 h. 1.5 g sodium cyanide in 5 ml water was added and the reaction mixture was stirred at 120 °C for 1 h. The product was poured on water and the product was filtered off. The product was washed with water. The product was decocted 2 times in 100 ml ethanol. Yield 16.3 g (100 %)
$^1H$ MMR (400 MHz, $CDCl_3$): 9.41 (s, 1H), 8.16 (d, 1H), 8.04 (d, 1H), 7.97 (d, 1H), 7.89-7.92 (m, 2H), 7.77 (d, 1H), 7.65-7.70 (m, 2H), 7.61 (d, 1H), 7.55 (d, 1H), 7.50 (tt,1H), 7.36-7-46 (m, 3H), 7.27-7.31 (dt, 1H) under $CD(H)Cl_3$ signal.

d.) 2-Chloro-1,3-bis(3,5-diisopropylphenyl)-2,3-dihydro-1H-imidazole

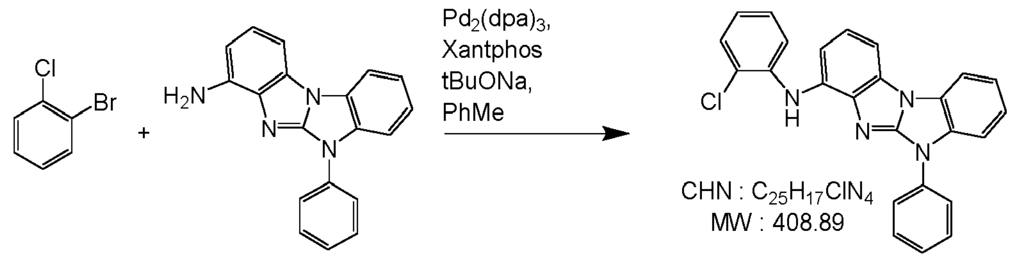


To 17.3 g (58.0 mmol) 5-phenyl-5H-benzo[d]benzo[4,5]imidazo[1,2-a]imidazol-7-amine 16.7 g (87.0 mmol) 1-bromo-2chlorobenzene and 11.1 g (116 mmol) tBuoNa in 240 ml water free toluene was added. The reaction mixture was degassed 5 times with argon. 1.06 g (1.16 mmol) Tris(dibenzylideneacetone)dipalladium(0) (Cas 51364-51-3) and 1.01 g (1.74 mmol) Xantphos (Cas 161265-03-8) was added and the reaction mixture was degassed 5 times. The reaction mixture was stirred under argon at 90 °C for 2 h.

200 mg sodium cyanide in 2 ml water was added. The reaction mixture was refluxed for 1 h. The reaction mixture was poured on water and was extracted with dichloromethane. The organic phase was dried with magnesium sulfate and the solvent was removed in vacuum. Column chromatography on silica gel with heptane/toluene 1/1 gave the product. Yield: 18.6 g 78 %
$^1H$ MMR (400 MHz, $CDCl_3$): 7.91-7.94 (m, 2H), 7.84-7.87 (m, 1H), 7.60-7.68 (m, 4H), 7.34-7.50 (m, 5H), 7.27-7.33 (m, 1H) under $CD(H)Cl_3$ signal, 7.25 (d, 1H), 7.19-7.23 (m, 1H), 7.02 (s, 1H), 6.88 (td, 1H).

e.) 5-Phenyl-5H-benzo[d]benzo[4,5]imidazo[1,2-a]imidazol-7-amine

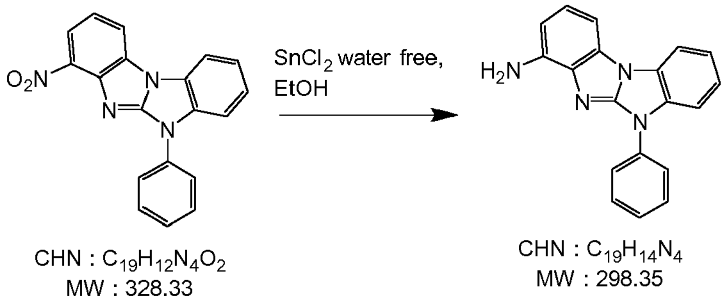


19.7 g (60.0 mmol) 7-nitro-5-phenyl-5H-benzo[d]benzo[4,5]imidazo[1,2-a]imidazole were dissolved in 220 ml ethanol and 90 ml 32 % hydrochloric acid. 34.1 g (180 mmol) water free tin(II)chloride was added at 25 °C. The reaction mixture was stirred at 80 °C for 1 h. 8.00 g tin(II)chloride was added and the reaction mixture was stirred for 30 min.

The reaction mixture was poured on ice and 150 ml of a 50 % solution of sodium hydroxide in water was added. The product was filtered off and was washed with water. The product was decocted with 100 ml ethanol. Yield 17.8 g (99 %)
$^1$H MMR (400 MHz, $CDCl_3$): 7.89-7.92 (m, 2H), 7.80-7.83 (m, 1H), 7.62-7.66 (m, 2H), 7.56-7.59 (m, 1H), 7.46 (t, 1H), 7.28-7.40 (m, 3H), 7.14 (t, 1H), 6.72 (d, 1H), 4.31 (s, 2H).

f.) 7-Nitro-5-phenyl-5H-benzo[d]benzo[4,5]imidazo[1,2-a]imidazole

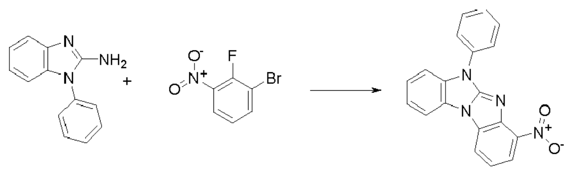

20.9 g (100 mmol) 1-phenyl-1H-benzo[d]imidazol-2-amine, 22.0 g (100 mmol) 3-bromo-2-fluoro-1-nitrobenzene (CAS: 58534-94-4) and 97.6 g (300 mmol) cesium carbonate in 250 ml DMSO were stirred at 25 °C for 3 h. Then the reaction mixture was stirred at 130 °C for 2 h.

The reaction mixture was poured on water 150 ml and the product was filtered off. The product was washed with water. The product was decocted two times in 200 ml ethanol. The product was filtered on silica gel with dichloromethane 2 times. Yield 13.4 g (41 %)
$^1$H MMR (400 MHz, $CDCl_3$): 8.27 (dd, 1H), 8.14 (d, 1H), 7.87-7.93 (m, 3H), 7.63-7.69 (m, 3H), 7.44-7.54 (m, 3H), 7.39 (t, 1H).

**Synthetic example 2**

a.) 2-(9H-Carbazol-9-yl)-6-(6-phenylbenzo[4',5']imidazo[1',2':1,2]imidazo[4,5-b] carbazol-8(6H)-yl)benzonitrile

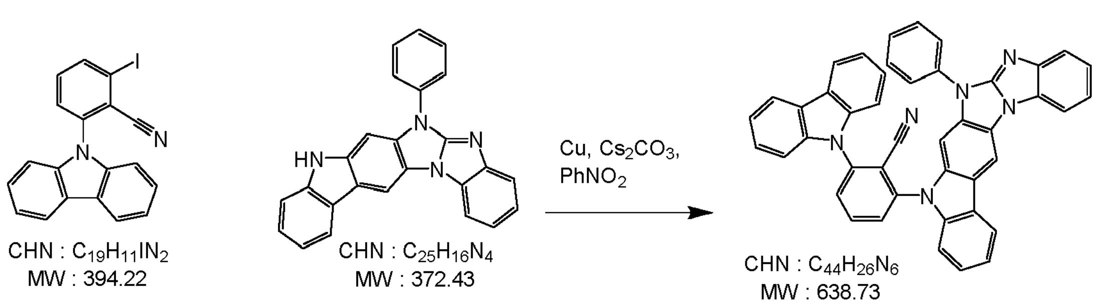


To 1.75 g (4.44 mmol) 2-(9H-Carbazol-9-yl)-6-iodobenzonitrile, 1.65 g (4.44 mmol) 6-phenyl-6,8-dihydrobenzo[4',5']imidazo[1',2':1,2]imidazo[4,5-b]carbazole, 14 mg (0.22 mmol) copper and 4.34 g (13.3 mmol) cesium carbonate

in 30 ml nitrobenzene were added. The reaction mixture was stirred at 200 °C for 34 h.

The reaction mixture was poured on 100 ml methanol and the product was filtered off. The product was washed with methanol, water and again methanol.

Column chromatography on silica gel with toluene/ethyl acetate 1/1 gave the product.
$^1$H MMR (400 MHz, THF-d8): 8.85 (s, 1H), 8.38 (d, 1H), 8.16-8.24 (m, 4H), 8.05-8.08 (m, 3H), 7.95 (dd, 1H), 7.65-7.69 (m, 2H), 7.58-7.63 (m, 2H), 7.45-7.52 (m, 4H), 7.39-7.44 (m, 2H), 7.28-7.38 (m, 6H).
MS(ESI) *m/z* $[M+H]^+$: 639

The synthesis of 6-Phenyl-6,8-dihydrobenzo[4',5']imidazo[1',2':1,2]imidazo[4,5-b]carbazole is described in WO2017093958A1 example 10d

**Synthetic example 3**

a.) 2-(9H-Carbazol-9-yl)-6-(7-phenylbenzo[4',5']imidazo[2',1':2,3]imidazo[4,5-a]carbazol-14(7H)-yl) benzonitrile

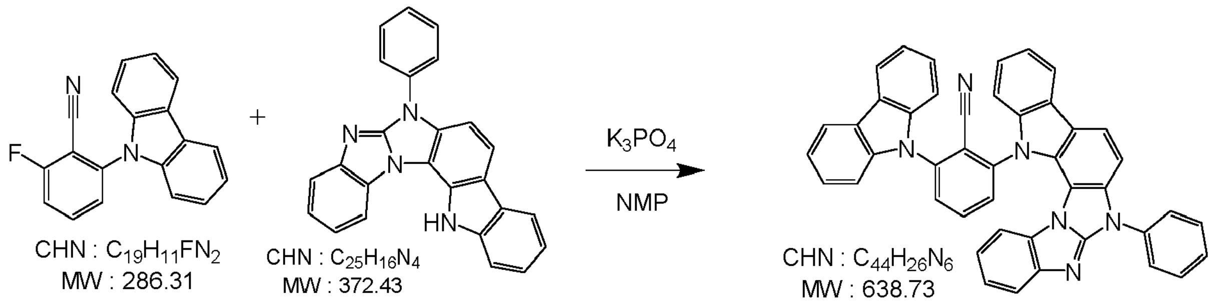


270 mg (0.733 mmol) 7-Phenyl-7,14-dihydrobenzo[4',5']imidazo[2',1':2,3]imidazo[4,5-a]carbazole, 200 mg (0.699 mmol) 2-(9H-carbazol-9-yl)-6-fluorobenzonitrile and 440 mg (0.210 mmol) potassium carbonate tribasic were dissolved in 10 ml NMP. The reaction mixture was stirred at 100 °C for 23 h.

The reaction mixture was poured on water and the product was filtered off. The product was washed with water. Column chromatography on silica gel with dichloromethane gave the product. Yield 190 mg (31 %).
$^1$H MMR (400 MHz, THF-d8): 8.39 (d,1H), 8.28 (d, 1H), 8.20-8.24 (m, 2H), 8.11 (d, 1H), 8.05 (d, 1H), 7.98 (d, 2H), 7.82 (d, 1H), 7.85 (d, 1H), 7.64-7.69 (m, 4H), 7.44-7.58 (m, 3H), 7.39 (t, 1H), 7.16-7.28 (m, 4H), 7.11 (td, 1H), 6.81-6.87 (m, 2H), 6.11 (d, 1H).
MS(ESI) *m/z* $[M+H]^+$: 639

The synthesis of 2-(9H-Carbazol-9-yl)-6-fluorobenzonitrile is described in WO2017115596A1

b.) 7-Phenyl-7,14-dihydrobenzo[4',5']imidazo[2',1':2,3]imidazo[4,5-a]carbazole

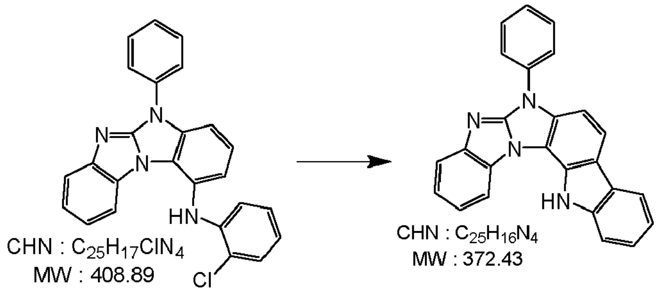


15.75 g N-(2-Chlorophenyl)-5-phenyl-5H-benzo[d]benzo[4,5]imidazo[1,2-a]imidazol-1-amine (38.1 mmol) and 10.54g (76 mmol) were added to Xylene (150ml) and the brown suspension was evacuated and backfilled with Argon four times. 0.257 g Diacetoxypalladium (1.144 mmol) and 0.827g 1,3-bis(2,6-diisopropylphenyl)-imidazolium chloride (1.907 mmol) were added and the reaction mixture was evacuated and backfilled with Argon four times and then heated to reflux for 2h. The reaction mixture was cooled to room temperature, filtered and the residue was washed with water

and methanol and dried at 80°C/125mbar overnight to yield 10.1g of crude product. It was dissolved in 400ml DMF at reflux, 10g silica were added, stirred for 15min and then filtered hot through Hyflo. The resulting solution was rotavaped down until a suspension formed, that was cooled to room temperature, filtered and washed with MeOH. The product was dried at 80°C/125mbar overnight to yield 9.1g (64.1%) of product as a white solid.
$^1$H NMR (400 MHz, DMSO-$d_6$) $\delta$ 11.28 (s, 1H), 8.84 (s, 1H), 8.19 (dd, $J$ = 12.1, 8.0 Hz, 2H), 8.02 - 7.93 (m, 2H), 7.83 (d, $J$ = 8.0 Hz, 1H), 7.78 - 7.70 (m, 2H), 7.70 - 7.61 (m, 1H), 7.57 (t, $J$ = 7.5 Hz, 1H), 7.46 (t, $J$ = 7.7 Hz, 1H), 7.43 - 7.35 (m, 3H), 7.25 (t, $J$ = 7.3 Hz, 1H).

c.) N-(2-Chlorophenyl)-5-phenyl-5H-benzo[d]benzo[4,5]imidazo[1,2-a]imidazol-1-amine

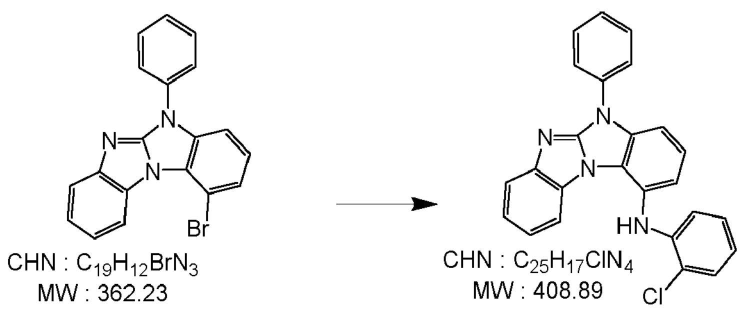


48.59g 1-Bromo-5-phenyl-5H-benzo[d]benzo[4,5]imidazo[1,2-a]imidazole (114 mmol), 22.59g sodium tert-butoxide (228 mmol) and 43.6g 2-chloroaniline (342 mmol) were added to toluene (500 ml). The dark suspension was evacuated and backfilled with Argon 5 times. 2.09g $Pd_2(dba)_3$ (2.280 mmol) and 2.04g Xantphos (3.42 mmol) were added and the reaction mixture was evacuated and backfilled with Argon 5 times. The reaction mixture was heated to 90°C internal temperature, stirred for 26h and then filtered hot through Hyflo. The residue was washed with EtOAc, the filtrate was dried with $Na_2SO_4$, filtered and rotavaped down to a dark brown suspension. The suspension was cooled to room temperature, filtered and the residue was washed with EtOH. The residue was again added to EtOH, stirred for 30min, filtered, the residue was washed with EtOH and dried at 80°C/125mbar overnight to yield 35.8g (76.8%) of product as a beige solid.
$^1$H NMR (400 MHz, DMSO-$d_6$) $\delta$ 8.20 (s, 1H), 7.97 - 7.89 (m, 2H), 7.87 - 7.80 (m, 1H), 7.75 - 7.67 (m, 2H), 7.61 - 7.51 (m, 2H), 7.48 (ddd, $J$ = 8.0, 4.0, 1.3 Hz, 2H), 7.41 (t, $J$ = 8.0 Hz, 1H), 7.21 (ddd, $J$ = 9.2, 7.9, 1.3 Hz, 2H), 7.02 (dddd, $J$ = 8.5, 7.3, 4.8, 1.3 Hz, 2H), 6.77 (td, $J$ = 7.6, 1.5 Hz, 1H), 6.57 (dd, $J$ = 8.1, 1.5 Hz, 1H).

d.) 1-Bromo-5-phenyl-5H-benzo[d]benzo[4,5]imidazo[1,2-a]imidazole

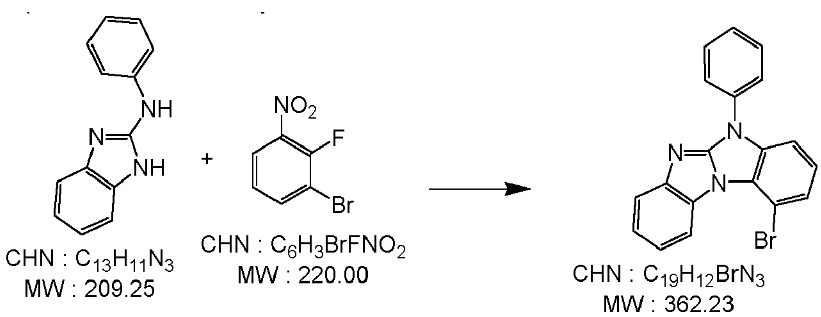


26.06 g N-Phenyl-1H-benzo[d]imidazol-2-amine (125 mmol) and 27.4 g 1-bromo-2-fluoro-3-nitrobenzene (125 mmol) were dissolved in DMA (300 ml). 101g $Cs_2CO_3$ (311 mmol) were added and the suspension was stirred at room temperature for 2h. The color changed from green to blue to brown. TLC (Toluene/EtOAc = 2:1) showed clean conversion to intermediate product. The suspension was then heated to 100°C external temperature for 4h. The reaction mixture was cooled to room temperature, then poured on 2l of water. The resulting suspension was filtered, the residue was washed with water and then dried at 80°C/125mbar over night. The crude product was added to 700ml water, stirred for 1h, filtered and dried at 80°C/125mbar over night to yield 41.4 g (91.9%) of yellow product that was used without

further purification.
$^1$H NMR (400 MHz, DMSO-$d_6$) δ 8.83 (d, $J$ = 8.2 Hz, 1H), 7.9 - 7.2 (m, 11H).

e.) N-Phenyl-1H-benzo[d]imidazol-2-amine

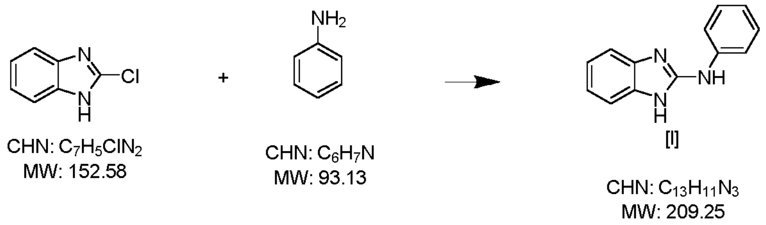


38.15 g 2-Chloro-benzimidazole (248 mmol) and 25.4g aniline (272 mmol) were disolved in NMP (250ml). 26.2 g Methanesulfonic acid (272 mmol) were added drop by drop at room temperature within 20 min. An exothermix reaction occured and the internal temperature rose to 50°C. The resulting suspension was heated to 100°C internal temperature for 2h, then cooled to room temperature and poured on 2l water and brought to pH =10 by adding solid NaOH. The suspension was extracted with EtOAc, the organic phase washed with water twice, dried with $Na_2SO_4$, filtered and evaporated to yield 52.6 g of crude product. The crude product was added to 60ml $CH_2Cl_2$, the resulting suspension was stirred for 30min and filtered. The residue was dried at 80°C/125mbar overnight to yield 43.3g (83.7%) of product as a white solid.
$^1$H NMR (400 MHz, DMSO-$d_6$) δ 10.86 (s, 1H), 9.36 (s, 1H), 7.80 - 7.66 (m, 2H), 7.38 - 7.19 (m, 4H), 6.97 (q, $J$ = 4.5 Hz, 2H), 6.93 - 6.85 (m, 1H).

**Synthetic example 4**

a.) 2,6-bis(7-Phenylbenzo[4',5']imidazo[2',1':2,3]imidazo[4,5-a]carbazol-14(7H)-yl) benzonitrile

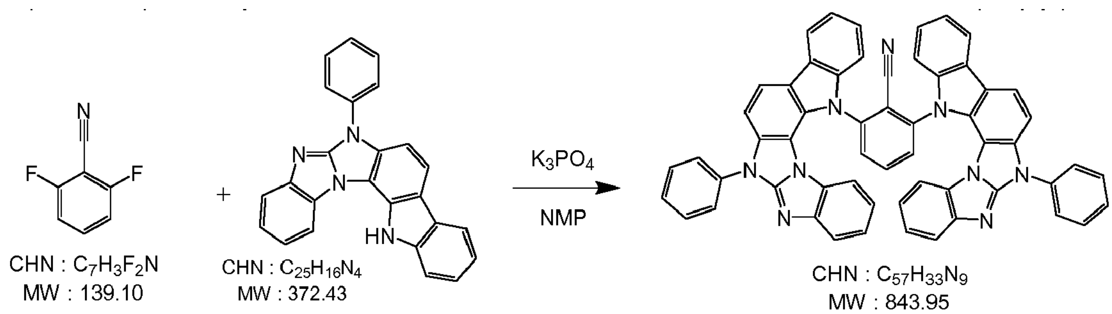


1.07 g (2.88 mmol) 7-Phenyl-7,14-dihydrobenzo[4',5']imidazo[2',1':2,3]imidazo[4,5-a]carbazole, 200 mg (1..44 mmol) 2,6-difluorobenzonitrile and 920 mg (4.31 mmol) potassium carbonate tribasic were dissolved in 20 ml NMP. The reaction mixture was stirred at 100 °C for 72 h.

The reaction mixture was poured on methanol and the product was filtered off. The product was washed with methanol. Column chromatography on silica gel with dichloromethane and then dichloromethane/methanol 95/5 gave the product. Yield 340 mg (19 %).
$^1$H MMR (400 MHz, THF-d8): 8.18 (t, 4H), 8.03 (d, 4H), 7.94 (d, 2H), 7.83 (dd, 1H), 7.67-7.73 (m, 6H), 7.49-7.56 (m, 4H), 7.37 (t, 2H), 7.30 (t, 2H), 7.11 (d, 2H), 6.98 (t, 2H), 6.66 (d, 2H), 6.43 (t, 2H)

**Synthetic example 5**

a.) 2-(13-Phenylbenzo[4',5']imidazo[1',2':1,2]imidazo[4,5-a]carbazol-1(13H)-yl)-6-(5-phenylindolo[3,2-a]carbazol-12(5H)-yl)benzonitrile

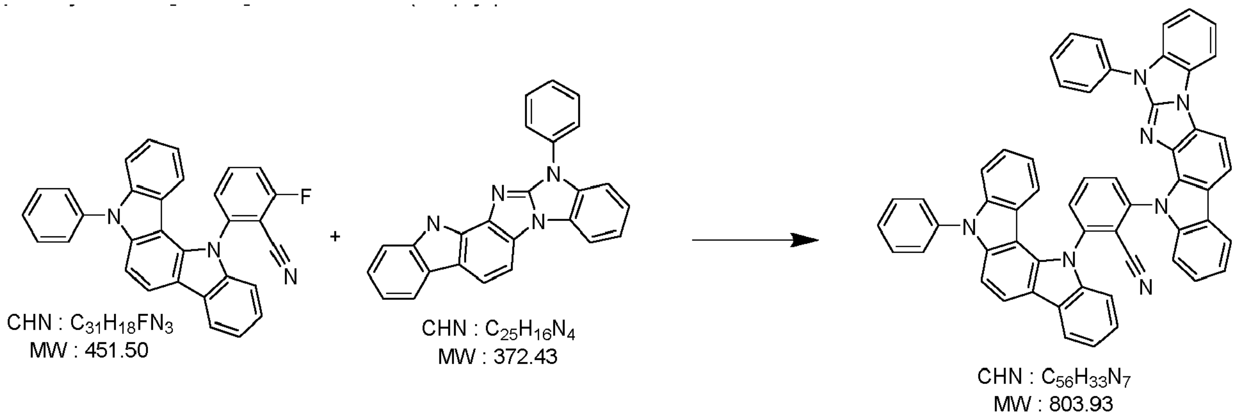


1.86 g (5.00 mmol) 13-Phenyl-13H-12-benzo[4',5']imidazo[1',2':1,2]imidazo[4,5-a]carbazole, 2.26 g (5.00 mmol) 2-fluoro-6-(5-phenylindolo[3,2-a]carbazol-12(5H)-yl)benzonitrile and 2.65 g (12.5 mmol) potassium carbonate in 50 ml water free DMA were stirred under nitrogen at 130 °C for 3 h.

The reaction mixture was poured on 200 ml methanol and the product was filtered off. The product was soxhlet extracted with toluene. Yield 1.6 g

$^1$H MMR (400 MHz, THF-d8): 8.14-8.26 (m, 5H), 8.06-8.11 (m, 3H), 7.87-7.93 (m 2H), 7.67-7.75 (m, 3H), 7.57-7.62 (m, 1H), 7.50-7.62 (m, 2H), 7.41-7.49 (m, 2H), 7.28-7.40 (m, 6H), 7.20-7.24 (m, 2H), 7.09-7.18 (m, 5H), 6.60 (t, 1H), 6.46 (d, 1H)

MS(ESI) *m/z* $[M+H]^+$ : 805 (M+1)

b.) 2-Fluoro-6-(5-phenylindolo[3,2-a]carbazol-12(5H)-yl)benzonitrile

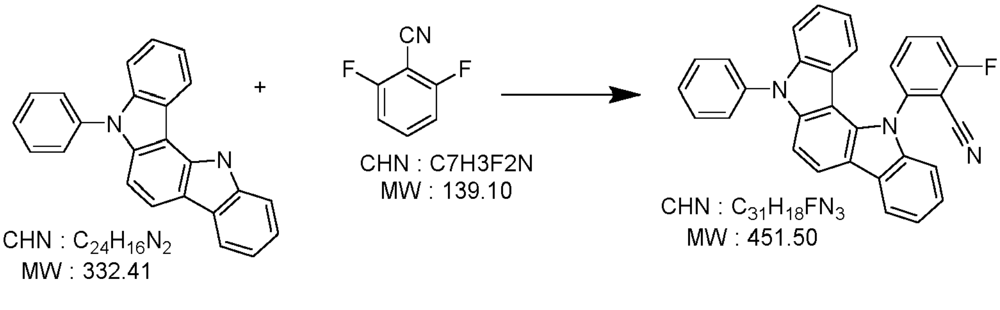


4.99 g (15.0 mmol) 5-Phenyl-5H-12l2-indolo[3,2-a]carbazole, 3.13 g (22.5 mmol) 2,6-difluorobenzonitrile and 11.9 g (56.3 mmol) potassium carbonate tribasic were dissolved in 75 ml NMP. The reaction mixture was stirred at 110 °C for 9 h.

The reaction mixture was poured on 200 ml water. The water phase was neutralized with HCl and the solid was filtered off. The product was dissolved in ethyl acetate and was washed with water. The organic phase was dried with magnesium sulfate and the solvent was distilled off. Column chromatography on silica gel with heptane/toluene 2/1 gave the product. Yield 3.6 g $^1$H MMR (400 MHz, $CDCl_3$): 8.18 (d, 1H), 8.15-8.18 (m, 1H), 7.78-7.84 (m, 1H), 7.66-7.70 (m, 2H), 7.53-7.63 (m, 4H), 7.29-7.48 (m, 7H), 6.90 (td, 1H), 6.03 (d,1H).

**Synthetic example 6**

a.) 2-(5H-Benzo[4,5]thieno[3,2-c]carbazol-5-yl)-6-(13-phenylbenzo[4',5']imidazo[1',2':1,2]imidazo[4,5-a]carbazol-1(13H)-yl)benzonitrile

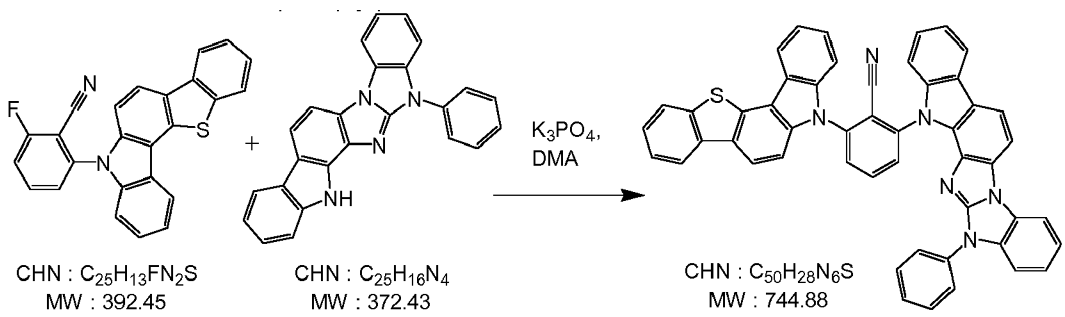


1.20 g (3.22 mmol) 7-Phenyl-7,14-dihydrobenzo[4',5']imidazo[2',1':2,3]imidazo[4,5-a]carbazole, 1.15 g (2.93 mmol) 2-(5H-benzo[4,5]thieno[3,2-c]carbazol-5-yl)-6-fluorobenzonitrile and 1.87 mg (8.79 mmol) potassium carbonate tribasic were dissolved in 30 ml NMP. The reaction mixture was stirred at 100 °C for 17 h.

The reaction mixture was poured on water and the product was filtered off. The product was washed with methanol. The product was crystalized from toluene. Yield 1.50 g (69 %)
MS(ESI) *m*/*z* $[M+H]^+$ : 745

b.) 7-Phenyl-7,14-dihydrobenzo[4',5']imidazo[2',1':2,3]imidazo[4,5-a]carbazole

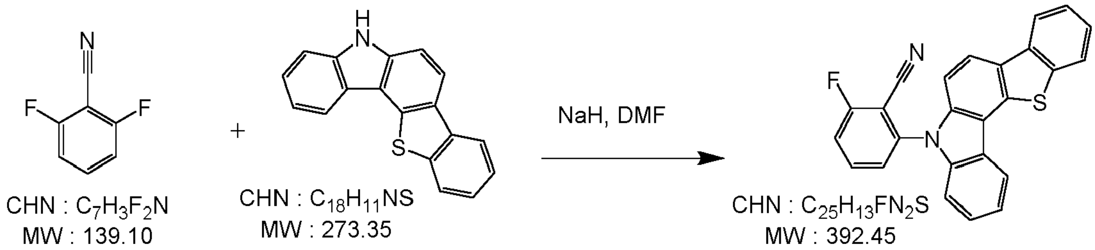


1.87 g (6.83 mmol) 5H-Benzo[4,5]thieno[3,2-c]carbazole, 1.00 g (7.19 mmol) 2,6-difluorobenzonitrile and 4.58 g (21.6 mmol) potassium phosphate tribasic were dissolved in 20 ml DMA. The reaction mixture was stirred at 50 °C for 17 h.

The reaction mixture was poured on water and the product was filtered off. Column chromatography on silica gel with dichloromethane/methanol 95/5 gave the product.
$^1$H MMR (400 MHz, $CDCl_3$): 8.32-8.38 (m, 1H), 8.23-8.26 (m, 2H), 8.01-8.04 (m, 1H), 7.86-7.94 (m, 1H), 7.57-7.44 (m, 6H), 7.23-7.39 (m, 2H).

**Synthetic example 7**

a.) 4-(13-Phenylbenzo[4',5']imidazo[1',2':1,2]imidazo[4,5-a]carbazol-1(13H)-yl)isophthalonitrile

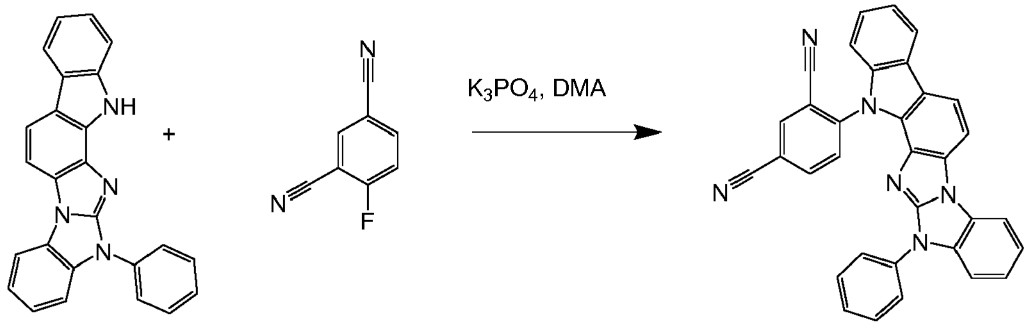


CHN : $C_{25}H_{16}N_4$ MW : 372.43    CHN : $C_8H_3FN_2$ MW : 146.12    CHN : $C_{33}H_{18}N_6$ MW : 498.55

2.00 g (5.37 mmol) 7-Phenyl-7,14-dihydrobenzo[4',5']imidazo[2',1':2,3]imidazo[4,5-a]carbazole, 942 mg (6.44 mmol) 4-fluoroisophthalonitrile and 3.42 g (16.1 mmol) potassium phosphate tribasic in 50 ml DMA were stirred under nitrogen at 100 °C for 5 h.

The reaction mixture was poured on water and the product was filtered off. The product was washed with water and methanol. Column chromatography on silica gel with dichloromethane/heptan 3/2 and than 3/2 gave the product. 1.75 g (65 %).

$^1$H MMR (400 MHz, THF-d6): 8.56 (d, 1H), 8.24 (dt, 1H), 8.21 (dd, 1H), 8.14-8.17 (m, 1H), 8.12 (d, 1H), 8.05 (d, 1H), 8.02 (d,1H), 7.95-7.99 (m, 2H), 7.81-7.84 (m, 1H), 7.60-7.64 (m, 2H), 7.35-7.47 (m, 6H).

**Synthetic example 8**

a.) 4-(7-Phenylbenzo[4',5']imidazo[2',1':2,3]imidazo[4,5-a]carbazol-14(7H)-yl)iso-phthalonitrile

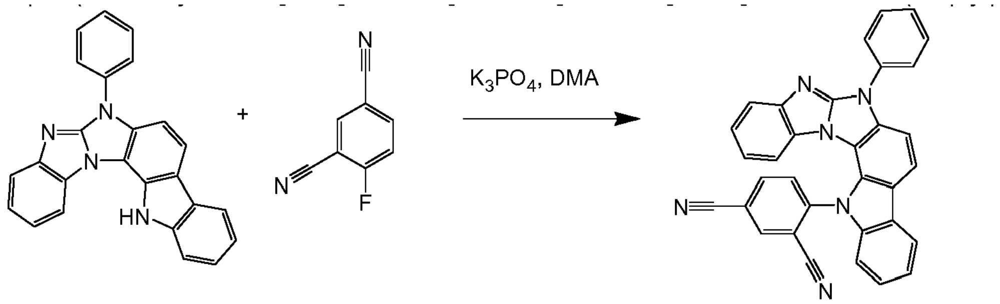


CHN : $C_{25}H_{16}N_4$ MW : 372.43    CHN : $C_8H_3FN_2$ MW : 146.12    CHN : $C_{33}H_{18}N_6$ MW : 498.55

2.00 g (5.37 mmol) 7-Phenyl-7,14-dihydrobenzo[4',5']imidazo[2',1':2,3]imidazo[4,5-a]carbazole, 942 mg (6.44 mmol) 4-fluoroisophthalonitrile and 3.42 g (16.1 mmol) potassium phosphate in 50 ml DMA were stirred under nitrogen at 100 °C for 5 h.

The reaction mixture was poured on water and the product was filtered off. The product was washed with water and methanol. Column chromatography on silica gel with toluene/ethyl acetate 10/1 gave the product. 1.88 g (70 %)

$^1$H MMR (400 MHz, THF-d6): 8.62 (d, 1H), 8.25-8.27 (m, 1H), 8.23 (d, 1H), 8.02 (dd, 1H), 7.96-7.98 (m, 2H), 7.90 (d, 1H), 7.67-7.72 (m, 4H), 7.44-7.56 (m, 4H), 7.13 (td, 1H), 6.74 (td, 1H), 6.30 (dt, 1H).

**Synthetic example 9**

a.) 2-(12H-Benzo[4,5]thieno[2,3-a]carbazol-12-yl)-6-(13-phenylbenzo[4',5']imidazo[1',2':1,2]imidazo[4,5-a]carbazol-1(13H)-yl)benzonitrile

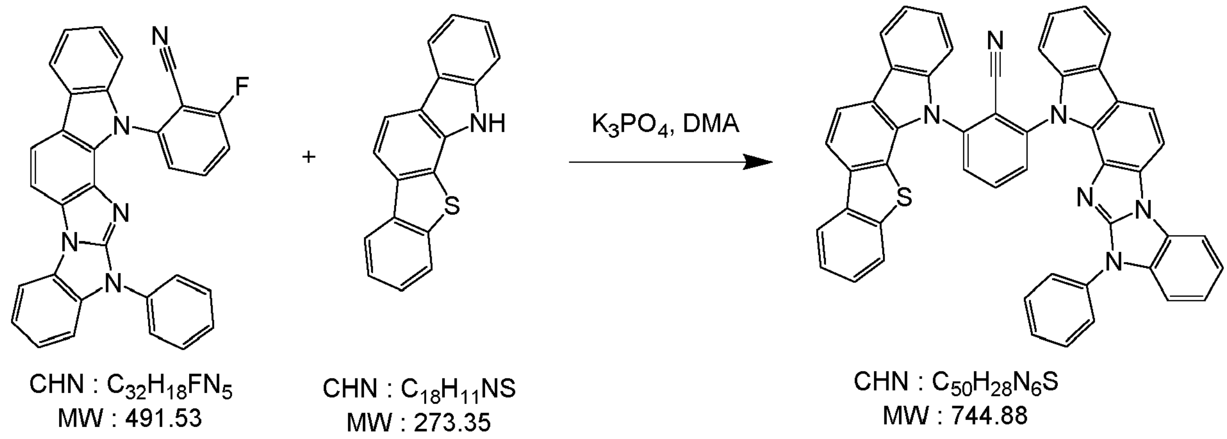


2.00 g (4.07 mmol) 2-Fluoro-6-(13-phenylbenzo[4',5']imidazo[1',2':1,2]imidazo[4,5-a]carbazol-1(13H)-yl)benzonitrile, 1.17 g (4.27 mmol) 12H-benzo[4,5]thieno[2,3-a]carbazole and 2.59 g ( 12.2 mmol) potassium phosphate tribasic in 50 ml DMA were stirred at 100 °C for 5 h under nitrogen.

The reaction mixture was poured on methanol and the product was filtered off. Column chromatography on silica gel with dichloromethane/heptane 2/3 and than 4/1 gave the product. 2.06 g (68 %).
MS(ESI) *m/z* $[M+H]^+$ : 746 (M+1)

b.) 2-Fluoro-6-(13-phenylbenzo[4',5']imidazo[1',2':1,2]imidazo[4,5-a]carbazol-1(13H)-yl)benzonitrile

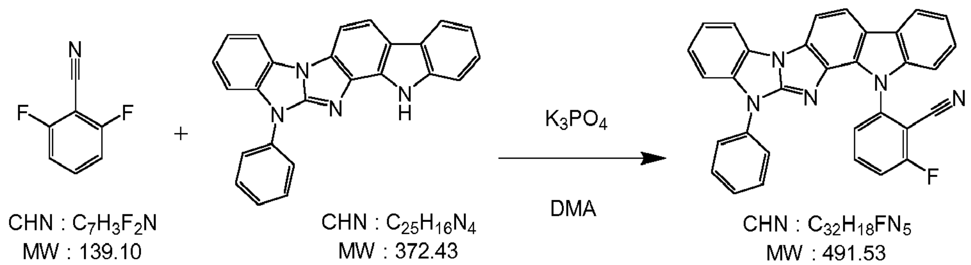


3.20 g (8.59 mmol) 7-Phenyl-7,14-dihydrobenzo[4',5']imidazo[2',1':2,3]imidazo [4,5-a]carbazole, 2.39 g (17.2 mmol) 2,6-difluorobenzonitrile, potassium phosphate tribasic in 50 ml DMA were stirred at 80 °C for 17 h nitrogen.

The reaction mixture was poured on water and the product was filtered off. The product was washed with water and ethanol. 3.82 g (91 %)
$^1$H MMR (400 MHz, THF-d6): 8.23 (td, 1H), 8.13-8.15 (m, 1H), 8.11 (d, 1H), 8.00-8.03 (m, 3H), 7.79-7.87 (m, 2H), 7.56-7.65 (m, 3H), 7.52 (td, 1H), 7.33-7.46 (m, 6H).

**Synthesis N-Phenyl-benzimidazolo[1,2-a]benzimidazole**

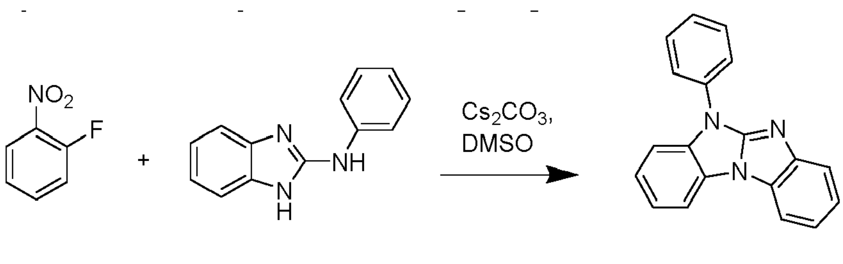


1.05 g (5.00 mmol) N-Phenyl-1H-benzimidazol-2-amine, 776 mg (5.50 mmol) 2-fluorenitrobenzene and 4.07 g (12.5 mmol) cesium carbonate in 10 ml DMSO were stirred at 80 °C under nitrogen. After 1 h the reaction temperature

was raced to 160 °C. After 2 h the reaction mixture was poured on water and the product was filtered off. The product was decocted in ethanol. Yield 660 mg. The NMR of the product is identical with the NMR given in WO2017056052A1. 1H MMR (400 MHz, $CDCl_3$): 5 7.81-7.88 (m, 5H), 7.57-7.67 (m, 2H), 7.45-7.50 (m, 1H), 7.31-7.40 (m, 4H)

**Synthesis of reference compound Ref. 1**

The reference compound Ref. 1 was prepared as described in WO2017115596A1.

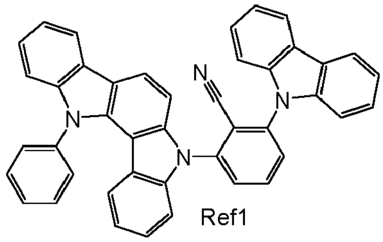

Ref1

**Evaluation of Compounds**

Next, properties of the compounds used in Example were measured. A measurement method and a calculation method are shown below.

**1. Example 1**

**1.1 Photoluminescence Data**

The PLQY was measured in the following manner

PLQY was measured in toluene solution at concentration of $10^{-4}$ using FP-8300 JASCO Spectrofluorometer with 100mm ϕ integration sphere IFL-835.
Before PLQY measurements, the solutions were deoxygenated with Argon to eliminate the deactivation of triplets.

Table 1:

| Example | Solvent | PLQY | · ST | $k_{RISC}$ / s-1 |
|---|---|---|---|---|
| Example 1 | toluene | 61 % | 0.03 eV | 3.91E+05 |
| Reference Example1 | toluene | 47 % | 0.13 eV | 2.12E+05 |
| Reference Example2 | toluene | 68 % | 0.19 eV | 8.76E+04 |

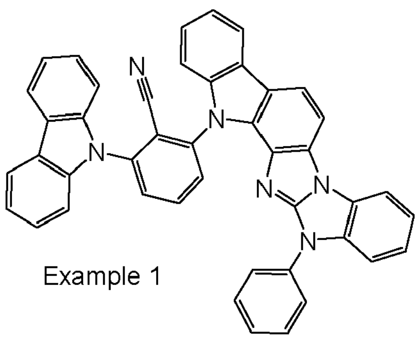

Example 1

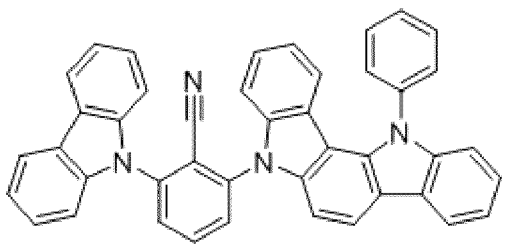

Reference Example 1

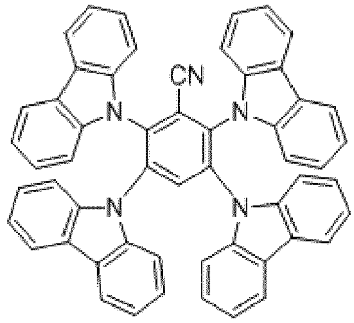

Reference Example 2

**1.2 Device Application Data (inventive compound as TADF emitter (dopant))**

Evaluation Results of Dopant Type (DPEPO Host)

*Preparation and Evaluation of Organic EL Device*

The organic EL device was prepared and evaluated as follows.

A glass substrate (size: 25 mm.times.75 mm.times.1.1 mm thick, manufactured by Geomatec Co., Ltd.) having an ITO transparent electrode (anode) was ultrasonic-cleaned in isopropyl alcohol for five minutes, and then UV/ozone-cleaned for 30 minutes. The film of ITO was 130 nm thick.

After the glass substrate having the transparent electrode line was cleaned, the glass substrate was mounted on a substrate holder of a vacuum evaporation apparatus.

Initially, a compound **HI** was vapor-deposited on a surface of the glass substrate where the transparent electrode line was provided in a manner to cover the transparent electrode, thereby forming a 5-nm-thick hole injecting layer.

Next, the compound **HT-1** was vapor-deposited on the hole injecting layer to form a 110-nm-thick first hole transporting layer on the HI film.

Next, the compound **HT-2** was deposited on the first hole transporting layer to form a 15-nm-thick second hole transporting layer.

Next, the compound **mCP** was deposited on the first hole transporting layer to form a 5-nm-thick second hole transporting layer.

Further, the host compound **H-1** (the first compound) and the dopant compound **D-1** (the second compound) were co-deposited on the second hole transporting layer to form a 25-nm-thick emitting layer. A concentration of the compound D-1 in the emitting layer was given at 24 mass %.
Next, a compound **HB** was deposited on the emitting layer to form a 5-nm-thick blocking layer.

Next, a compound **ET-1** was deposited on the blocking layer to form a 35-nm-thick electron transporting layer.

Lithium fluoride (LiF) was then deposited on the electron transporting layer to form a 1-nm-thick electron injecting electrode (cathode).

A metal aluminum (Al) was then deposited on the electron injecting electrode to form an 80-nm-thick metal Al cathode.

A device arrangement of the organic EL device of **Example 1** is shown in symbols as follows. ITO(130)/HI(5)/HT-1(110)/HT-2(15)/ mCP(5)/H-1: D-1 (25, 24%)/HB(5)/ET(35)/LiF(1)/Al(80)

Numerals in parentheses represent the film thickness (unit: nm). The numerals in the form of percentage in parentheses indicate the ratio (mass %) of the second compound in the emitting layer (the sum of all components in the emitting layer is 100 wt%).

Table 2:

| TADF-dopant | Amount [wt%] | V @10mA | EQE @0.1mA | EQE @10mA | $\lambda_p$[1)] @10mA |
|---|---|---|---|---|---|
| Example 1 | 24 | 5.1 | 13.4 | 10.2 | 461 |
| Reference Example 1 | 24 | 5.1 | 11.2 | 7.7 | 458 |
| Reference Example 2 | 24 | 5.0 | 12.1 | 6.2 | 458 |
| 1) $\lambda_p$ emission wavelength | | | | | |

Higher EQE (at 1 and especially at 10 mA) for the inventive example was measured compared with the comparative examples.

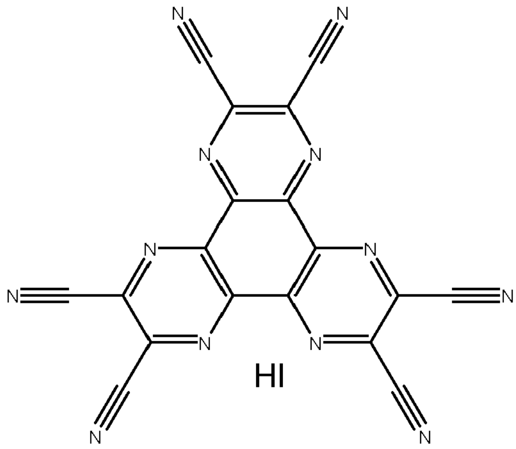
HI
;

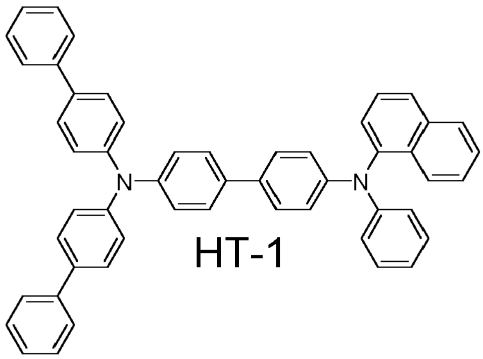
HT-1
;

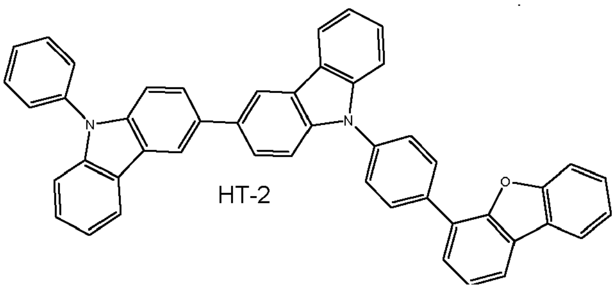
HT-2

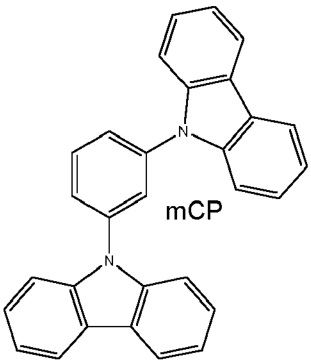
mCP
;

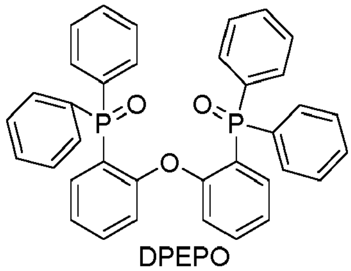
DPEPO
H-1
,

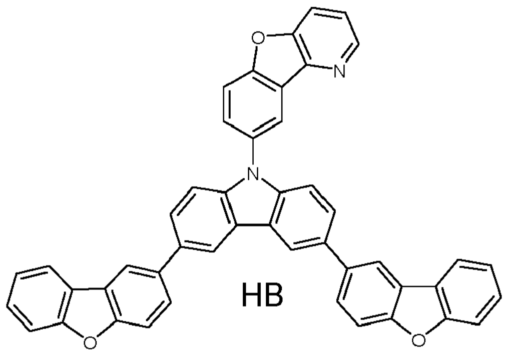
HB

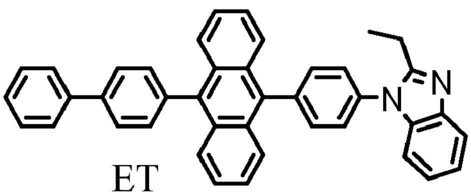
ET

## 2. Example 2

### 2.1 Photoluminescence Data

The PLQY (photoluminescence quantum yield) was measured in the following manner

A solution 5 [μmol/L] of the TADF-BD in the solvent in the table was measured for PLQY by using an absolute PL quantum yield spectrometer (Quantaurus-QY(Model:C11347) manufactured by Hamamatsu Photonics K.K.). The

PLQY value was are given in the table 3:

Table 3

| Example | Solvent | PLQY | · ST |
|---|---|---|---|
| Reference Example 3 | toluene | 61 % | -0.12 |
| Example 2 | toluene | 87 % | 0.00 |
| Example 3 | toluene | 100 % | 0.05 |

Higher PLQY measured for the compounds of examples 2 and 3 compared with the compound of reference example 3.

**2.2 Device Application Data (inventive compound as TADF host)**

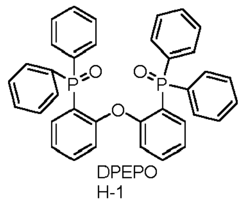

DPEPO
H-1

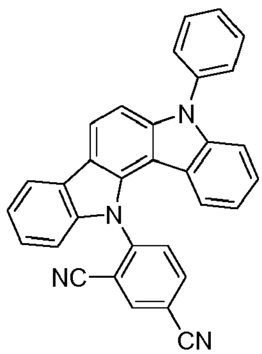

Reference Example 3

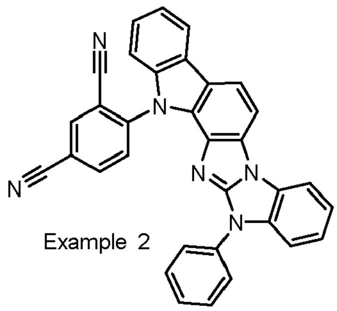

Example 2

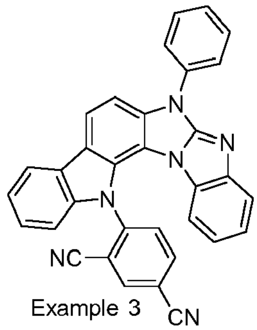

Example 3

An organic EL device of Example 2 was prepared in the same manner as the organic EL device of Example 1 except that a process for forming the emitting layer was changed as follows. In Example 2, the compound H-1 (the first compound), the compound BD(the second compound) and a compound CH-1 (the third compound) were co-deposited on the second hole transporting layer to form a 25-nm-thick emitting layer. The concentration of the compound BD in the emitting layer was given at 1 mass % and the concentration of the compound H-1 in the emitting layer was given at 50 mass %.

A device arrangement of the organic EL device of Example 2 is shown in symbols as follows. ITO(130)/HI(5)/HT-1(110)/HT-2(15)/CH-1: H-1: BD (25, 50%, 1%)/HB(5)/ET(35)/LiF(1)/Al(80)

Numerals in parentheses represent a film thickness (unit: nm). The numerals in the form of percentage in parentheses indicate the ratio (mass %) of the first compound and the ratio (mass %) of the second compound in the emitting layer.

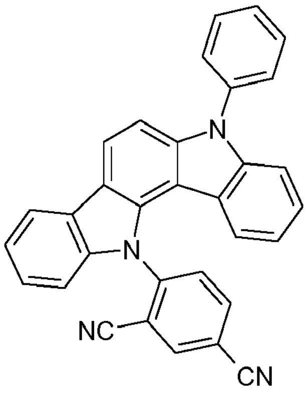 respectively 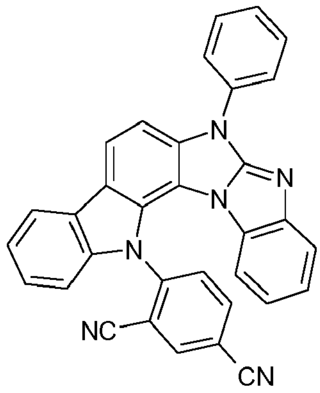 = **H-1 = TADF (Material)**

Reference Example 3 Example 3

Table 4:

| TADF (Material) | BD (Material) | CIEx @10mA | CIEy @10mA | EQE @0.1mA | EQE @10mA | Λp @10mA |
|---|---|---|---|---|---|---|
| Reference Example 3 | compound A | 0.175 | 0.288 | 15.1 | 8.9 | 466 |
| Example 3 | compound A | 0.162 | 0.263 | 15.4 | 10.6 | 467 |

Higher EQE (at 1 and especially at 10 mA) for the inventive example was measured compared with the comparative example.

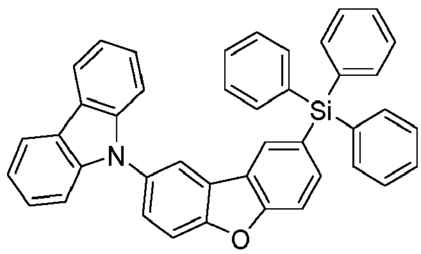

CH-1

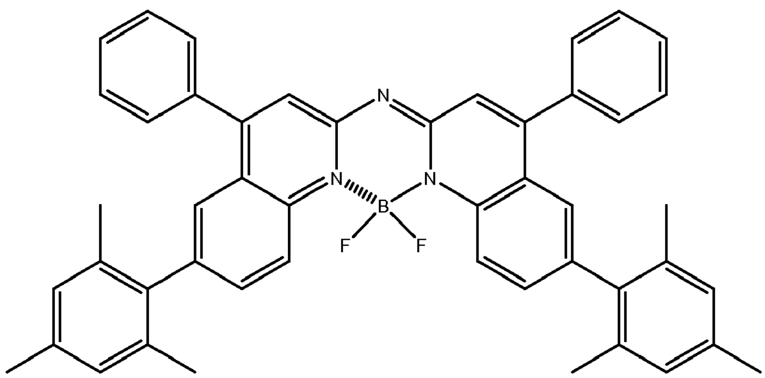

Compound A

### 3. Example 3

### 3.1 Photoluminescence Data

The PLQY was measured in the following manner.

A solution 5 [μmol/L] of the TADF-BD in the solvent in the table was measured for PLQY by using an absolute PL quantum yield spectrometer (Quantaurus-QY(Model:C11347) manufactured by Hamamatsu Photonics K.K.). The PLQY value was are given in the table 5:

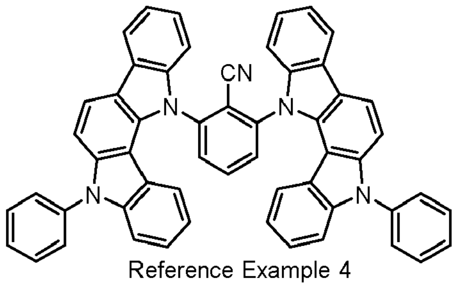

Reference Example 4

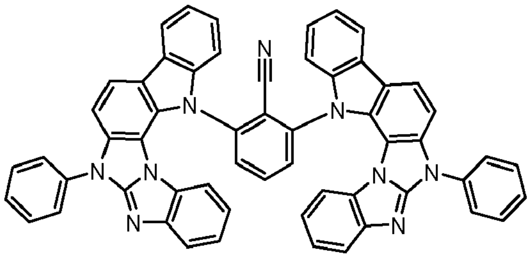

Example 4

Table 5

| Example | Solvent | PLQY | · ST | $k_{RISC}$ / s-1 |
|---|---|---|---|---|
| Example 4 | toluene | 41 % | 0.16 eV | 1.18E+05 |
| Reference Example 4 | toluene | 27 % | 0.14 eV | 8.99E+04 |

Higher PLQY and $k_{RISC}$ measured for the compound of example 4 compared with the compound of reference example 4.

## Claims

**1.** A compound of formula (I)

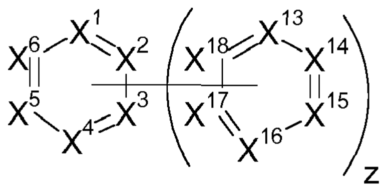

(I)

wherein

z is 0 or 1, preferably 0;
$X^1$ is $CR^{1a}$ or N;
$X^2$ is $CR^{2a}$ or N;
$X^3$ is $CR^{3a}$ or N;
$X^4$ is $CR^{4a}$ or N;
$X^5$ is $CR^{5a}$ or N;
$X^6$ is $CR^{6a}$ or N;

wherein

0, 1 or 2, preferably 0 or 1, more preferably 0 of the groups $X^1$, $X^2$, $X^3$, $X^4$, $X^5$ and $X^6$ are N;
$R^{1a}$, $R^{2a}$, $R^{3a}$, $R^{4a}$, $R^{5a}$ and $R^{6a}$ are each independently CN, $R^A$, $D^1$ or $D^2$;

wherein

1, 2, 3, 4 or 5, preferably 1, 2, 3 or 4, more preferably 1 or 2 of the residues $R^{1a}$, $R^{2a}$, $R^{3a}$, $R^{4a}$, $R^{5a}$ and $R^{6a}$ are CN;
0, 1 or 2 of the residues $R^{1a}$, $R^{2a}$, $R^{3a}$, $R^{4a}$, $R^{5a}$ and $R^{6a}$ are $R^A$;

0 or 1, 2 preferably 0 or 1 of the residues $R^{1a}$, $R^{2a}$, $R^{3a}$, $R^{4a}$, $R^{5a}$ and $R^{6a}$ are $D^2$; and
1 of the residues $R^{1a}$, $R^{2a}$, $R^{3a}$, $R^{4a}$, $R^{5a}$ and $R^{6a}$ is $D^1$;
$X^{13}$ is $CR^{13a}$ or N;
$X^{14}$ is $CR^{14a}$ or N;
$X^{15}$ is $CR^{15a}$ or N;
$X^{16}$ is $CR^{16a}$ or N;
$X^{17}$ is $CR^{17a}$ or N;
$X^{18}$ is $CR^{18a}$ or N;

wherein

0, 1 or 2, preferably 0 or 1, more preferably 0 of the groups $X^{13}$, $X^{14}$, $X^{15}$, $X^{16}$, $X^{17}$ and $X^{18}$ are N;
$R^{13a}$, $R^{14a}$, $R^{15a}$, $R^{16a}$, $R^{17a}$ and $R^{18a}$ are each independently CN, $R^A$, $D^1$ or $D^2$;

wherein

1, 2, 3 or 4, preferably 1 or 2 of the residues $R^{13a}$, $R^{14a}$, $R^{15a}$, $R^{16a}$, $R^{17a}$ and $R^{18a}$ are CN;
0, 1 or 2 of the residues $R^{13a}$, $R^{14a}$, $R^{15a}$, $R^{16a}$, $R^{17a}$ and $R^{18a}$ are $R^A$;
0 or 1 of the residues $R^{13a}$, $R^{14a}$, $R^{15a}$, $R^{16a}$, $R^{17a}$ and $R^{18a}$ are $D^1$; and
1 of the residues $R^{13a}$, $R^{14a}$, $R^{15a}$, $R^{16a}$, $R^{17a}$ and $R^{18a}$ is $D^2$;
$D_2$ is a donor group,
$D_1$ is a group of formula (III)

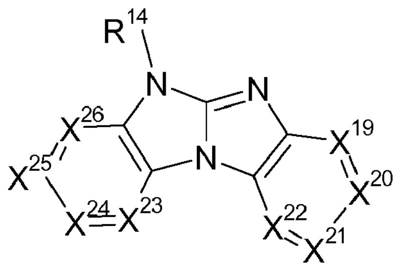

(III)

wherein

$X^{19}$ is $CR^1$ or N;
$X^{20}$ is $CR^2$ or N;
$X^{21}$ is $CR^3$ or N;
$X^{22}$ is $CR^4$ or N;
$X^{23}$ is $CR^5$ or N;
$X^{24}$ is $CR^6$ or N;
$X^{25}$ is $CR^7$ or N;
$X^{26}$ is $CR^8$ or N;

wherein
0, 1 or 2, preferably 0 or 1, more preferably 0 of the groups $X^{19}$, $X^{20}$, $X^{21}$, $X^{22}$, $X^{23}$, $X^{24}$, $X^{25}$ and $X^{26}$ are N;
wherein
at least two adjacent residues $R^1$, $R^2$, $R^3$, $R^4$, $R^5$, $R^6$, $R^7$ and $R^8$ of at least two adjacent groups $CR^1$, $CR^2$, $CR^3$, $CR^4$, $CR^5$, $CR^6$, $CR^7$ and $CR^8$ together form a ring of formula (IV)

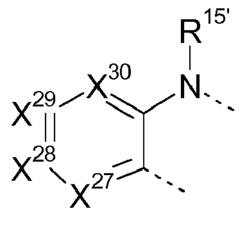

(IV)

wherein

$X^{27}$ is $CR^9$ or N;
$X^{28}$ is $CR^{10}$ or N;
$X^{29}$ is $CR^{11}$ or N;
$X^{30}$ is $CR^{12}$ or N;

wherein
0, 1 or 2, preferably 0 or 1, more preferably 0 of the groups $X^{27}$, $X^{28}$, $X^{29}$ and $X^{30}$ are N; wherein
the dotted lines are bonding sites to the group of formula (III), and
the remaining residues $R^1$ to $R^8$ and $R^9$ to $R^{12}$ are independently of each other H, -CN, a $C_1$-$C_{25}$alkyl group which is unsubstituted or substituted by at least one group E and/or interrupted by at least one group D, a $C_6$-$C_{40}$aryl group which is unsubstituted or substituted by at least one group E, a $C_1$-$C_{24}$heteroaryl group which is unsubstituted or substituted by at least one group E, a $C_7$-$C_{25}$aralkyl group which is unsubstituted or substituted by at least one group E, a $C_5$-$C_{12}$cycloalkyl group which is unsubstituted or substituted by at least one group E, $-OR^{21}$, $-SR^{21}$, $-NR^{17}R^{18}$, $-COR^{20}$, $-COOR^{19}$, $-CONR^{17}R^{18}$, $-SiR^{22}R^{23}R^{24}$,$-POR^{25}R^{27}$, or halogen, or
at least two of the remaining residues $R^1$ to $R^8$ and $R^9$ to $R^{12}$ if present at adjacent carbon atoms together form at least one $C_6$-$C_{18}$aryl or $C_1$-$C_{24}$heteroaryl ring or ring system,
$R^A$ is H, a $C_1$-$C_{25}$alkyl group which is unsubstituted or substituted by at least one group E and/or interrupted by at least one group D, a $C_6$-$C_{40}$aryl group which is unsubstituted or substituted by at least one group E, a $C_1$-$C_{24}$heteroaryl group which is unsubstituted or substituted by at least one group E, a $C_7$-$C_{25}$aralkyl group which is unsubstituted or substituted by at least one group E, a $C_5$-$C_{12}$cycloalkyl group which is unsubstituted or substituted by at least one group E, $-OR^{21}$, $-SR^{21}$, $-NR^{17}R^{18}$, $-COR^{20}$, $-COOR^{19}$, $-CONR^{17}R^{18}$,$-SiR^{22}R^{23}R^{24}$, $-POR^{25}R^{27}$, or halogen, or
at least two of the residues $R^A$ if present at adjacent carbon atoms together form at least one $C_6$-$C_{18}$aryl ring or ring system which is unsubstituted or substituted by at least one group E or $C_1$-$C_{24}$heteroaryl ring or ring system which is unsubstituted or substituted by at least one group E,
$R^{14}$ and $R^{15'}$ are each independently a $C_6$-$C_{40}$aryl group which is unsubstituted or substituted by at least one group E or a $C_1$-$C_{24}$heteroaryl group which is unsubstituted or substituted by at least one group E,
wherein one of $R^{14}$ and $R^{15'}$ is a group $-(M)_m$---,
wherein the dotted line is a bonding site to formula (I),
M is a $C_6$-$C_{40}$arylene group which is unsubstituted or substituted by at least one group E, a $C_1$-$C_{24}$heteroarylene group which is unsubstituted or substituted by at least one group E, a $C_1$-$C_{25}$alkylene group which is unsubstituted or substituted by at least one group E and/or interrupted by at least one group D, or a $C_2$-$C_{25}$alkenylene group which is unsubstituted or substituted by at least one group E and/or interrupted by at least one group D,
m is 0, 1 or 2, preferably 0 or 1, more preferably 0,
D is -CO-, -COO-, -S-, -SO-, $-SO_2-$, -O-, $-CR^{15}=CR^{16}-$, $-NR^{17}-$, $-SiR^{22}R^{23}-$, $-POR^{25}-$, -C≡C-,
E is $-OR^{21}$, $-SR^{21}$, $-NR^{17}R^{18}$, $-COR^{20}$, $-COOR^{19}$, $-CONR^{17}R^{18}$, -CN, $-SiR^{22}R^{23}R^{24}$,$-POR^{25}R^{27}$, halogen, preferably F,
a $C_6$-$C_{40}$aryl group which is unsubstituted or is substituted by at least one group selected from a $C_1$-$C_{18}$alkyl group, a $C_1$-$C_{18}$alkoxy group, a $C_1$-$C_{18}$alkyl group, which is interrupted by at least one O, a $C_6$-$C_{18}$aryl group, which is in turn unsubstituted or substituted by at least one $C_1$-$C_{18}$alkyl group or at least one $C_1$-$C_{18}$alkoxy group, a $C_5$-$C_{12}$cycloalkyl group, which is in turn unsubstituted or substituted by at least one $C_1$-$C_{18}$alkyl group or at least one $C_1$-$C_{18}$alkoxy group, a $C_1$-$C_{18}$heteroaryl group, which is in turn unsubstituted or substituted by at least one $C_1$-$C_{18}$alkyl group or at least one $C_1$-$C_{18}$alkoxy group, -F, $-CF_3$,$-CF_2CF_3$, $-CF_2CF_2CF_3$, $-CF(CF_3)_2$, $-(CF_2)_3CF_3$ and $-C(CF_3)_3$,
a $C_1$-$C_{18}$ alkyl or a $C_1$-$C_{18}$alkyl group which is interrupted by at least one O,
a $C_1$-$C_{24}$heteroaryl group which is unsubstituted or substituted by at least one group selected from a $C_1$-$C_{18}$alkyl group, a $C_1$-$C_{18}$alkoxy group, a $C_1$-$C_{18}$alkyl group, which is interrupted by at least one O, a $C_6$-$C_{18}$aryl group,

which is in turn unsubstituted or substituted by at least one $C_1$-$C_{18}$alkyl group or at least one $C_1$-$C_{18}$alkoxy group, a $C_5$-$C_{12}$cycloalkyl group, which is in turn unsubstituted or substituted by at least one $C_1$-$C_{18}$alkyl group or at least one $C_1$-$C_{18}$alkoxy group, a $C_1$-$C_{18}$heteroaryl group, which is in turn unsubstituted or substituted by at least one $C_1$-$C_{18}$alkyl group or at least one $C_1$-$C_{18}$alkoxy group, -F, $-CF_3$,$-CF_2CF_3$, $-CF_2CF_2CF_3$, $-CF(CF_3)_2$, $-(CF_2)_3CF_3$ and $-C(CF_3)_3$,
$R^{15}$ and $R^{16}$ are independently of each other H, a $C_6$-$C_{18}$aryl group which is unsubstituted or substituted by at least one $C_1$-$C_{18}$alkyl group or at least one $C_1$-$C_{18}$alkoxy group,
$R^{17}$ and $R^{18}$ are independently of each other H, a $C_6$-$C_{18}$aryl group which is unsubstituted or substituted by at least one $C_1$-$C_{18}$alkyl group or at least one $C_1$-$C_{18}$alkoxy group, a $C_1$-$C_{18}$alkyl group or a $C_1$-$C_{18}$alkyl group, which is interrupted by at least one O, or
$R^{17}$ and $R^{18}$ together form a five or six membered aliphatic, aromatic or heteroaromatic ring, which is unsubstituted or substituted by at least one group E,
$R^{19}$ is H, a $C_6$-$C_{18}$aryl group which is unsubstituted or substituted by at least one $C_1$-$C_{18}$alkyl group or at least one $C_1$-$C_{18}$alkoxy group, a $C_1$-$C_{18}$alkyl group or a $C_1$-$C_{18}$alkyl group, which is interrupted by at least one O,
$R^{20}$ is H or a $C_6$-$C_{18}$aryl group which is unsubstituted or substituted by at least one $C_1$-$C_{18}$alkyl group or at least one $C_1$-$C_{18}$alkoxy group, a $C_1$-$C_{18}$ alkyl group or a $C_1$-$C_{18}$alkyl group, which is interrupted by at least one O,
$R_{21}$ is independently of each other H, a $C_6$-$C_{18}$aryl group which is unsubstituted or substituted by at least one $C_1$-$C_{18}$alkyl group or at least one $C_1$-$C_{18}$alkoxy group, a $C_1$-$C_{18}$alkyl group or a $C_1$-$C_{18}$alkyl group, which is interrupted by at least one O,
$R^{22}$, $R^{23}$ and $R^{24}$ are independently of each other H, a $C_1$-$C_{18}$alkyl group, a $C_6$-$C_{18}$aryl group which is unsubstituted or substituted by at least one $C_1$-$C_{18}$alkyl group, and
$R^{25}$ and $R^{27}$ are independently of each other H, a $C_1$-$C_{18}$alkyl group, a $C_6$-$C_{18}$aryl group which is unsubstituted or substituted by at least one $C_1$-$C_{18}$alkyl group, a $C_7$-$C_{25}$aralkyl which is unsubstituted or substituted by at least one group E, a $C_5$-$C_{12}$cycloalkyl group which is unsubstituted or substituted by at least one group E.

**2.** A compound according to claim 1, having the following formula

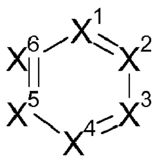
(I*)

wherein

$X^1$ is $CR^{1a}$ or N;
$X^2$ is $CR^{2a}$ or N;
$X^3$ is $CR^{3a}$ or N;
$X^4$ is $CR^{4a}$ or N;
$X^5$ is $CR^{5a}$ or N;
$X^6$ is $CR^{6a}$ or N;

wherein

0, 1 or 2, preferably 0 or 1, more preferably 0 of the groups $X^1$, $X^2$, $X^3$, $X^4$, $X^5$ and $X^6$ are N;
$R^{1a}$, $R^{2a}$, $R^{3a}$, $R^{4a}$, $R^{5a}$ and $R^{6a}$ are each independently CN, $R^A$, $D^1$ or $D^2$;

wherein

1, 2, 3, 4 or 5, preferably 1, 2, 3 or 4, more preferably 1 or 2 of the residues $R^{1a}$, $R^{2a}$, $R^{3a}$, $R^{4a}$, $R^{5a}$ and $R^{6a}$ are CN;
0, 1 or 2 of the residues $R^{1a}$, $R^{2a}$, $R^{3a}$, $R^{4a}$, $R^{5a}$ and $R^{6a}$ are $R^A$;
0 or 1, 2 preferably 0 or 1 of the residues $R^{1a}$, $R^{2a}$, $R^{3a}$, $R^{4a}$, $R^{5a}$ and $R^{6a}$ are $D^2$; and
1 of the residues $R^{1a}$, $R^{2a}$, $R^{3a}$, $R^{4a}$, $R^{5a}$ and $R^{6a}$ is $D^1$.

**3.** The compound according to claim 1 or 2, having the following formula

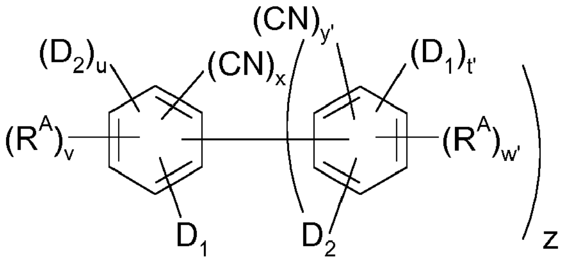

(IA),

preferably formula (I*A),

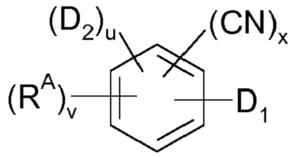

(I*A)

wherein

x is 1, 2, 3, 4 or 5, preferably 1, 2, 3 or 4, more preferably 1 or 2,
v is 0, 1 or 2,
u is 0 or 1, 2 preferably 0 or 1,
wherein the sum of x, v and u is 1, 2, 3, 4 or 5, preferably 1, 2, 3 or 4, more preferably 1, 2 or 3,
y' is 1, 2, 3 or 4, preferably 1 or 2,
w' is 0, 1 or 2,
t' is 0 or 1,
wherein the sum of y', w' and t' is 1, 2, 3 or 4, preferably 1, 2 or 3,
$D_1$ is a group of formula (IIIA)

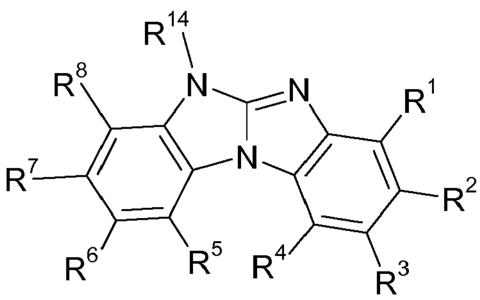

(IIIA)

wherein
at least two adjacent residues $R^1$ to $R^8$ together form a ring of formula (IVA)

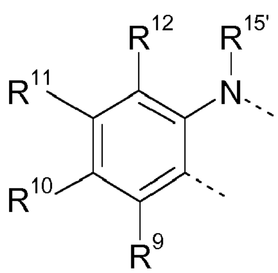

(IVA)

wherein

the dotted lines are bonding sites to the group of formula (IIIA).

**4.** The compound according to claim 3, wherein
x is 1 or 2, preferably 1;
u is 1;
v is 0, 1 or 2, preferably 0 or 1, more preferably 0; and
y' is 1 or 2, preferably 1;
t' is 1; is 0 (claim 7 (IAAA)).
w' is 0, 1 or 2, preferably 0 or 1, more preferably 0.
z is 0.

**5.** The compound according to any one of claims 1 to 4, wherein $R^A$ is hydrogen.

**6.** The compound according to any one of claims 1 to 5, wherein $R^1$ to $R^8$ and $R^9$ to $R^{12}$ are hydrogen.

**7.** The compound according to any one of claims 1 to 6, wherein the compound of formula (IA) is represented by a compound of formula (I*AA), (IAA) or (IAAA)

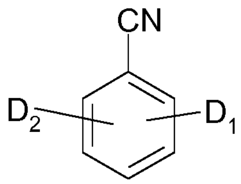

(I*AA),

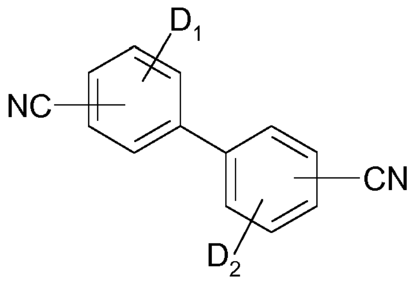

(IAA),

preferably

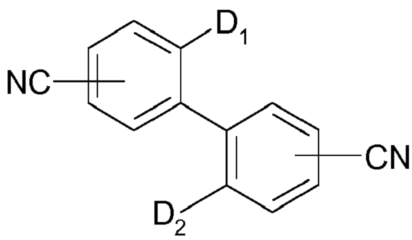

(IAAA).

**8.** The compound according to claim 7, wherein the compound of formula (I*AA) is selected from the group consisting of the following formulae

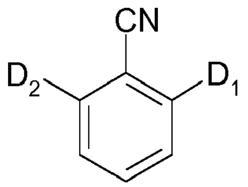 (I*AA-1), 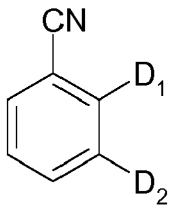 (I*AA-2), 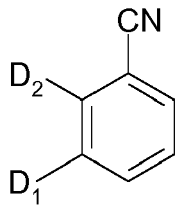 (I*AA-3),

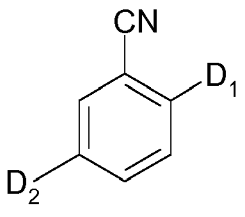 (I*AA-4), 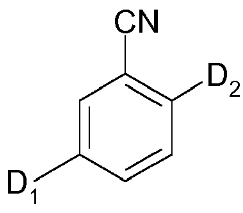 (I*AA-5), 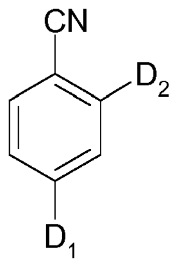 (I*AA-6),

and

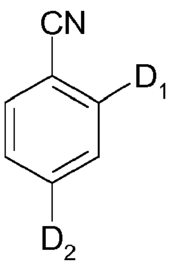 (I*AA-7),

preferably (I*AA1), (I*AA-3), (I*AA-6) and (I*AA-7), more preferably (I*AA-1),
and the compound of formula (IAAA) is selected from the group consisting of the following formulae

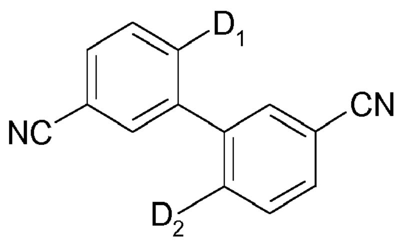 (IIAAA-1), 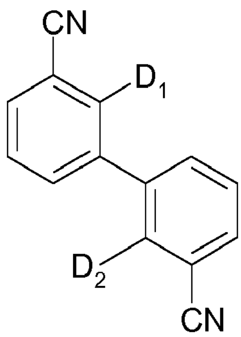 (IIAAA-2),

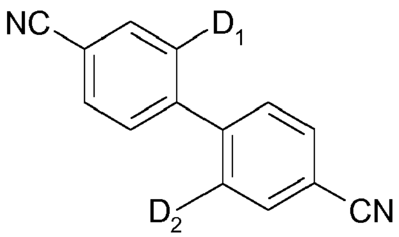 (IIAAA-3), 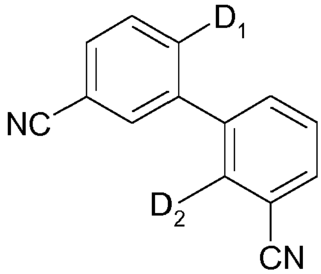 (IIAAA-4),

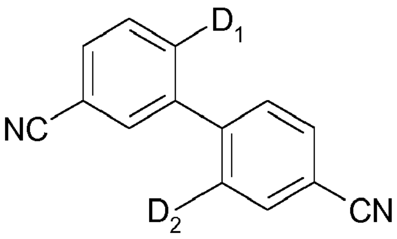 (IIAAA-5), 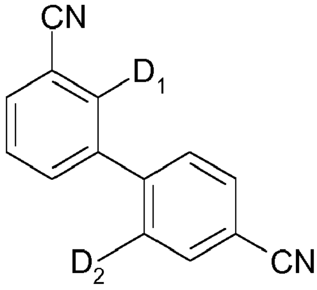 (IIAAA-6),

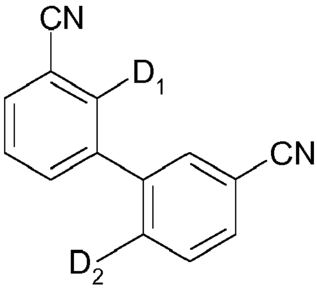 (IIAAA-7), 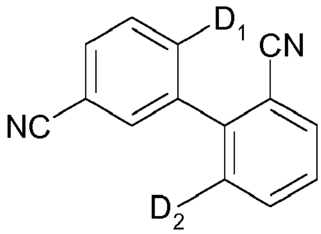 (IIAAA-8)

and

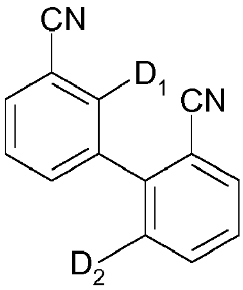 (IIAAA-9),

preferably (IIAAA-1) and (IIAAA-2).

**9.** The compound according to any one of claims 1 to 8, wherein $D_1$ is selected from the group consisting of

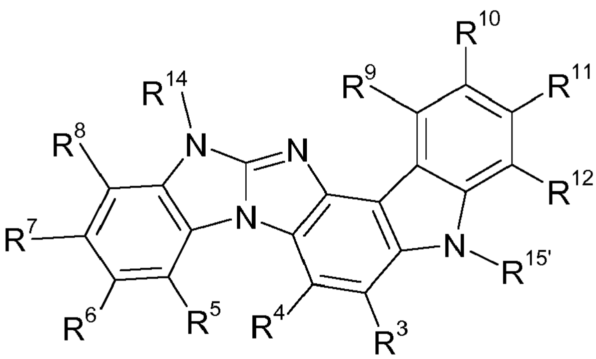 (IIIA-1),

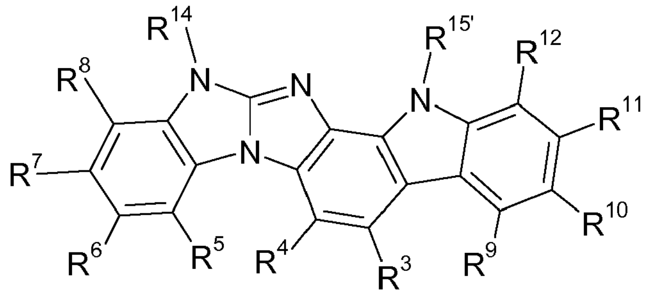

(IIIA-2),

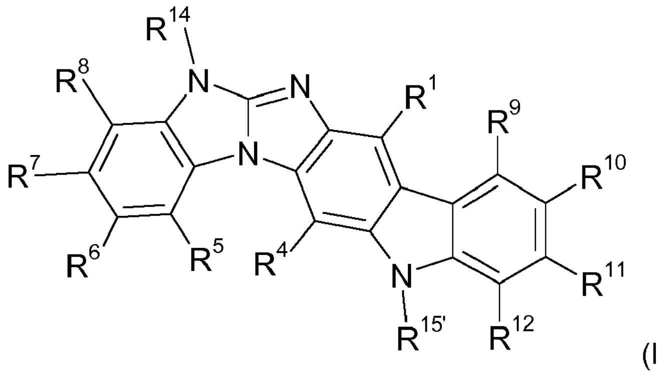

(IIIA-3),

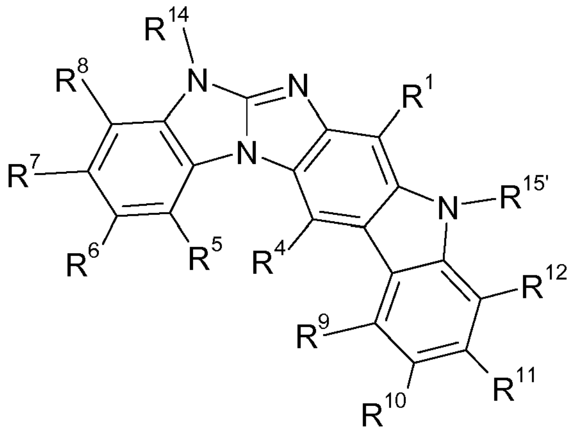

(IIIA-4),

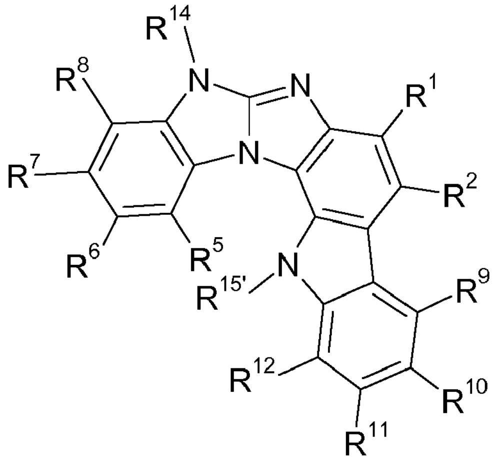

(IIIA-5),

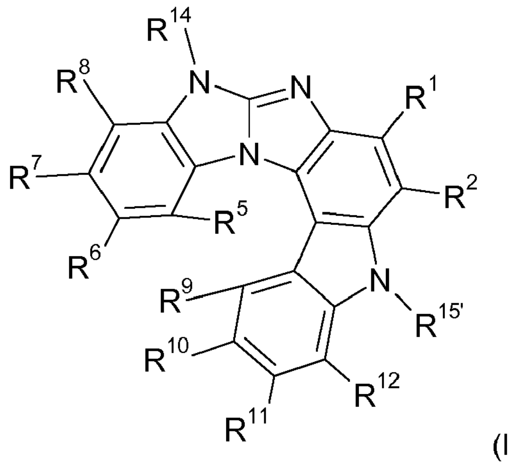

(IIIA-6),

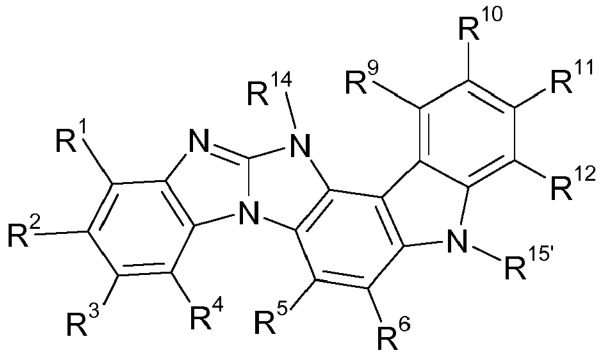

(IIIA-7),

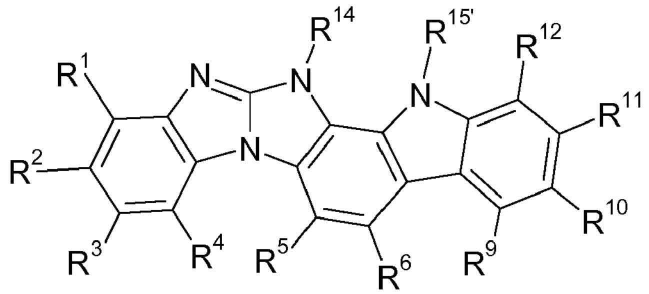

(IIIA-8),

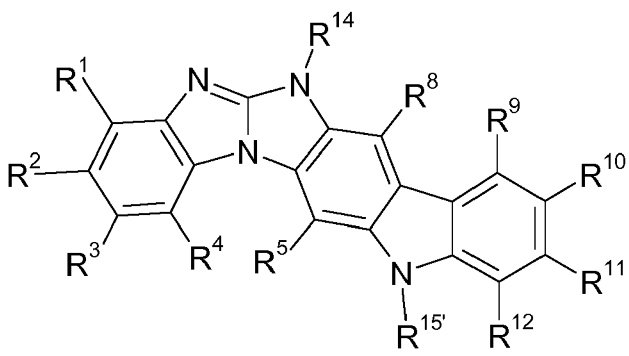

(IIIA-9),

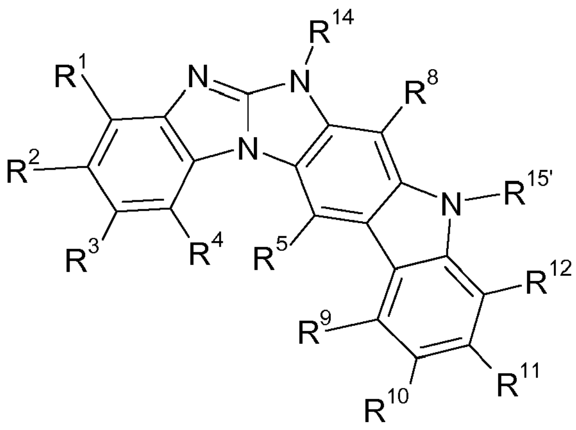

(IIIA-10),

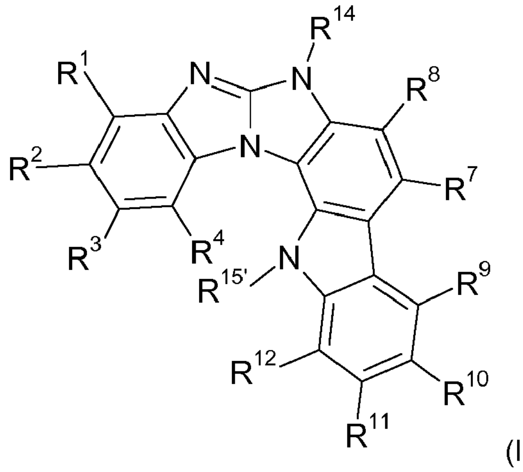

(IIIA-11),

and

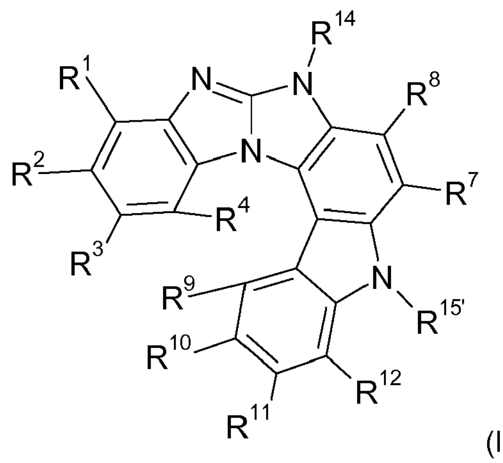

(IIIA-12).

**10.** The compound according to any one of claims 1 to 9, wherein $D_2$ is selected from the groups consisting of (i), (ii), (iii) and (iv)

(v) a group of formula (V),

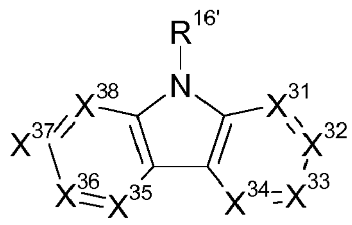

(V)

wherein

$X^{31}$ is $CR^{31}$ or N;
$X^{32}$ is $CR^{32}$ or N;
$X^{33}$ is $CR^{33}$ or N;
$X^{34}$ is $CR^{34}$ or N;
$X^{35}$ is $CR^{35}$ or N;
$X^{36}$ is $CR^{36}$ or N;
$X^{37}$ is $CR^{37}$ or N;
$X^{38}$ is $CR^{38}$ or N;

wherein
0, 1 or 2, preferably 0 or 1, more preferably 0 of the groups $X^{31}$, $X^{32}$, $X^{33}$, $X^{34}$, $X^{35}$, $X^{36}$, $X^{37}$ and $X^{38}$ are N;
or
at least two adjacent residues $R^{31}$, $R^{32}$, $R^{33}$, $R^{34}$, $R^{35}$, $R^{36}$, $R^{37}$ and $R^{38}$ of at least two adjacent groups $CR^{31}$, $CR^{32}$, $CR^{33}$, $CR^{34}$, $CR^{35}$, $CR^{36}$, $CR^{37}$ and $CR^{38}$ together may form a ring of formula (VI)

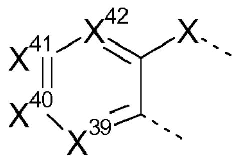

(VI)

wherein

$X^{39}$ is $CR^{39}$ or N;
$X^{40}$ is $CR^{40}$ or N;
$X^{41}$ is $CR^{41}$ or N;
$X^{42}$ is $CR^{42}$ or N;

wherein

0, 1 or 2, preferably 0 or 1, more preferably 0 of the groups $X^{39}$, $X^{40}$, $X^{41}$ and $X^{42}$ are N;
X is O, S, $NR^{17'}$, $CR^{18'}R^{19'}$, $SiR^{18'}R^{19'}$ or $PO(OR^{18'})$; preferably O, S, $NR^{17'}$ or $CR^{18'}R^{19'}$,

wherein
the dotted lines are bonding sites to the group of formula (V), and
the residues $R^{31}$ to $R^{38}$ and $R^{39}$ to $R^{42}$ are independently of each other H, -CN, a $C_1$-$C_{25}$alkyl group which is unsubstituted or substituted by at least one group E and/or interrupted by at least one group D, a $C_6$-$C_{40}$aryl group which is unsubstituted or substituted by at least one group E, a $C_1$-$C_{24}$heteroaryl group which is unsubstituted or substituted by at least one group E, a $C_7$-$C_{25}$aralkyl group which is unsubstituted or substituted by at least one group E, a $C_5$-$C_{12}$cycloalkyl group which is unsubstituted or substituted by at least one group E, $-OR^{21}$, $-SR^{21}$, $-NR^{17}R^{18}$, $-COR^{20}$, $-COOR^{19}$, $-CONR^{17}R^{18}$, $-SiR^{22}R^{23}R^{24}$,$-POR^{25}R^{27}$, or halogen, or
at least two of the remaining residues $R^{31}$ to $R^{38}$ and $R^{39}$ to $R^{42}$ if present at adjacent carbon atoms together may form at least one $C_6$-$C_{18}$aryl ring, which is unsubstituted or substituted by at least one group E or at least one $C_1$-$C_{24}$heteroaryl ring, which is unsubstituted or substituted by at least one group E,
$R^{16'}$ and $R^{17'}$ are each independently a $C_6$-$C_{40}$aryl group which is unsubstituted or substituted by at least one group E or a $C_1$-$C_{24}$heteroaryl group which is unsubstituted or substituted by at least one group E,

$R^{18'}$ and $R^{19'}$ are each independently hydrogen, a $C_1$-$C_{25}$alkyl group which is unsubstituted or substituted by at least one group E and/or interrupted by at least one group D, a $C_6$-$C_{40}$aryl group which is unsubstituted or substituted by at least one group E or a $C_1$-$C_{24}$heteroaryl group which is unsubstituted or substituted by at least one group E,
wherein one of $R^{16'}$ and $R^{17'}$ is a group -$(M)_m$---,
wherein the dotted line is a bonding site to formula (I);
(vi) a group of formula (VII),

---$(M)_m$-$NAr_1Ar_2$ (VII)

wherein

$Ar_1$ and $Ar_2$ are each independently a $C_6$-$C_{40}$aryl group which is unsubstituted or substituted by at least one group E or a $C_1$-$C_{24}$heteroaryl group which is unsubstituted or substituted by at least one group E; or $Ar_1$ and $Ar_2$ may be connected together to form a ring,
wherein the dotted line is a bonding site to formula (I);

(vii) a group ---$(M)_m$-$D_1$,
preferably a group of formula (IIIA), more preferably a group of formula of formula ((IIIA-1), (IIIA-2), (IIIA-3), (IIIA-4), (IIIA-5), (IIIA-6), (IIIA-7), (IIIA-8), (IIIA-9), (IIIA-10), (IIIA-11), (IIIA-12),
wherein the dotted line is a bonding site to formula (I);
and
(viii) a group of formula (VIII),

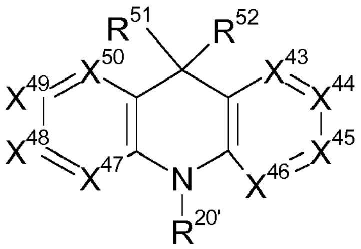

(VIII)

wherein

$X^{43}$ is $CR^{43}$ or N;
$X^{44}$ is $CR^{44}$ or N;
$X^{45}$ is $CR^{45}$ or N;
$X^{46}$ is $CR^{46}$ or N;
$X^{47}$ is $CR^{47}$ or N;
$X^{48}$ is $CR^{48}$ or N;
$X^{49}$ is $CR^{49}$ or N;
$X^{50}$ is $CR^{50}$ or N;

wherein
0, 1 or 2, preferably 0 or 1, more preferably 0 of the groups $X^{43}$, $X^{44}$, $X^{45}$, $X^{46}$, $X^{47}$, $X^{48}$, $X^{49}$ and $X^{50}$ are N;
$R^{43}$ to $R^{50}$ are independently of each other H, -CN, a $C_1$-$C_{25}$alkyl group which is unsubstituted or substituted by at least one group E and/or interrupted by at least one group D, a $C_6$-$C_{40}$aryl group which is unsubstituted or substituted by at least one group E, a $C_1$-$C_{24}$heteroaryl group which is unsubstituted or substituted by at least one group E, a $C_7$-$C_{25}$aralkyl group which is unsubstituted or substituted by at least one group E, a $C_5$-$C_{12}$cycloalkyl group which is unsubstituted or substituted by at least one group E, -$OR^{21}$, - $SR^{21}$, -$NR^{17}R^{18}$, -$COR^{20}$, -$COOR^{19}$, -$CONR^{17}R^{18}$, -$SiR^{22}R^{23}R^{24}$, -$POR^{25}R^{27}$, or halogen, or at least two of the residues $R^{43}$ to $R^{50}$ if present at adjacent carbon atoms together may form at least one $C_6$-$C_{18}$aryl ring, which is unsubstituted or substituted by at least one group E or at least one $C_1$-$C_{24}$heteroaryl ring, which is unsubstituted or substituted by at least one group E
wherein $R^{20'}$ is a group -$(M)_m$---,
wherein the dotted line is a bonding site to formula (I);

$R^{51}$ and $R^{52}$ are each independently hydrogen, a $C_1$-$C_{25}$alkyl group which is unsubstituted or substituted by at least one group E and/or interrupted by at least one group D, a $C_6$-$C_{40}$aryl group which is unsubstituted or substituted by at least one group E or a $C_1$-$C_{24}$heteroaryl group which is unsubstituted or substituted by at least one group E,
or
$R^{51}$ and $R^{52}$ may together form a ring.

**11.** The compound according claim 9 or 10, wherein $D_1$ is a group of formula (IIIA-2).

**12.** The compound according any one of claims 8 or 11, wherein the compound of formula (I*AA-1) is represented by a compound of the following formula

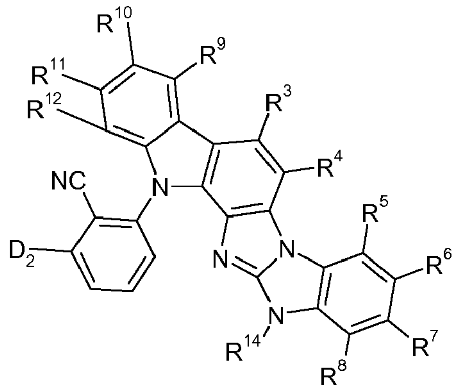

wherein
$D_2$ is a group of formula (V),

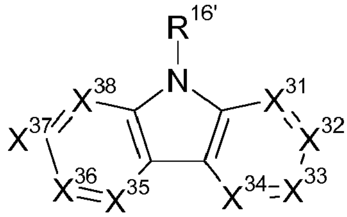

(V).

**13.** A process for the preparation of a compound according to formula (I) as defined in any one of claims 1 to 12, at least comprising the step:

(i) Preparation of a benzimidazolo[1,2-a]benzimidazole intermediate (IIIa) or (III'a) comprising the steps

(ia) Preparation of a nitro group comprising intermediate (C) by reacting a ortho-nitro halobenzene (A) with a benzimidazole derivative (B)

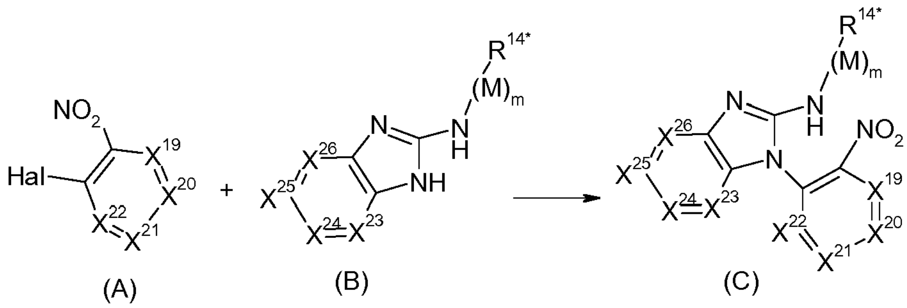

(A) (B) (C)

or

(I'a) Preparation of a nitro group comprising intermediate (C') by reacting a ortho-nitro halobenzene (A) with a benzimidazole derivative (B')

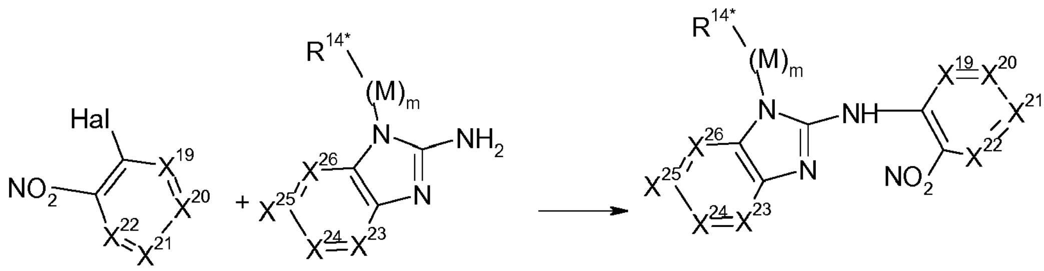

(A) (B') (C')

and

(ib) Cyclization of the intermediate (C) to the benzimidazolo[1,2-a]benzimidazole intermediate (IIIa)

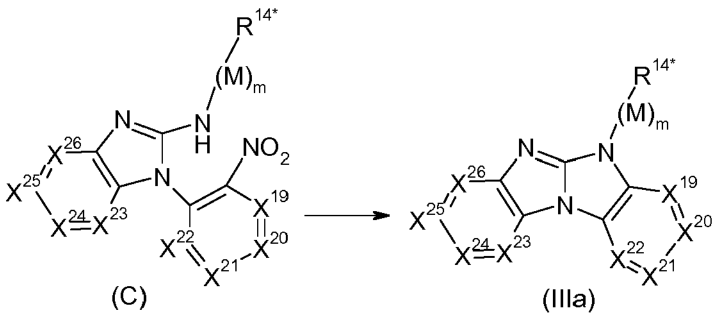

(C) (IIIa)

or

(I'b) Cyclization of the intermediate (C') to the benzimidazolo[1,2-a]benzimidazole intermediate (III'a)

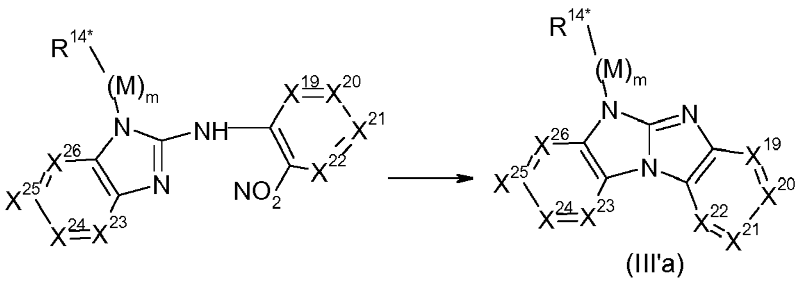

(III'a)

(C')

wherein

Hal is F, Cl or Br, preferably F or Cl, more preferably F;
$X^{19}$ is $CR^1$ or N;
$X^{20}$ is $CR^2$ or N;
$X^{21}$ is $CR^3$ or N;
$X^{22}$ is $CR^4$ or N;
$X^{23}$ is $CR^5$ or N;
$X^{24}$ is $CR^6$ or N;
$X^{25}$ is $CR^7$ or N;
$X^{26}$ is $CR^8$ or N;

wherein

0, 1 or 2, preferably 0 or 1, more preferably 0 of the groups $X^{19}$, $X^{20}$, $X^{21}$, $X^{22}$, $X^{23}$, $X^{24}$, $X^{25}$ and $X^{26}$ are N;
$R^1$ to $R^8$ are independently of each other H, -CN, a $C_1$-$C_{25}$alkyl group which is unsubstituted or substituted by at least one group E and/or interrupted by at least one group D, a $C_6$-$C_{40}$aryl group which is unsubstituted or substituted by at least one group E, a $C_1$-$C_{24}$heteroaryl group which is unsubstituted or substituted by at least one group E, a $C_7$-$C_{25}$aralkyl group which is unsubstituted or substituted by at least one group E, a $C_5$-$C_{12}$cycloalkyl group which is unsubstituted or substituted by at least one group E, $-OR^{21}$, - $SR^{21}$, $-NR^{17}R^{18}$, $-COR^{20}$, $-COOR^{19}$, $-CONR^{17}R^{18}$, $-SiR^{22}R^{23}R^{24}$, $-POR^{25}R^{27}$, or halogen, or at least two of the remaining residues $R^1$ to $R^8$ and $R^9$ to $R^{12}$ if present at adjacent carbon atoms may together form at least one $C_6$-$C_{18}$aryl, which is unsubstituted or substituted by at least one group E, or at least one $C_1$-$C_{24}$heteroaryl ring, which is unsubstituted or substituted by at least one group E,
$R^{14*}$ is H, a $C_1$-$C_{25}$alkyl group which is unsubstituted or substituted by at least one group E and/or interrupted by at least one group D, a $C_6$-$C_{40}$aryl group which is unsubstituted or substituted by at least one group E or a $C_1$-$C_{24}$heteroaryl group which is unsubstituted or substituted by at least one group E,
M is a $C_6$-$C_{40}$arylene group which is unsubstituted or substituted by at least one group E, a $C_1$-$C_{24}$heteroarylene group which is unsubstituted or substituted by at least one group E, a $C_1$-$C_{25}$alkylene group which is unsubstituted or substituted by at least one group E and/or interrupted by at least one group D, or a $C_2$-$C_{25}$alkenylene group which is unsubstituted or substituted by at least one group E and/or interrupted by at least one group D,
m is 0, 1 or 2, preferably 0 or 1, more preferably 0,
D is -CO-, -COO-, -S-, -SO-, $-SO_2-$, -O-, $-CR^{15}=CR^{16}-$, $-NR^{17}-$, $-SiR^{22}R^{23}-$, $-POR^{25}-$, -C≡C-,
E is $-OR^{21}$, $-SR^{21}$, $-NR^{17}R^{18}$, $-COR^{20}$, $-COOR^{19}$, $-CONR^{17}R^{18}$, -CN, $-SiR^{22}R^{23}R^{24}$,$-POR^{25}R^{27}$, halogen, preferably F,
a $C_6$-$C_{40}$aryl group which is unsubstituted or is substituted by at least one group selected from a $C_1$-$C_{18}$alkyl group, a $C_1$-$C_{18}$alkoxy group, a $C_1$-$C_{18}$alkyl group, which is interrupted by at least one O, a $C_6$-$C_{18}$aryl group, which is in turn unsubstituted or substituted by at least one $C_1$-$C_{18}$alkyl group or at least one $C_1$-$C_{18}$alkoxy group, a $C_5$-$C_{12}$cycloalkyl group, which is in turn unsubstituted or substituted by at least one $C_1$-$C_{18}$alkyl group or at least one $C_1$-$C_{18}$alkoxy group, a $C_1$-$C_{18}$heteroaryl group, which is in turn unsubstituted or substituted by at least one $C_1$-$C_{18}$alkyl group or at least one $C_1$-$C_{18}$alkoxy group, -F, $-CF_3$, - $CF_2CF_3$, $-CF_2CF_2CF_3$, $-CF(CF_3)_2$, $-(CF_2)_3CF_3$ and $-C(CF_3)_3$,
a $C_1$-$C_{18}$ alkyl group,
a $C_1$-$C_{18}$ alkoxy group,
a $C_1$-$C_{24}$heteroaryl group which is unsubstituted or substituted by at least one group selected from a $C_1$-$C_{18}$alkyl group, a $C_1$-$C_{18}$alkyl group, which is interrupted by at least one O, a $C_6$-$C_{18}$aryl group, which is in turn unsubstituted or substituted by at least one $C_1$-$C_{18}$alkyl group or at least one $C_1$-$C_{18}$alkoxy group, a $C_5$-$C_{12}$cycloalkyl group, which is in turn unsubstituted or substituted by at least one $C_1$-$C_{18}$alkyl group or at least one $C_1$-$C_{18}$alkoxy group, a $C_1$-$C_{18}$heteroaryl group, which is in turn unsubstituted or substituted by at least one $C_1$-$C_{18}$alkyl group or at least one $C_1$-$C_{18}$alkoxy group, -F, $-CF_3$, $-CF_2CF_3$,$-CF_2CF_2CF_3$, $-CF(CF_3)_2$, $-(CF_2)_3CF_3$ and $-C(CF_3)_3$,
$R^{15}$ and $R^{16}$ are independently of each other H, a $C_6$-$C_{18}$aryl group which is unsubstituted or substituted by at least one $C_1$-$C_{18}$alkyl group or at least one $C_1$-$C_{18}$alkoxy group,
$R^{17}$ and $R^{18}$ are independently of each other H, a $C_6$-$C_{18}$aryl group which is unsubstituted or substituted by at least one $C_1$-$C_{18}$alkyl group or at least one $C_1$-$C_{18}$alkoxy group, a $C_1$-$C_{18}$alkyl group or a $C_1$-$C_{18}$alkyl group, which is interrupted by at least one O, or
$R^{17}$ and $R^{18}$ together form a five or six membered aliphatic, aromatic or heteroaromatic ring, which is unsubstituted or substituted by at least one group E,
$R^{19}$ is H , a $C_6$-$C_{18}$aryl group which is unsubstituted or substituted by at least one $C_1$-$C_{18}$alkyl group or at least one $C_1$-$C_{18}$alkoxy group, a $C_1$-$C_{18}$alkyl group or a $C_1$-$C_{18}$alkyl group, which is interrupted by at least one O,
$R^{20}$ is H or a $C_6$-$C_{18}$aryl group which is unsubstituted or substituted by at least one $C_1$-$C_{18}$alkyl group or at least one $C_1$-$C_{18}$alkoxy group, a $C_1$-$C_{18}$alkyl group or a $C_1$-$C_{18}$alkyl group, which is interrupted by at least one O,
$R^{21}$ is independently of each other H, a $C_6$-$C_{18}$aryl group which is unsubstituted or substituted by at least one $C_1$-$C_{18}$alkyl group or at least one $C_1$-$C_{18}$alkoxy group, a $C_1$-$C_{18}$alkyl group or a $C_1$-$C_{18}$alkoxy group,
$R^{22,}$ $R^{23}$ and $R^{24}$ are independently of each other H, a $C_1$-$C_{18}$alkyl group, a $C_6$-$C_{18}$aryl group which is unsubstituted or substituted by at least one $C_1$-$C_{18}$alkyl group, and
$R^{25}$ and $R^{27}$ are independently of each other H, a $C_1$-$C_{18}$alkyl group, a $C_6$-$C_{18}$aryl group which is unsubstituted or substituted by at least one $C_1$-$C_{18}$alkyl group, a $C_7$-$C_{25}$aralkyl which is unsubstituted or substituted by at least one group E, a $C_5$-$C_{12}$cycloalkyl group which is unsubstituted or substituted by at least one group E.

**14.** An organic electroluminescence device comprising a cathode, an anode, and a plurality of organic layers provided between the cathode and the anode, wherein at least one of the plurality of organic layers is an emitting layer, wherein at least one organic layer comprises at least one compound of formula (I) as defined in any one of claims 1 to 12.

**15.** The organic electroluminescence device according to claim 13, wherein the emitting layer comprises the at least one compound of formula (I) as defined in any one of claims 1 to 12 as a delayed-fluorescent emitter and/or as a host material.

**16.** The organic electroluminescence device according to claim 14 or 15, further comprising a hole transporting layer between the anode and the emitting layer.

**17.** The organic electroluminescence device according to any one of claims 14 to 16, further comprising an electron transporting layer between the cathode and the emitting layer.

**18.** An electronic device comprising the organic electroluminescence device according to any one of claims 14 to 17.

**19.** An emitting layer comprising at least one compound of formula (I) as defined in any one of claims 1 to 12.

**20.** Use of the compound of formula (I) as defined in any one of claims 1 to 12 in an electronic device as a delayed-fluorescent emitter and/or as a host material.

FIG.1

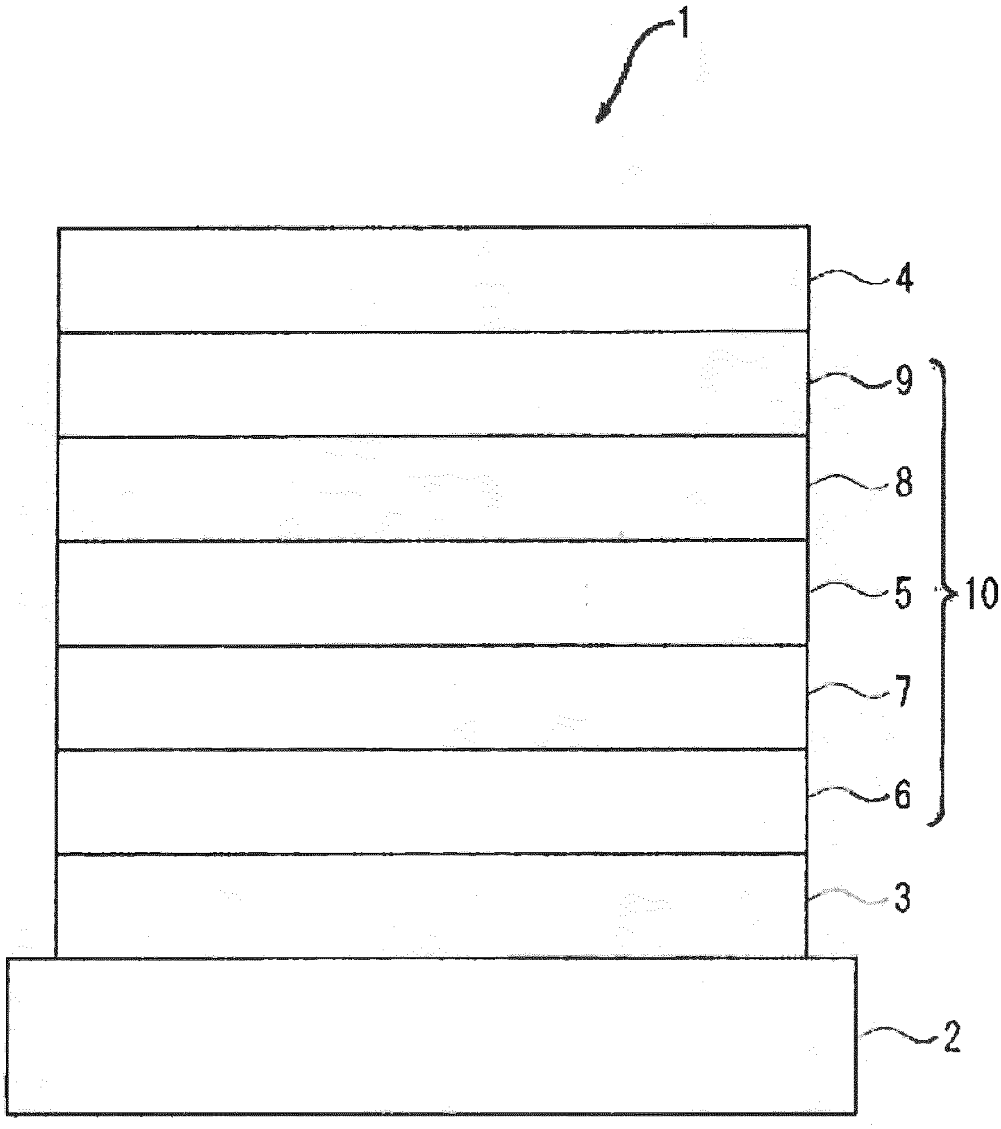

FIG. 2

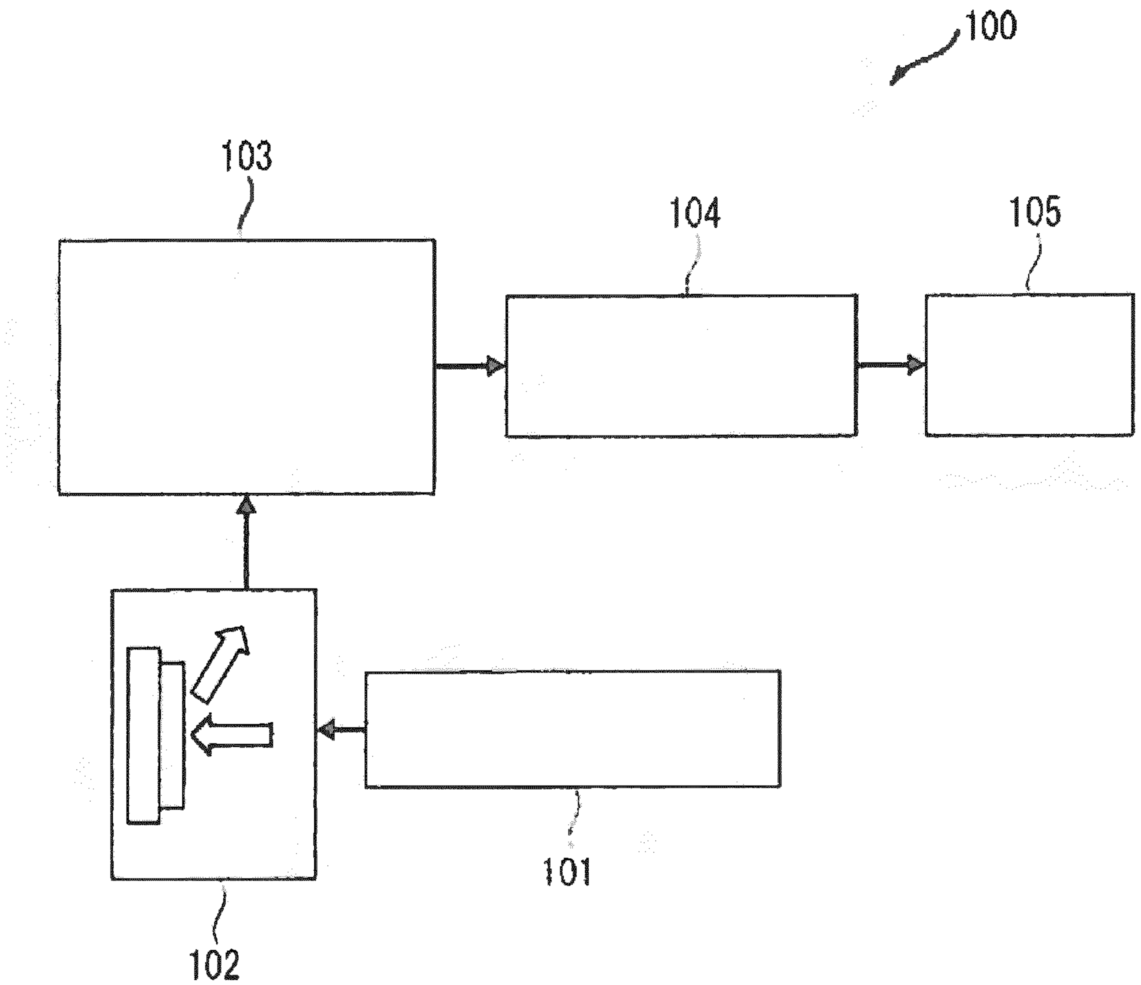

FIG. 3

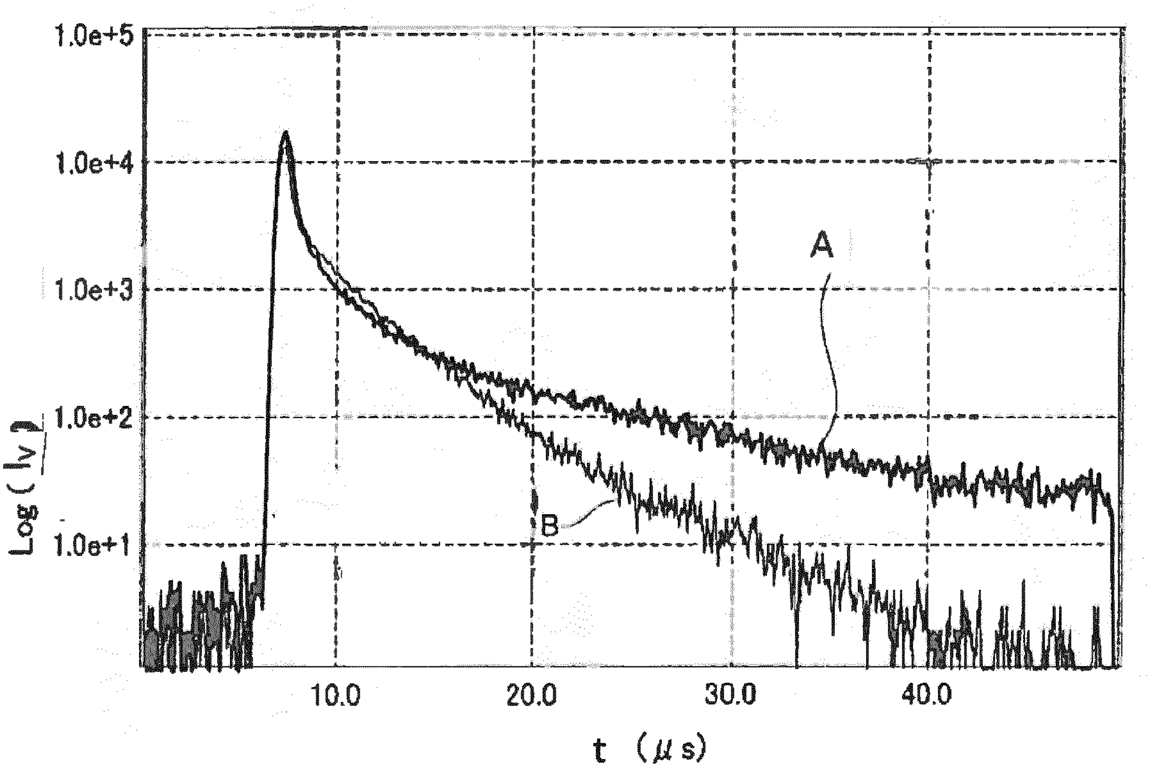

FIG.4

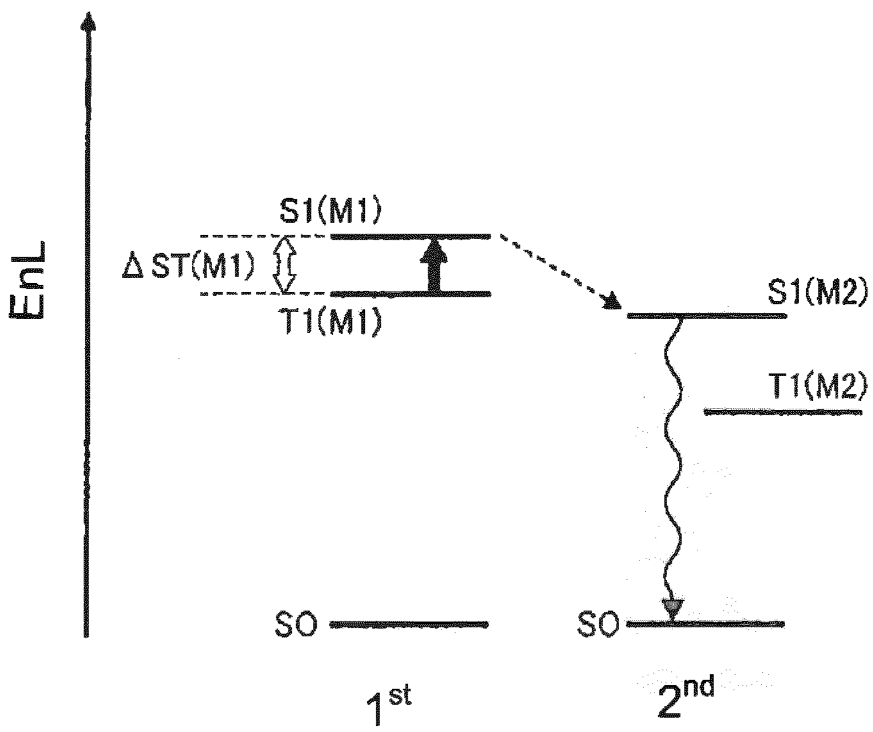

FIG.5

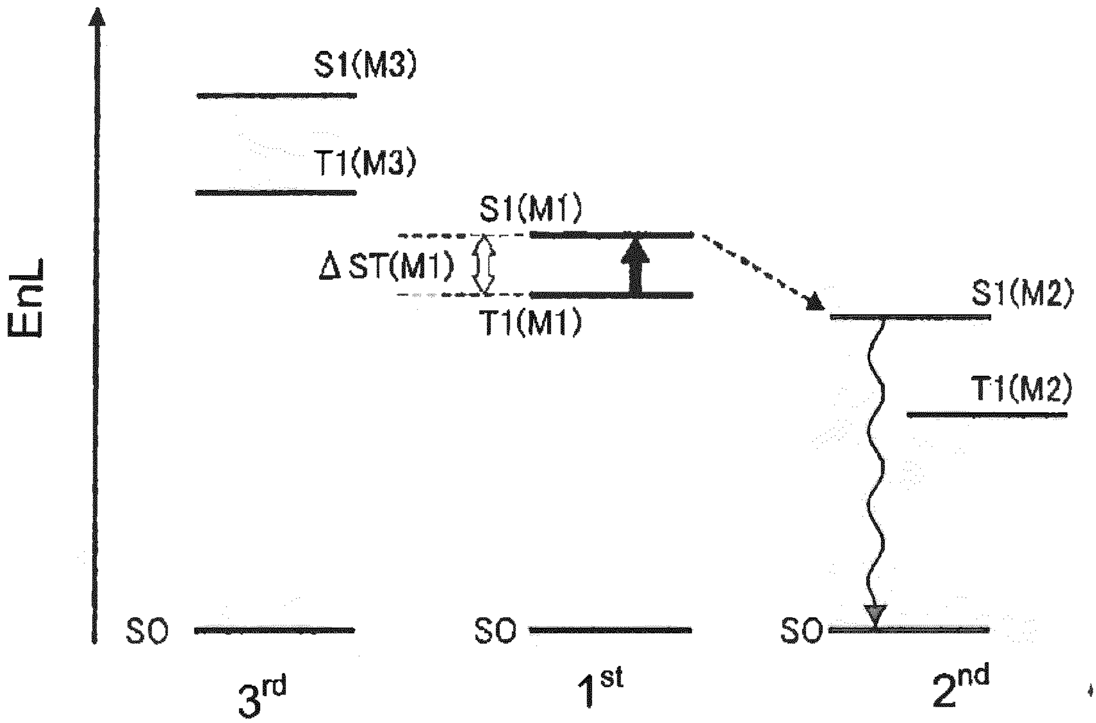

Europäisches Patentamt
European Patent Office
Office européen des brevets

# EUROPEAN SEARCH REPORT

Application Number
EP 18 18 0155

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 2017/093958 A1 (IDEMITSU KOSAN CO [JP]) 8 June 2017 (2017-06-08) * compounds of formula (I'-II'caH) on pages 78-92, in particular nos. 5-7, 29-31 etc. and compounds of formula (I'-II'c'aH) on pages 92-106, in particular nos. 6-8, 30-32 etc.; application examples 3 and 4 featuring compounds 10 and 11 on pages 213-214 (no CN group); claims * ----- | 1-20 | INV. C07D487/14 H01L51/50 |
| Y,D | KR 2017 0081438 A (SAMSUNG SDI CO LTD [KR]) 12 July 2017 (2017-07-12) * claims; compounds 1-210 * ----- | 1-20 | |
| Y,D | WO 2016/157113 A1 (IDEMITSU KOSAN CO [JP]) 6 October 2016 (2016-10-06) * preferred compounds of pages 27-34; claims; examples * ----- | 1-20 | |

TECHNICAL FIELDS SEARCHED (IPC)

C07D

The present search report has been drawn up for all claims

1

| Place of search | Date of completion of the search | Examiner |
|---|---|---|
| Munich | 4 September 2018 | Stroeter, Thomas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# ANNEX TO THE EUROPEAN SEARCH REPORT ON EUROPEAN PATENT APPLICATION NO. EP 18 18 0155

This annex lists the patent family members relating to the patent documents cited in the above-mentioned European search report. The members are as contained in the European Patent Office EDP file on
The European Patent Office is in no way liable for these particulars which are merely given for the purpose of information.

04-09-2018

| Patent document cited in search report | Publication date | Patent family member(s) | Publication date |
| --- | --- | --- | --- |
| WO 2017093958 A1 | 08-06-2017 | NONE | |
| KR 20170081438 A | 12-07-2017 | NONE | |
| WO 2016157113 A1 | 06-10-2016 | EP 3075737 A1 | 05-10-2016 |
| | | US 2018086763 A1 | 29-03-2018 |
| | | WO 2016157113 A1 | 06-10-2016 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


### Patent documents cited in the description

- WO 2016157113 A1
- WO 2017115596 A1
- WO 2017093958 A1
- KR 20170081438
- US 20160172601 A1
- US 20160190469 A1
- WO 2017146397 A
- US 20170141322 A
- EP 3109253 A1
- EP 3255693 A1
- WO 2012153780 A
- WO 2013038650 A
- EP 3255693 A
- WO 2017056052 A1

### Non-patent literature cited in the description

- Yuki Hando-tai no Debaisu Bussei (Device Physics of Organic Semiconductors). Kodansha, 22 March 2012, 261-262
- **HAJIME NAKANOTANI et al.** High-Efficiency organic light-emitting diodes with fluorescent emitters. *NATURE COMMUNICATIONS,* 30 May 2014, vol. 5, 4016
- Thermally Activated Delayed Fluorescence. *Adv. Mater.,* 2014, vol. 26, 7931-7958
- Yuki Hando-tai no Debaisu Bussei (Device Physics of Organic Semiconductors). Kodansha, 261-268
- *Nature,* 2012, vol. 492, 234-238
- *Advanced Materials,* 2016, vol. 28 (14), 2777-2781
- *Chem. Soc. Rev,* 2017, vol. 46 (915), 974
- *Adv. Mater.,* 13 June 2017, vol. 29 (22), 39
- *J. Mater. Chem. C,* 2016, vol. 4, 11355-11381
- *J. Mater. Chem. C,* 2017, vol. 5, 8622-8653